# EUROPÄISCHE PATENTANMELDUNG

(11) **EP 2 163 238 A2**
(43) Veröffentlichungstag der Anmeldung: **17.03.2010**
(21) Anmeldenummer: 09155888.2
(22) Anmeldetag: 23.03.2009
(51) Int. Cl.: A61K 8/362, A61K 8/46, A61Q 19/02

(54) **Zusammensetzung zur Hautaufhellung enthaltend Sulforaphan oder Sulforaphen oder deren Derivate**

(30) Priorität: 15.09.2008 DE 102008047362
(71) Anmelder: Henkel AG & Co. KGaA, 40589 Düsseldorf (DE)
(72) Erfinder: Janßen, Frank, 41470, Neuss (DE); Waldmann-Laue, Marianne, 40789, Monheim (DE)

(57) **Zusammenfassung**

Gegenstand der vorliegenden Anmeldung sind kosmetische oder dermatologische Zusammensetzungen zur Aufhellung der Haut, die 8-Hexadecen-1,16-dicarbonsäure und mindestens eine Substanz, ausgewählt aus der Gruppe, bestehend aus Sulforaphan, Sulforaphen, Sulforaphan-Analoga und Sulforaphen-Analoga, enthalten.

## Beschreibung

Die vorliegende Erfindung betrifft kosmetische oder dermatologische Zusammensetzungen zur kosmetischen und topischen dermatologischen Hautaufhellung oder zur Verhinderung der Hautbräunung, insbesondere der durch UV-Strahlung hervorgerufenen Hautbräunung.

Die Pigmentierung der Haut erfolgt durch Melanozyten, welche in der untersten Schicht der Epidermis neben den Basalzellen als je nach Hauttyp entweder vereinzelt oder gehäuft auftretende pigmentbildende Zellen vorzufinden sind. Melanozyten enthalten Melanosomen, in denen Melanin gebildet wird. Durch verschiedene chemische und/oder physikalische Einflüsse, insbesondere durch UV- Strahlung, wird verstärkt Melanin gebildet. Dieses wird über die Keratinozyten in die Corneozyten (Hornschicht) transportiert und führt zu einer bräunlichen bis braun-schwarzen Hautfarbe. Melanin wird als Endstufe eines oxidativen Prozesses gebildet, in welchem Tyrosin unter Mitwirkung der Enzyms Tyrosinase über mehrere Zwischenstufen zu den braun bis braunschwarzen Eumelaninen (DHICA- und DHI-Melanin) bzw. unter Beteiligung von schwefelhaltigen Verbindungen zum rötlichen Phäomelanin umgewandelt wird. Die Melaninbildung - und damit Hautfarbe - unterliegt äußeren Einflüssen und kann neben erwünschten Effekten ("gesunde Bräune") auch zu unerwünschten Erscheinungen führen. So kann z.B. UV-Strahlung zu Sommersprossen führen. Fehlpigmentierungen können auch aufgrund genetischer Disposition, Wundheilung bzw. -vernarbung oder Hautalterung ("Altersflecken") auftreten. Da der Prozess der Desquamation (oberflächliche Loslösung von Zellen oder Zellgruppen aus ihrem epithelialen Verband) einen beständigen Verlust an Melanin beinhaltet, kann eine Aufhellung der Haut durch Inhibierung der Neusynthese von Melanin erreicht werden.
Wirksame kosmetische Zusammensetzungen zur Hautaufhellung sind bekannt. Diese enthalten als Wirkstoff jedoch zumeist Hydrochinon oder Kojisäure (Inhibierung der Tyrosinase). Beide Substanzen sind mutagen und sollten daher nicht über längere Zeiträume verwendet werden.
Es bestand daher nach wie vor die Aufgabe, hautaufhellende Zusammensetzungen zur Verfügung zu stellen, die frei von den genannten Nachteilen sind.
Es hat sich überraschenderweise gezeigt, dass die Kombination von mindestens einer Dicarbonsäure, ausgewählt aus 8-Hexadecen-1,16-dicarbonsäure, 7-Tetradecen-1,14-dicarbonsäure, 9-Octadecen-1,18-dicarbonsäure, 6-Dodecen-1,12-dicarbonsäure, 5-Decen-1,10-dicarbonsäure, Sebacinsäure und Azelainsäure sowie Mischungen dieser Dicarbonsäuren, mit mindestens einer Substanz, ausgewählt aus der Gruppe, bestehend aus Sulforaphan, Sulforaphen, Sulforaphan-Analoga und Sulforaphen-Analoga, zu einer synergistischen Inhibition der Melaninsynthese führt. Bei großflächiger Anwendung auf der Haut führt dies zu einer Aufhellung der Hautfarbe. Bei lokaler Anwendung auf Pigmentstörungen wie Altersflecken verlieren diese an Kontrast gegenüber dem angrenzenden Hautareal und sind daher weniger sichtbar.
Gegenstand der vorliegenden Anmeldung ist in einer ersten Ausführungsform eine kosmetische oder dermatologische Zusammensetzung zur Aufhellung der Haut, die 8-Hexadecen-1,16-dicarbonsäure und/oder 7-Tetradecen-1,14-dicarbonsäure und/oder 9-Octadecen-1,18-dicarbonsäure und/oder 6-Dodecen-1,12-dicarbonsäure und/oder 5-Decen-1,10-dicarbonsäure und/oder Decandisäure (Sebacinsäure) und/oder Nonandisäure (Azelainsäure) in einer Gesamtmenge von 0,01 - 5 Gew.-% in Kombination mit 0,0001 bis 5 Gew.-% Gesamtgehalt an mindestens einer Substanz, ausgewählt aus der Gruppe, bestehend aus Sulforaphan, Sulforaphen, Sulforaphan-Analoga und
Sulforaphen-Analoga, enthält.
Die erfindungsgemäßen Zusammensetzungen enthalten 8-Hexadecen-1,16-dicarbonsäure, 7-Tetradecen-1,14-dicarbonsäure, 9-Octadecen-1,18-dicarbonsäure, 6-Dodecen-1,12-dicarbonsäure, 5-Decen-1,10-dicarbonsäure, Decandisäure (Sebacinsäure) und/oder Nonandisäure (Azelainsäure) in einer Gesamtmenge von 0,01 - 5 Gew.-%, bevorzugt 0,05 - 3,0 Gew.-%, besonders bevorzugt 0,07 - 2,0 Gew.-% und außerordentlich bevorzugt 0,1 - 1,0 Gew.-%. Gesamtmengen von 0,2 - 0,8 Gew.-%, insbesondere 0,3 - 0,6 Gew.-% und vor allem 0,4 - 0,5 Gew.-%, jeweils bezogen auf das Gewicht der gesamten erfindungsgemäßen Zusammensetzung, können ebenfalls bevorzugt sein. 8-Hexadecen-1,16-dicarbonsäure (Dioic acid, INCI-Bezeichnung Octadecenedioic acid), die auch als 9-Octadecen-1,18-disäure bezeichnet wird (siehe z. B. RÖMPP Chemielexikon zur Nomenklatur von Dicarbonsäuren), ist ein Stoffwechselprodukt von Hefezellen aus selektierten Mutanten-Stämmen des Candida Stammes, wobei als Ausgangssubstanz eine Fettsäure rein pflanzlichen Ursprungs dient, die in die Hydroxyfettsäure umgesetzt wird, welche anschließend über die Stufe des Fettsäurealdehyd zur Dicarboxysäure oxidiert wird. Das Handelsprodukt besitzt eine Reinheit von 95%, wobei die 8- Hexadecen-1,16-dicarbonsäure darin als Gemisch des cis- und trans- Isomeren vorliegt und das cis-Isomere mengenmäßig überwiegt. Das Produkt kann bis zu 3 Gew.-% Ölsäure enthalten.
Decandisäure (Sebacinsäure), HOOC-(CH₂)₈-COOH, kommt in Form farbloser, monoklin-prismatischer Blättchen in den Handel und wird durch Erhitzen von Ricinusöl oder Ricinolsäure mit NaOH und Luft oder durch Kolbe-Synthese aus Adipinsäuremonomethylester oder durch Oxidation von Cyclodecanol hergestellt. Nonandisäure (Azelainsäure), HOOC-(CH₂)₇-COOH, kommt ebenfalls in Form farbloser Blättchen oder Nadel in den Handel und wird durch Oxidation aus Ricinolsäure, oder durch Ozonolyse von Ölsäure hergestellt.
Bevorzugte erfindungsgemäße kosmetische oder dermatologische Zusammensetzungen enthalten, bezogen auf ihr Gewicht, 0,05 - 3,0 Gew.-%, vorzugsweise 0,07 - 2,0 Gew.-% und insbesondere 0,1 - 1,0 Gew.-% 8-Hexadecen-1,16-dicarbonsäure.
Weitere bevorzugte erfindungsgemäße kosmetische oder dermatologische Zusammensetzungen enthalten, bezogen auf ihr Gewicht, 0,05 - 3,0 Gew.-%, vorzugsweise 0,07 - 2,0 Gew.-% und insbesondere 0,1 - 1,0 Gew.-% 7-Tetradecen-1,14-dicarbonsäure.
Weitere bevorzugte erfindungsgemäße kosmetische oder dermatologische Zusammensetzungen enthalten, bezogen auf ihr Gewicht, 0,05 - 3,0 Gew.-%, vorzugsweise 0,07 - 2,0 Gew.-% und insbesondere 0,1 - 1,0 Gew.-% 9-Octadecen-1,18-dicarbonsäure.
Weitere bevorzugte erfindungsgemäße kosmetische oder dermatologische Zusammensetzungen enthalten, bezogen auf ihr Gewicht, 0,05 - 3,0 Gew.-%, vorzugsweise 0,07 - 2,0 Gew.-% und insbesondere 0,1 - 1,0 Gew.-% 6-Dodecen-1,12-dicarbonsäure.
Weitere bevorzugte erfindungsgemäße kosmetische oder dermatologische Zusammensetzungen enthalten, bezogen auf ihr Gewicht, 0,05 - 3,0 Gew.-%, vorzugsweise 0,07 - 2,0 Gew.-% und insbesondere 0,1 - 1,0 Gew.-% 5-Decen-1,10-dicarbonsäure.
Weitere bevorzugte erfindungsgemäße kosmetische oder dermatologische Zusammensetzungen enthalten, bezogen auf ihr Gewicht, 0,05 - 3,0 Gew.-%, vorzugsweise 0,07 - 2,0 Gew.-% und insbesondere 0,1 - 1,0 Gew.-% Sebacinsäure.
Weitere bevorzugte erfindungsgemäße kosmetische oder dermatologische Zusammensetzungen enthalten, bezogen auf ihr Gewicht, 0,05 - 3,0 Gew.-%, vorzugsweise 0,07 - 2,0 Gew.-% und insbesondere 0,1 - 1,0 Gew.-% Azelainsäure.
Als zweite obligatorische Komponente enthalten die erfindungsgemäßen Zusammensetzungen mindestens eine Substanz, ausgewählt aus der Gruppe, bestehend aus Sulforaphan, Sulforaphen, Sulforaphan-Analoga und Sulforaphen-Analoga, in einer Gesamtmenge von 0,0001 bis 5 Gew.-%, bezogen auf das Gesamtgewicht der erfindungsgemäßen Zusammensetzung.
Bevorzugte erfindungsgemäße kosmetische oder dermatologische Zusammensetzungen sind enthalten, bezogen auf ihr Gewicht, insgesamt 0,0005 bis 3,0 Gew.-%, vorzugsweise 0,001 bis 2,0 Gew.-% und insbesondere 0,01 bis 1,0 Gew.-%, mindestens einer Substanz, ausgewählt aus der Gruppe, bestehend aus Sulforaphan, Sulforaphen, Sulforaphan-Analoga und Sulforaphen-Analoga. Gesamtmengen von 0,1 - 0,8 Gew.-%, insbesondere 0,2 - 0,6 Gew.-% und vor allem 0,3 - 0,5 Gew.-%, bezogen auf die gesamte erfindungsgemäße Zusammensetzung, können ebenfalls bevorzugt sein.
Sulforaphan ist ein organisches Isothiocyanat mit der Strukturformel 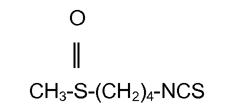 bzw.

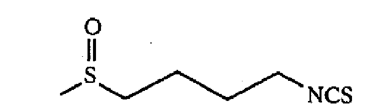

### Sulforaphen ist ein organisches Isothiocyanat mit der Strukturformel

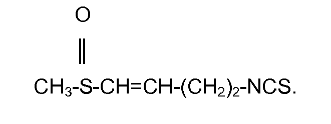

Bevorzugte Sulforaphan-Analoga sind ausgewählt ist aus folgenden Verbindungen: 6-Isothiocyanato-2-hexanon (GHP 1105), exo-2-Acetyl-6-isothiocyanatonorbornan (GHP 1066), exo-2-Isothiocyanato-6-methylsulfonylnorbornan (GHP 1068), 6-Isothiocyanato-2-hexanol (GHP 1106), 1-Isothiocyanato-4-dimethylphosphonylbutan (GHP 1078), exo-2-(1'-Hydroxyethyl)-5-isothiocyanatonorbor-nan (GHP 1075), exo-2-Acetyl-5-isothiocyanatonorbornan (GHP 1067), 1-Isothiocyanato-8-(methylsulfinyl)-octan (Hirsutin), 1-Isothiocyanato-5-methylsulfonylpentan (GHP 1003), 1-Isothiocyanato-5-methylsulfinylpentan (Alyssin, GHP 1002), 1-Isothiocyanato-4-methylsulfonylbutan (Erysolin, GHP 1007), 1-Isothiocyanato-3-methylsulfinylpropan (Iberin, GHP 1009), 1-Isothiocyanato-3-methyl-sulfonylpropan (GHP 1010), cis-3-(Methylsulfonyl)cyclohexylisothiocyanat (GHP 1073), cis-3-(Methylsulfonyl)cyclohexylmethylisothiocyanat (GHP 1079), trans-3-(Methylsulfonyl)cyclohexyl-methylisothiocyanat (GHP 1080), 2-Isothiocyanato-4-methylsulfonyl-ethylbutyrat (GHP 1074), 9-Isothiocyanato-5-nonanon (GHP 1081), 7-Isothiocyanato-3-heptanon (Norcappasalin), 8-Isothiocyanato-4-octanon (Cappasalin), 7-Isothiocyanato-4-heptanon (Capangulin), exo-2-Cyano-5-iso-thiocyanatonorbornan (GHP 1071), 1-Cyano-4-isothiocyanatobutan (GHP 1101), 5-Isothiocyanato-methylpentanoat (GHP 1102), 5-Isothiocyanatopentansäure (GHP 1103), 2-Isothiocyanato-ethylacetat (GHP 1061), 4-Isothiocyanato-methylbutyrat (Erypestrin), 4-Isothiocyanato-ethylbutyrat, 1-Isothiocyanato-2-methoxyphenol (GHP 1031), Erucin (4-Methylthiobutylisothiocyanat, GHP 1006), Iberverin (3-Methylthiopropylisothiocyanat, GHP 1008), Berteroin (5-Methylthiopentylisothiocyanat, GHP 1001), Lesquerellin (6-Methylthiohexylisothiocyanat), Jirsutin (8-Methylthiooctylisothiocyanat), Arabin (9-Methylthiononylisothiocyanat), Allylisothiocyanat, Brassicin (Indol-3-ylmethylisothiocya-nat), Goitrin (2-Hydroxybut-3-enyl-isothiocyanat), Napin (But-3-enyl-isothiocyanat), Neobrassicin (*N*-Methoxyindol-3-ylmethyl-isothiocyanat), *N*-Acetylindol-3-ylmethyl-isothiocyanat, Nasturtiin ((2-Phenylethyl)-isothiocyanat), Barbarin (2-Hydroxy-2-phenylethyl-isothiocyanat), Tropaeolin (Benzylisothiocyanat), Sinalbin (4-Hydroxybenzylisothiocyanat), Capparin (Methylisothiocyanat), Lepidin (Ethylisothiocyanat), Putranjivin (Isopropylisothiocyanat), Jiaputin (2-Methylbutylisothiocyanat), Raphasatin (4-Methylthiobut-3-enyl-isothiocyanat), Cochlearin (1-Methylpropylisothiocyanat), Brassicanapin (4-Pentenylisothiocyanat), Napoleiferin (2-Hydroxypent-4-enylisothiocyanat),

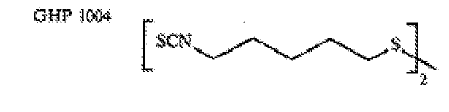

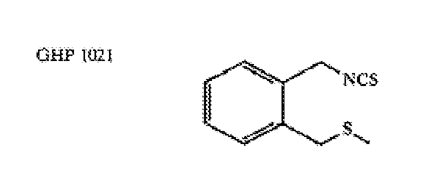

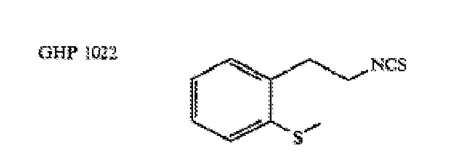

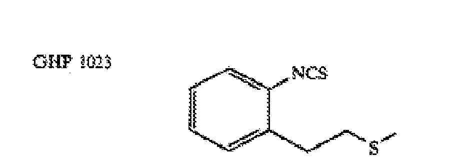

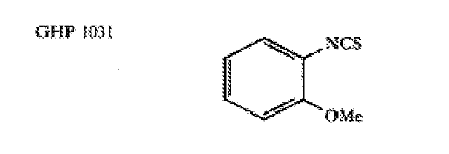

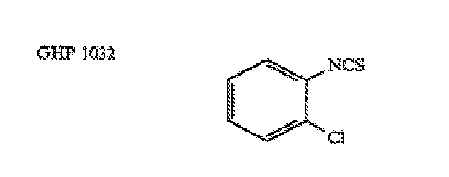

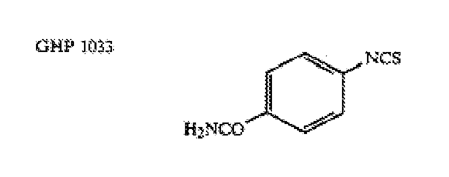

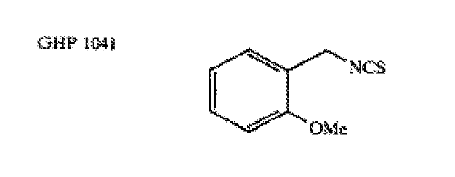

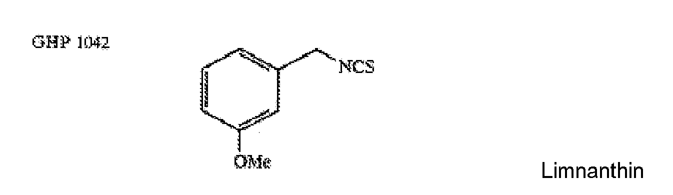

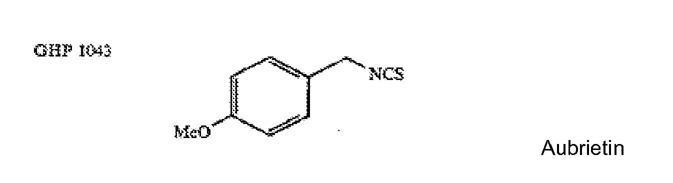

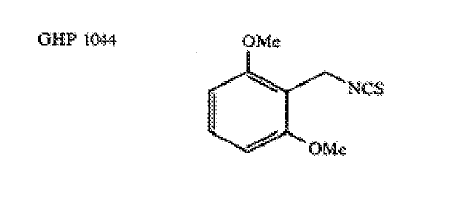

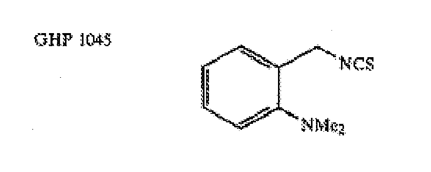

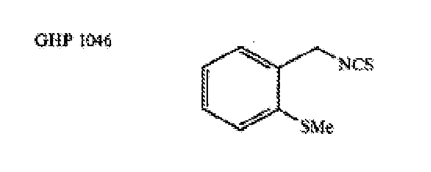

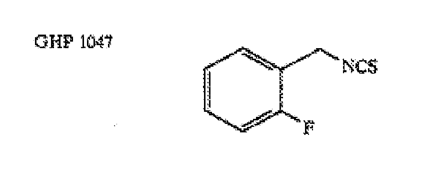

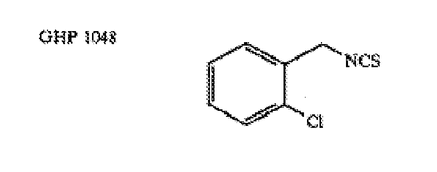

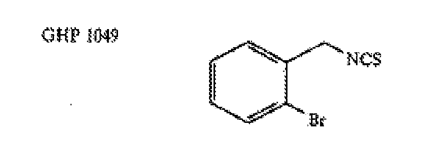

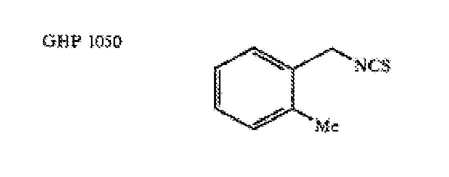

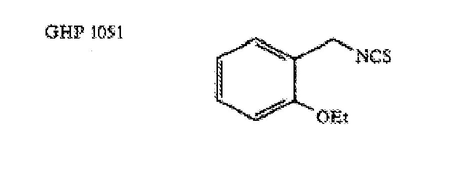

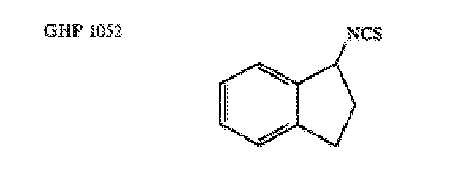

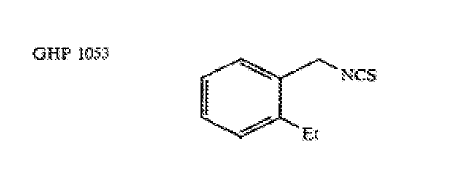

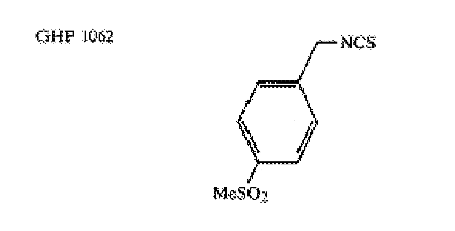

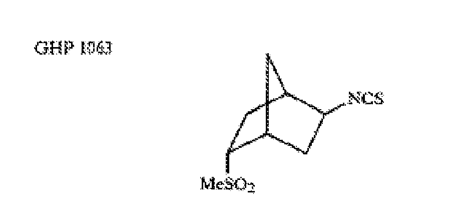

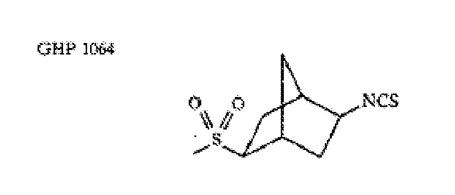

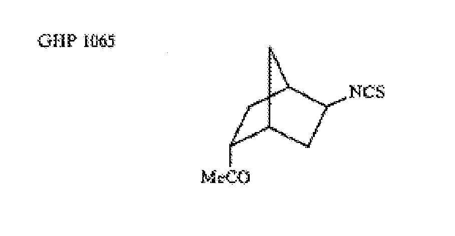

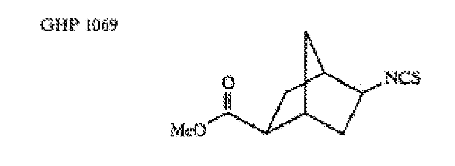

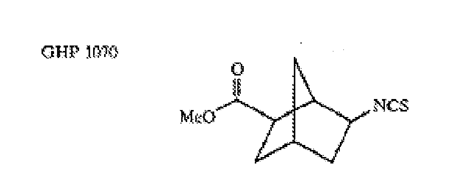

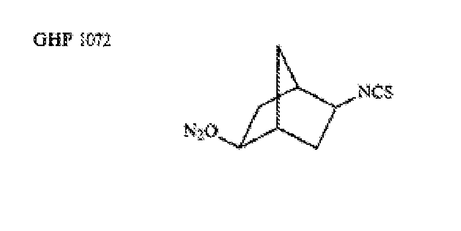

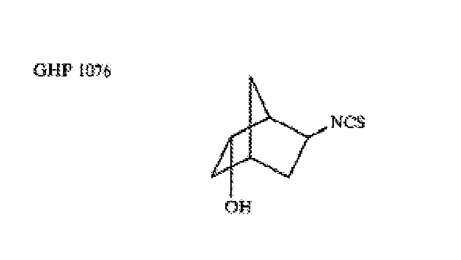

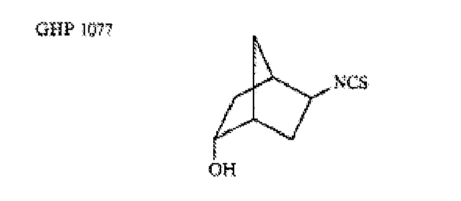

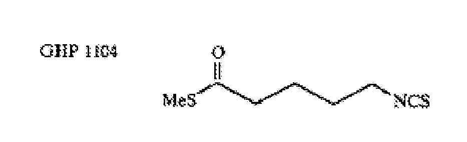

Besonders bevorzugte erfindungsgemäße Zusammensetzungen enthalten 0,01 - 5 Gew.-% 8-Hexadecen-1,16-dicarbonsäure und 0,0001 bis 5 Gew.-% Sulforaphan.

Weitere besonders bevorzugte erfindungsgemäße Zusammensetzungen enthalten 0,01 - 5 Gew.-% 8-Hexadecen-1,16-dicarbonsäure und 0,0001 bis 5 Gew.-% Sulforaphen.

Weitere besonders bevorzugte erfindungsgemäße Zusammensetzungen enthalten 0,01 - 5 Gew.-% 8-Hexadecen-1,16-dicarbonsäure und 0,0001 bis 5 Gew.-% 6-Isothiocyanato-2-hexanon.

Weitere besonders bevorzugte Zusammensetzungen enthalten 0,01 - 5 Gew.-% 8-Hexadecen-1,16-dicarbonsäure und 0,0001 bis 5 Gew.-% exo-2-Acetyl-6-isothiocyanatonorbornan.

Weitere besonders bevorzugte Zusammensetzungen enthalten 0,01 - 5 Gew.-% 8-Hexadecen-1,16-dicarbonsäure und 0,0001 bis 5 Gew.-% exo-2-Isothiocyanato-6-methylsulfonylnorbornan.

Weitere besonders bevorzugte Zusammensetzungen enthalten 0,01 - 5 Gew.-% 8-Hexadecen-1,16-dicarbonsäure und 0,0001 bis 5 Gew.-% 6-Isothiocyanato-2-hexanol.

Weitere besonders bevorzugte Zusammensetzungen enthalten 0,01 - 5 Gew.-% 8-Hexadecen-1,16-dicarbonsäure und 0,0001 bis 5 Gew.-% 1-Isothiocyanato-4-dimethylphosphonylbutan.

Weitere besonders bevorzugte Zusammensetzungen enthalten 0,01 - 5 Gew.-% 8-Hexadecen-1,16-dicarbonsäure und 0,0001 bis 5 Gew.-% exo-2-(1'-Hydroxyethyl)-5-isothiocyanatonorbornan.

Weitere besonders bevorzugte Zusammensetzungen enthalten 0,01 - 5 Gew.-% 8-Hexadecen-1,16-dicarbonsäure und 0,0001 bis 5 Gew.-% exo-2-Acetyl-5-isothiocyanatonorbornan.

Weitere besonders bevorzugte Zusammensetzungen enthalten 0,01 - 5 Gew.-% 8-Hexadecen-1,16-dicarbonsäure und 0,0001 bis 5 Gew.-% 1-Isothiocyanato-8-(methylsulfinyl)-octan (Hirsutin).

Weitere besonders bevorzugte Zusammensetzungen enthalten 0,01 - 5 Gew.-% 8-Hexadecen-1,16-dicarbonsäure und 0,0001 bis 5 Gew.-% 1-Isothiocyanato-5-methylsulfonylpentan.

Weitere besonders bevorzugte Zusammensetzungen enthalten 0,01 - 5 Gew.-% 8-Hexadecen-1,16-dicarbonsäure und 0,0001 bis 5 Gew.-% 1-Isothiocyanato-5-methylsulfinylpentan (Alyssin).

Weitere besonders bevorzugte Zusammensetzungen enthalten 0,01 - 5 Gew.-% 8-Hexadecen-1,16-dicarbonsäure und 0,0001 bis 5 Gew.-% 1-Isothiocyanato-4-methylsulfonylbutan (Erysolin).

Weitere besonders bevorzugte Zusammensetzungen enthalten 0,01 - 5 Gew.-% 8-Hexadecen-1,16-dicarbonsäure und 0,0001 bis 5 Gew.-% 1-Isothiocyanato-3-methylsulfinylpropan (Iberin).

Weitere besonders bevorzugte Zusammensetzungen enthalten 0,01 - 5 Gew.-% 8-Hexadecen-1,16-dicarbonsäure und 0,0001 bis 5 Gew.-% 1-Isothiocyanato-3-methylsulfonylpropan.

Weitere besonders bevorzugte Zusammensetzungen enthalten 0,01 - 5 Gew.-% 8-Hexadecen-1,16-dicarbonsäure und 0,0001 bis 5 Gew.-% cis-3-(Methylsulfonyl)cyclohexylisothiocyanat.

Weitere besonders bevorzugte Zusammensetzungen enthalten 0,01 - 5 Gew.-% 8-Hexadecen-1,16-dicarbonsäure und 0,0001 bis 5 Gew.-% cis-3-(Methylsulfonyl)cyclohexylmethylisothiocyanat.

Weitere besonders bevorzugte Zusammensetzungen enthalten 0,01 - 5 Gew.-% 8-Hexadecen-1,16-dicarbonsäure und 0,0001 bis 5 Gew.-% trans-3-(Methylsulfonyl)cyclohexylmethylisothio-cyanat.

Weitere besonders bevorzugte Zusammensetzungen enthalten 0,01 - 5 Gew.-% 8-Hexadecen-1,16-dicarbonsäure und 0,0001 bis 5 Gew.-% 2-Isothiocyanato-4-methylsulfonyl-ethylbutyrat.

Weitere besonders bevorzugte Zusammensetzungen enthalten 0,01 - 5 Gew.-% 8-Hexadecen-1,16-dicarbonsäure und 0,0001 bis 5 Gew.-% 9-Isothiocyanato-5-nonanon.

Weitere besonders bevorzugte Zusammensetzungen enthalten 0,01 - 5 Gew.-% 8-Hexadecen-1,16-dicarbonsäure und 0,0001 bis 5 Gew.-% 7-Isothiocyanato-3-heptanon (Norcappasalin).

Weitere besonders bevorzugte Zusammensetzungen enthalten 0,01 - 5 Gew.-% 8-Hexadecen-1,16-dicarbonsäure und 0,0001 bis 5 Gew.-% 8-Isothiocyanato-4-octanon (Cappasalin).

Weitere besonders bevorzugte Zusammensetzungen enthalten 0,01 - 5 Gew.-% 8-Hexadecen-1,16-dicarbonsäure und 0,0001 bis 5 Gew.-% 7-Isothiocyanato-4-heptanon (Capangulin).

Weitere besonders bevorzugte Zusammensetzungen enthalten 0,01 - 5 Gew.-% 8-Hexadecen-1,16-dicarbonsäure und 0,0001 bis 5 Gew.-% exo-2-Cyano-5-isothiocyanatonorbornan.

Weitere besonders bevorzugte Zusammensetzungen enthalten 0,01 - 5 Gew.-% 8-Hexadecen-1,16-dicarbonsäure und 0,0001 bis 5 Gew.-% 1-Cyano-4-isothiocyanatobutan.

Weitere besonders bevorzugte Zusammensetzungen enthalten 0,01 - 5 Gew.-% 8-Hexadecen-1,16-dicarbonsäure und 0,0001 bis 5 Gew.-% 5-Isothiocyanato-methylpentanoat.

Weitere besonders bevorzugte Zusammensetzungen enthalten 0,01 - 5 Gew.-% 8-Hexadecen-1,16-dicarbonsäure und 0,0001 bis 5 Gew.-% 5-Isothiocyanatopentansäure.

Weitere besonders bevorzugte erfindungsgemäße Zusammensetzungen enthalten 0,01 - 5 Gew.-% 8-Hexadecen-1,16-dicarbonsäure und 0,0001 bis 5 Gew.-% 2-Isothiocyanato-ethylacetat.

Weitere besonders bevorzugte Zusammensetzungen enthalten 0,01 - 5 Gew.-% 8-Hexadecen-1,16-dicarbonsäure und 0,0001 bis 5 Gew.-% 4-Isothiocyanato-methylbutyrat (Erypestrin).

Weitere besonders bevorzugte erfindungsgemäße Zusammensetzungen enthalten 0,01 - 5 Gew.-% 8-Hexadecen-1,16-dicarbonsäure und 0,0001 bis 5 Gew.-% 4-Isothiocyanato-ethylbutyrat.

Weitere besonders bevorzugte erfindungsgemäße Zusammensetzungen enthalten 0,01 - 5 Gew.-% 8-Hexadecen-1,16-dicarbonsäure und 0,0001 bis 5 Gew.-% 1-Isothiocyanato-2-methoxyphenol.

Weitere besonders bevorzugte Zusammensetzungen enthalten 0,01 - 5 Gew.-% 8-Hexadecen-1,16-dicarbonsäure und 0,0001 bis 5 Gew.-% Erucin (4-Methylthiobutylisothiocyanat.

Weitere besonders bevorzugte Zusammensetzungen enthalten 0,01 - 5 Gew.-% 8-Hexadecen-1,16-dicarbonsäure und 0,0001 bis 5 Gew.-% Iberverin (3-Methylthiopropylisothiocyanat.

Weitere besonders bevorzugte Zusammensetzungen enthalten 0,01 - 5 Gew.-% 8-Hexadecen-1,16-dicarbonsäure und 0,0001 bis 5 Gew.-% Berteroin (5-Methylthiopentylisothiocyanat.

Weitere besonders bevorzugte Zusammensetzungen enthalten 0,01 - 5 Gew.-% 8-Hexadecen-1,16-dicarbonsäure und 0,0001 bis 5 Gew.-% Lesquerellin (6-Methylthiohexylisothiocyanat).

Weitere besonders bevorzugte Zusammensetzungen enthalten 0,01 - 5 Gew.-% 8-Hexadecen-1,16-dicarbonsäure und 0,0001 bis 5 Gew.-% Jirsutin (8-Methylthiooctylisothiocyanat).

Weitere besonders bevorzugte Zusammensetzungen enthalten 0,01 - 5 Gew.-% 8-Hexadecen-1,16-dicarbonsäure und 0,0001 bis 5 Gew.-% Arabin (9-Methylthiononylisothiocyanat).

Weitere besonders bevorzugte Zusammensetzungen enthalten 0,01 - 5 Gew.-% 8-Hexadecen-1,16-dicarbonsäure und 0,0001 bis 5 Gew.-% Allylisothiocyanat.

Weitere besonders bevorzugte Zusammensetzungen enthalten 0,01 - 5 Gew.-% 8-Hexadecen-1,16-dicarbonsäure und 0,0001 bis 5 Gew.-% Brassicin (Indol-3-ylmethylisothiocyanat).

Weitere besonders bevorzugte Zusammensetzungen enthalten 0,01 - 5 Gew.-% 8-Hexadecen-1,16-dicarbonsäure und 0,0001 bis 5 Gew.-% Goitrin (2-Hydroxybut-3-enyl-isothiocyanat).

Weitere besonders bevorzugte erfindungsgemäße Zusammensetzungen enthalten 0,01 - 5 Gew.-% 8-Hexadecen-1,16-dicarbonsäure und 0,0001 bis 5 Gew.-% Napin (But-3-enyl-isothiocyanat).

Weitere besonders bevorzugte Zusammensetzungen enthalten 0,01 - 5 Gew.-% 8-Hexadecen-1,16-dicarbonsäure und 0,0001 bis 5 Gew.-% Neobrassicin (*N*-Methoxyindol-3-ylmethyl-isothiocyanat).

Weitere besonders bevorzugte Zusammensetzungen enthalten 0,01 - 5 Gew.-% 8-Hexadecen-1,16-dicarbonsäure und 0,0001 bis 5 Gew.-% *N*-Acetylindol-3-ylmethyl-isothiocyanat.

Weitere besonders bevorzugte Zusammensetzungen enthalten 0,01 - 5 Gew.-% 8-Hexadecen-1,16-dicarbonsäure und 0,0001 bis 5 Gew.-% Nasturtiin ((2-Phenylethyl)-isothiocyanat).

Weitere besonders bevorzugte Zusammensetzungen enthalten 0,01 - 5 Gew.-% 8-Hexadecen-1,16-dicarbonsäure und 0,0001 bis 5 Gew.-% Barbarin (2-Hydroxy-2-phenylethyl-isothiocyanat).

Weitere besonders bevorzugte Zusammensetzungen enthalten 0,01 - 5 Gew.-% 8-Hexadecen-1,16-dicarbonsäure und 0,0001 bis 5 Gew.-% Tropaeolin (Benzylisothiocyanat).

Weitere besonders bevorzugte Zusammensetzungen enthalten 0,01 - 5 Gew.-% 8-Hexadecen-1,16-dicarbonsäure und 0,0001 bis 5 Gew.-% Sinalbin (4-Hydroxybenzylisothiocyanat).

Weitere besonders bevorzugte erfindungsgemäße Zusammensetzungen enthalten 0,01 - 5 Gew.-% 8-Hexadecen-1,16-dicarbonsäure und 0,0001 bis 5 Gew.-% Capparin (Methylisothiocyanat).

Weitere besonders bevorzugte erfindungsgemäße Zusammensetzungen enthalten 0,01 - 5 Gew.-% 8-Hexadecen-1,16-dicarbonsäure und 0,0001 bis 5 Gew.-% Lepidin (Ethylisothiocyanat).

Weitere besonders bevorzugte erfindungsgemäße Zusammensetzungen enthalten 0,01 - 5 Gew.-% 8-Hexadecen-1,16-dicarbonsäure und 0,0001 bis 5 Gew.-% Putranjivin (Isopropylisothiocyanat).

Weitere besonders bevorzugte Zusammensetzungen enthalten 0,01 - 5 Gew.-% 8-Hexadecen-1,16-dicarbonsäure und 0,0001 bis 5 Gew.-% Jiaputin (2-Methylbutylisothiocyanat).

Weitere besonders bevorzugte Zusammensetzungen enthalten 0,01 - 5 Gew.-% 8-Hexadecen-1,16-dicarbonsäure und 0,0001 bis 5 Gew.-% Raphasatin (4-Methylthiobut-3-enyl-isothiocyanat).

Weitere besonders bevorzugte Zusammensetzungen enthalten 0,01 - 5 Gew.-% 8-Hexadecen-1,16-dicarbonsäure und 0,0001 bis 5 Gew.-% Cochlearin (1-Methylpropylisothiocyanat).

Weitere besonders bevorzugte Zusammensetzungen enthalten 0,01 - 5 Gew.-% 8-Hexadecen-1,16-dicarbonsäure und 0,0001 bis 5 Gew.-% Brassicanapin (4-Pentenylisothiocyanat).

Weitere besonders bevorzugte Zusammensetzungen enthalten 0,01 - 5 Gew.-% 8-Hexadecen-1,16-dicarbonsäure und 0,0001 bis 5 Gew.-% Napoleiferin (2-Hydroxypent-4-enylisothiocyanat).

Weitere besonders bevorzugte erfindungsgemäße Zusammensetzungen enthalten 0,01 - 5 Gew.-% 8-Hexadecen-1,16-dicarbonsäure und 0,0001 bis 5 Gew.-% Aubrietin.

Weitere besonders bevorzugte erfindungsgemäße Zusammensetzungen enthalten 0,01 - 5 Gew.-% 8-Hexadecen-1,16-dicarbonsäure und 0,0001 bis 5 Gew.-% Limnanthin.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1004.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1021.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1022.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1023.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1031.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1032.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1033.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1041.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1042.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1043.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1044.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1045.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1046.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1047.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1048.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1049.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1050.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1051.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1052.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1053.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1062.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1063.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1064.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1065.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1069.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1070.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1072.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1076.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1077.

Weitere besonders bevorzugte Zusammensetzungen enthalten 0,01 - 5 Gew.-% 8-Hexadecen-1,16-dicarbonsäure und 0,0001 bis 5 Gew.-% eines Isothiocyanats mit der Struktur GHP 1104.

Besonders bevorzugte erfindungsgemäße Zusammensetzungen enthalten 0,01 - 5 Gew.-% 7-Tetradecen-1,14-dicarbonsäure und 0,0001 bis 5 Gew.-% Sulforaphan.

Weitere besonders bevorzugte erfindungsgemäße Zusammensetzungen enthalten 0,01 - 5 Gew.-% 7-Tetradecen-1,14-dicarbonsäure und 0,0001 bis 5 Gew.-% Sulforaphen.

Weitere besonders bevorzugte erfindungsgemäße Zusammensetzungen enthalten 0,01 - 5 Gew.-% 7-Tetradecen-1,14-dicarbonsäure und 0,0001 bis 5 Gew.-% 6-Isothiocyanato-2-hexanon.

Weitere besonders bevorzugte Zusammensetzungen enthalten 0,01 - 5 Gew.-% 7-Tetradecen-1,14-dicarbonsäure und 0,0001 bis 5 Gew.-% exo-2-Acetyl-6-isothiocyanatonorbornan.

Weitere besonders bevorzugte Zusammensetzungen enthalten 0,01 - 5 Gew.-% 7-Tetradecen-1,14-dicarbonsäure und 0,0001 bis 5 Gew.-% exo-2-Isothiocyanato-6-methylsulfonylnorbornan.

Weitere besonders bevorzugte erfindungsgemäße Zusammensetzungen enthalten 0,01 - 5 Gew.-% 7-Tetradecen-1,14-dicarbonsäure und 0,0001 bis 5 Gew.-% 6-Isothiocyanato-2-hexanol.

Weitere besonders bevorzugte Zusammensetzungen enthalten 0,01 - 5 Gew.-% 7-Tetradecen-1,14-dicarbonsäure und 0,0001 bis 5 Gew.-% 1-Isothiocyanato-4-dimethylphosphonylbutan.

Weitere besonders bevorzugte Zusammensetzungen enthalten 0,01 - 5 Gew.-% 7-Tetradecen-1,14-dicarbonsäure und 0,0001 bis 5 Gew.-% exo-2-(1'-Hydroxyethyl)-5-isothiocyanatonorbornan.

Weitere besonders bevorzugte Zusammensetzungen enthalten 0,01 - 5 Gew.-% 7-Tetradecen-1,14-dicarbonsäure und 0,0001 bis 5 Gew.-% exo-2-Acetyl-5-isothiocyanatonorbornan.

Weitere besonders bevorzugte Zusammensetzungen enthalten 0,01 - 5 Gew.-% 7-Tetradecen-1,14-dicarbonsäure und 0,0001 bis 5 Gew.-% 1-Isothiocyanato-8-(methylsulfinyl)-octan (Hirsutin).

Weitere besonders bevorzugte Zusammensetzungen enthalten 0,01 - 5 Gew.-% 7-Tetradecen-1,14-dicarbonsäure und 0,0001 bis 5 Gew.-% 1-Isothiocyanato-5-methylsulfonylpentan.

Weitere besonders bevorzugte Zusammensetzungen enthalten 0,01 - 5 Gew.-% 7-Tetradecen-1,14-dicarbonsäure und 0,0001 bis 5 Gew.-% 1-Isothiocyanato-5-methylsulfinylpentan (Alyssin).

Weitere besonders bevorzugte Zusammensetzungen enthalten 0,01 - 5 Gew.-% 7-Tetradecen-1,14-dicarbonsäure und 0,0001 bis 5 Gew.-% 1-Isothiocyanato-4-methylsulfonylbutan (Erysolin).

Weitere besonders bevorzugte Zusammensetzungen enthalten 0,01 - 5 Gew.-% 7-Tetradecen-1,14-dicarbonsäure und 0,0001 bis 5 Gew.-% 1-Isothiocyanato-3-methylsulfinylpropan (Iberin).

Weitere besonders bevorzugte Zusammensetzungen enthalten 0,01 - 5 Gew.-% 7-Tetradecen-1,14-dicarbonsäure und 0,0001 bis 5 Gew.-% 1-Isothiocyanato-3-methylsulfonylpropan.

Weitere besonders bevorzugte Zusammensetzungen enthalten 0,01 - 5 Gew.-% 7-Tetradecen-1,14-dicarbonsäure und 0,0001 bis 5 Gew.-% cis-3-(Methylsulfonyl)cyclohexylisothiocyanat.

Weitere besonders bevorzugte Zusammensetzungen enthalten 0,01 - 5 Gew.-% 7-Tetradecen-1,14-dicarbonsäure und 0,0001 bis 5 Gew.-% cis-3-(Methylsulfonyl)cyclohexylmethylisothiocyanat.

Weitere besonders bevorzugte Zusammensetzungen enthalten 0,01 - 5 Gew.-% 7-Tetradecen-1,14-dicarbonsäure und 0,0001 bis 5 Gew.-% trans-3-(Methylsulfonyl)cyclohexylmethylisothio-cyanat.

Weitere besonders bevorzugte Zusammensetzungen enthalten 0,01 - 5 Gew.-% 7-Tetradecen-1,14-dicarbonsäure und 0,0001 bis 5 Gew.-% 2-Isothiocyanato-4-methylsulfonyl-ethylbutyrat.

Weitere besonders bevorzugte Zusammensetzungen enthalten 0,01 - 5 Gew.-% 7-Tetradecen-1,14-dicarbonsäure und 0,0001 bis 5 Gew.-% 9-Isothiocyanato-5-nonanon.

Weitere besonders bevorzugte Zusammensetzungen enthalten 0,01 - 5 Gew.-% 7-Tetradecen-1,14-dicarbonsäure und 0,0001 bis 5 Gew.-% 7-Isothiocyanato-3-heptanon (Norcappasalin).

Weitere besonders bevorzugte Zusammensetzungen enthalten 0,01 - 5 Gew.-% 7-Tetradecen-1,14-dicarbonsäure und 0,0001 bis 5 Gew.-% 8-Isothiocyanato-4-octanon (Cappasalin).

Weitere besonders bevorzugte Zusammensetzungen enthalten 0,01 - 5 Gew.-% 7-Tetradecen-1,14-dicarbonsäure und 0,0001 bis 5 Gew.-% 7-Isothiocyanato-4-heptanon (Capangulin).

Weitere besonders bevorzugte Zusammensetzungen enthalten 0,01 - 5 Gew.-% 7-Tetradecen-1,14-dicarbonsäure und 0,0001 bis 5 Gew.-% exo-2-Cyano-5-isothiocyanatonorbornan.

Weitere besonders bevorzugte erfindungsgemäße Zusammensetzungen enthalten 0,01 - 5 Gew.-% 7-Tetradecen-1,14-dicarbonsäure und 0,0001 bis 5 Gew.-% 1-Cyano-4-isothiocyanatobutan.

Weitere besonders bevorzugte erfindungsgemäße Zusammensetzungen enthalten 0,01 - 5 Gew.-% 7-Tetradecen-1,14-dicarbonsäure und 0,0001 bis 5 Gew.-% 5-Isothiocyanato-methylpentanoat.

Weitere besonders bevorzugte erfindungsgemäße Zusammensetzungen enthalten 0,01 - 5 Gew.-% 7-Tetradecen-1,14-dicarbonsäure und 0,0001 bis 5 Gew.-% 5-Isothiocyanatopentansäure.

Weitere besonders bevorzugte erfindungsgemäße Zusammensetzungen enthalten 0,01 - 5 Gew.-% 7-Tetradecen-1,14-dicarbonsäure und 0,0001 bis 5 Gew.-% 2-Isothiocyanato-ethylacetat.

Weitere besonders bevorzugte Zusammensetzungen enthalten 0,01 - 5 Gew.-% 7-Tetradecen-1,14-dicarbonsäure und 0,0001 bis 5 Gew.-% 4-Isothiocyanato-methylbutyrat (Erypestrin).

Weitere besonders bevorzugte erfindungsgemäße Zusammensetzungen enthalten 0,01 - 5 Gew.-% 7-Tetradecen-1,14-dicarbonsäure und 0,0001 bis 5 Gew.-% 4-Isothiocyanato-ethylbutyrat.

Weitere besonders bevorzugte erfindungsgemäße Zusammensetzungen enthalten 0,01 - 5 Gew.-% 7-Tetradecen-1,14-dicarbonsäure und 0,0001 bis 5 Gew.-% 1-Isothiocyanato-2-methoxyphenol.

Weitere besonders bevorzugte Zusammensetzungen enthalten 0,01 - 5 Gew.-% 7-Tetradecen-1,14-dicarbonsäure und 0,0001 bis 5 Gew.-% Erucin (4-Methylthiobutylisothiocyanat.

Weitere besonders bevorzugte Zusammensetzungen enthalten 0,01 - 5 Gew.-% 7-Tetradecen-1,14-dicarbonsäure und 0,0001 bis 5 Gew.-% Iberverin (3-Methylthiopropylisothiocyanat.

Weitere besonders bevorzugte Zusammensetzungen enthalten 0,01 - 5 Gew.-% 7-Tetradecen-1,14-dicarbonsäure und 0,0001 bis 5 Gew.-% Berteroin (5-Methylthiopentylisothiocyanat.

Weitere besonders bevorzugte Zusammensetzungen enthalten 0,01 - 5 Gew.-% 7-Tetradecen-1,14-dicarbonsäure und 0,0001 bis 5 Gew.-% Lesquerellin (6-Methylthiohexylisothiocyanat).

Weitere besonders bevorzugte Zusammensetzungen enthalten 0,01 - 5 Gew.-% 7-Tetradecen-1,14-dicarbonsäure und 0,0001 bis 5 Gew.-% Jirsutin (8-Methylthiooctylisothiocyanat).

Weitere besonders bevorzugte Zusammensetzungen enthalten 0,01 - 5 Gew.-% 7-Tetradecen-1,14-dicarbonsäure und 0,0001 bis 5 Gew.-% Arabin (9-Methylthiononylisothiocyanat).

Weitere besonders bevorzugte erfindungsgemäße Zusammensetzungen enthalten 0,01 - 5 Gew.-% 7-Tetradecen-1,14-dicarbonsäure und 0,0001 bis 5 Gew.-% Allylisothiocyanat.

Weitere besonders bevorzugte Zusammensetzungen enthalten 0,01 - 5 Gew.-% 7-Tetradecen-1,14-dicarbonsäure und 0,0001 bis 5 Gew.-% Brassicin (Indol-3-ylmethylisothiocyanat).

Weitere besonders bevorzugte Zusammensetzungen enthalten 0,01 - 5 Gew.-% 7-Tetradecen-1,14-dicarbonsäure und 0,0001 bis 5 Gew.-% Goitrin (2-Hydroxybut-3-enyl-isothiocyanat).

Weitere besonders bevorzugte erfindungsgemäße Zusammensetzungen enthalten 0,01 - 5 Gew.-% 7-Tetradecen-1,14-dicarbonsäure und 0,0001 bis 5 Gew.-% Napin (But-3-enyl-isothiocyanat).

Weitere besonders bevorzugte Zusammensetzungen enthalten 0,01 - 5 Gew.-% 7-Tetradecen-1,14-dicarbonsäure und 0,0001 bis 5 Gew.-% Neobrassicin (*N*-Methoxyindol-3-ylmethyl-isothiocyanat).

Weitere besonders bevorzugte Zusammensetzungen enthalten 0,01 - 5 Gew.-% 7-Tetradecen-1,14-dicarbonsäure und 0,0001 bis 5 Gew.-% *N*-Acetylindol-3-ylmethyl-isothiocyanat.

Weitere besonders bevorzugte Zusammensetzungen enthalten 0,01 - 5 Gew.-% 7-Tetradecen-1,14-dicarbonsäure und 0,0001 bis 5 Gew.-% Nasturtiin ((2-Phenylethyl)-isothiocyanat).

Weitere besonders bevorzugte Zusammensetzungen enthalten 0,01 - 5 Gew.-% 7-Tetradecen-1,14-dicarbonsäure und 0,0001 bis 5 Gew.-% Barbarin (2-Hydroxy-2-phenylethyl-isothiocyanat).

Weitere besonders bevorzugte erfindungsgemäße Zusammensetzungen enthalten 0,01 - 5 Gew.-% 7-Tetradecen-1,14-dicarbonsäure und 0,0001 bis 5 Gew.-% Tropaeolin (Benzylisothiocyanat).

Weitere besonders bevorzugte Zusammensetzungen enthalten 0,01 - 5 Gew.-% 7-Tetradecen-1,14-dicarbonsäure und 0,0001 bis 5 Gew.-% Sinalbin (4-Hydroxybenzylisothiocyanat).

Weitere besonders bevorzugte erfindungsgemäße Zusammensetzungen enthalten 0,01 - 5 Gew.-% 7-Tetradecen-1,14-dicarbonsäure und 0,0001 bis 5 Gew.-% Capparin (Methylisothiocyanat).

Weitere besonders bevorzugte erfindungsgemäße Zusammensetzungen enthalten 0,01 - 5 Gew.-% 7-Tetradecen-1,14-dicarbonsäure und 0,0001 bis 5 Gew.-% Lepidin (Ethylisothiocyanat).

Weitere besonders bevorzugte erfindungsgemäße Zusammensetzungen enthalten 0,01 - 5 Gew.-% 7-Tetradecen-1,14-dicarbonsäure und 0,0001 bis 5 Gew.-% Putranjivin (Isopropylisothiocyanat).

Weitere besonders bevorzugte Zusammensetzungen enthalten 0,01 - 5 Gew.-% 7-Tetradecen-1,14-dicarbonsäure und 0,0001 bis 5 Gew.-% Jiaputin (2-Methylbutylisothiocyanat).

Weitere besonders bevorzugte Zusammensetzungen enthalten 0,01 - 5 Gew.-% 7-Tetradecen-1,14-dicarbonsäure und 0,0001 bis 5 Gew.-% Raphasatin (4-Methylthiobut-3-enyl-isothiocyanat).

Weitere besonders bevorzugte Zusammensetzungen enthalten 0,01 - 5 Gew.-% 7-Tetradecen-1,14-dicarbonsäure und 0,0001 bis 5 Gew.-% Cochlearin (1-Methylpropylisothiocyanat).

Weitere besonders bevorzugte Zusammensetzungen enthalten 0,01 - 5 Gew.-% 7-Tetradecen-1,14-dicarbonsäure und 0,0001 bis 5 Gew.-% Brassicanapin (4-Pentenylisothiocyanat).

Weitere besonders bevorzugte Zusammensetzungen enthalten 0,01 - 5 Gew.-% 7-Tetradecen-1,14-dicarbonsäure und 0,0001 bis 5 Gew.-% Napoleiferin (2-Hydroxypent-4-enylisothiocyanat).

Weitere besonders bevorzugte erfindungsgemäße Zusammensetzungen enthalten 0,01 - 5 Gew.-% 7-Tetradecen-1,14-dicarbonsäure und 0,0001 bis 5 Gew.-% Aubrietin.

Weitere besonders bevorzugte erfindungsgemäße Zusammensetzungen enthalten 0,01 - 5 Gew.-% 7-Tetradecen-1,14-dicarbonsäure und 0,0001 bis 5 Gew.-% Limnanthin.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1004.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1021.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1022.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1023.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1031.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1032.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1033.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1041.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1042.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1043.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1044.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1045.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1046.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1047.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1048.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1049.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1050.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1051.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1052.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1053.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1062.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1063.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1064.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1065.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1069.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1070.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1072.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1076.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1077.

Weitere besonders bevorzugte Zusammensetzungen enthalten 0,01 - 5 Gew.-% 7-Tetradecen-1,14-dicarbonsäure und 0,0001 bis 5 Gew.-% eines Isothiocyanats mit der Struktur GHP 1104.

Besonders bevorzugte erfindungsgemäße Zusammensetzungen enthalten 0,01 - 5 Gew.-% 9-Octadecen-1,18-dicarbonsäure und 0,0001 bis 5 Gew.-% Sulforaphan.

Weitere besonders bevorzugte erfindungsgemäße Zusammensetzungen enthalten 0,01 - 5 Gew.-% 9-Octadecen-1,18-dicarbonsäure und 0,0001 bis 5 Gew.-% Sulforaphen.

Weitere besonders bevorzugte erfindungsgemäße Zusammensetzungen enthalten 0,01 - 5 Gew.-% 9-Octadecen-1,18-dicarbonsäure und 0,0001 bis 5 Gew.-% 6-Isothiocyanato-2-hexanon.

Weitere besonders bevorzugte Zusammensetzungen enthalten 0,01 - 5 Gew.-% 9-Octadecen-1,18-dicarbonsäure und 0,0001 bis 5 Gew.-% exo-2-Acetyl-6-isothiocyanatonorbornan.

Weitere besonders bevorzugte Zusammensetzungen enthalten 0,01 - 5 Gew.-% 9-Octadecen-1,18-dicarbonsäure und 0,0001 bis 5 Gew.-% exo-2-Isothiocyanato-6-methylsulfonylnorbornan.

Weitere besonders bevorzugte erfindungsgemäße Zusammensetzungen enthalten 0,01 - 5 Gew.-% 9-Octadecen-1,18-dicarbonsäure und 0,0001 bis 5 Gew.-% 6-Isothiocyanato-2-hexanol.

Weitere besonders bevorzugte Zusammensetzungen enthalten 0,01 - 5 Gew.-% 9-Octadecen-1,18-dicarbonsäure und 0,0001 bis 5 Gew.-% 1-Isothiocyanato-4-dimethylphosphonylbutan.

Weitere besonders bevorzugte Zusammensetzungen enthalten 0,01 - 5 Gew.-% 9-Octadecen-1,18-dicarbonsäure und 0,0001 bis 5 Gew.-% exo-2-(1'-Hydroxyethyl)-5-isothiocyanatonorbornan.

Weitere besonders bevorzugte Zusammensetzungen enthalten 0,01 - 5 Gew.-% 9-Octadecen-1,18-dicarbonsäure und 0,0001 bis 5 Gew.-% exo-2-Acetyl-5-isothiocyanatonorbornan.

Weitere besonders bevorzugte Zusammensetzungen enthalten 0,01 - 5 Gew.-% 9-Octadecen-1,18-dicarbonsäure und 0,0001 bis 5 Gew.-% 1-Isothiocyanato-8-(methylsulfinyl)-octan (Hirsutin).

Weitere besonders bevorzugte Zusammensetzungen enthalten 0,01 - 5 Gew.-% 9-Octadecen-1,18-dicarbonsäure und 0,0001 bis 5 Gew.-% 1-Isothiocyanato-5-methylsulfonylpentan.

Weitere besonders bevorzugte Zusammensetzungen enthalten 0,01 - 5 Gew.-% 9-Octadecen-1,18-dicarbonsäure und 0,0001 bis 5 Gew.-% 1-Isothiocyanato-5-methylsulfinylpentan (Alyssin).

Weitere besonders bevorzugte Zusammensetzungen enthalten 0,01 - 5 Gew.-% 9-Octadecen-1,18-dicarbonsäure und 0,0001 bis 5 Gew.-% 1-Isothiocyanato-4-methylsulfonylbutan (Erysolin).

Weitere besonders bevorzugte Zusammensetzungen enthalten 0,01 - 5 Gew.-% 9-Octadecen-1,18-dicarbonsäure und 0,0001 bis 5 Gew.-% 1-Isothiocyanato-3-methylsulfinylpropan (Iberin).

Weitere besonders bevorzugte Zusammensetzungen enthalten 0,01 - 5 Gew.-% 9-Octadecen-1,18-dicarbonsäure und 0,0001 bis 5 Gew.-% 1-Isothiocyanato-3-methylsulfonylpropan.

Weitere besonders bevorzugte Zusammensetzungen enthalten 0,01 - 5 Gew.-% 9-Octadecen-1,18-dicarbonsäure und 0,0001 bis 5 Gew.-% cis-3-(Methylsulfonyl)cyclohexylisothiocyanat.

Weitere besonders bevorzugte Zusammensetzungen enthalten 0,01 - 5 Gew.-% 9-Octadecen-1,18-dicarbonsäure und 0,0001 bis 5 Gew.-% cis-3-(Methylsulfonyl)cyclohexylmethylisothiocyanat.

Weitere besonders bevorzugte Zusammensetzungen enthalten 0,01 - 5 Gew.-% 9-Octadecen-1,18-dicarbonsäure und 0,0001 bis 5 Gew.-% trans-3-(Methylsulfonyl)cyclohexylmethylisothio-cyanat.

Weitere besonders bevorzugte Zusammensetzungen enthalten 0,01 - 5 Gew.-% 9-Octadecen-1,18-dicarbonsäure und 0,0001 bis 5 Gew.-% 2-Isothiocyanato-4-methylsulfonyl-ethylbutyrat.

Weitere besonders bevorzugte erfindungsgemäße Zusammensetzungen enthalten 0,01 - 5 Gew.-% 9-Octadecen-1,18-dicarbonsäure und 0,0001 bis 5 Gew.-% 9-Isothiocyanato-5-nonanon.

Weitere besonders bevorzugte Zusammensetzungen enthalten 0,01 - 5 Gew.-% 9-Octadecen-1,18-dicarbonsäure und 0,0001 bis 5 Gew.-% 7-Isothiocyanato-3-heptanon (Norcappasalin).

Weitere besonders bevorzugte Zusammensetzungen enthalten 0,01 - 5 Gew.-% 9-Octadecen-1,18-dicarbonsäure und 0,0001 bis 5 Gew.-% 8-Isothiocyanato-4-octanon (Cappasalin).

Weitere besonders bevorzugte Zusammensetzungen enthalten 0,01 - 5 Gew.-% 9-Octadecen-1,18-dicarbonsäure und 0,0001 bis 5 Gew.-% 7-Isothiocyanato-4-heptanon (Capangulin).

Weitere besonders bevorzugte Zusammensetzungen enthalten 0,01 - 5 Gew.-% 9-Octadecen-1,18-dicarbonsäure und 0,0001 bis 5 Gew.-% exo-2-Cyano-5-isothiocyanatonorbornan.

Weitere besonders bevorzugte erfindungsgemäße Zusammensetzungen enthalten 0,01 - 5 Gew.-% 9-Octadecen-1,18-dicarbonsäure und 0,0001 bis 5 Gew.-% 1-Cyano-4-isothiocyanatobutan.

Weitere besonders bevorzugte erfindungsgemäße Zusammensetzungen enthalten 0,01 - 5 Gew.-% 9-Octadecen-1,18-dicarbonsäure und 0,0001 bis 5 Gew.-% 5-Isothiocyanato-methylpentanoat.

Weitere besonders bevorzugte erfindungsgemäße Zusammensetzungen enthalten 0,01 - 5 Gew.-% 9-Octadecen-1,18-dicarbonsäure und 0,0001 bis 5 Gew.-% 5-Isothiocyanatopentansäure.

Weitere besonders bevorzugte erfindungsgemäße Zusammensetzungen enthalten 0,01 - 5 Gew.-% 9-Octadecen-1,18-dicarbonsäure und 0,0001 bis 5 Gew.-% 2-Isothiocyanato-ethylacetat.

Weitere besonders bevorzugte Zusammensetzungen enthalten 0,01 - 5 Gew.-% 9-Octadecen-1,18-dicarbonsäure und 0,0001 bis 5 Gew.-% 4-Isothiocyanato-methylbutyrat (Erypestrin).

Weitere besonders bevorzugte erfindungsgemäße Zusammensetzungen enthalten 0,01 - 5 Gew.-% 9-Octadecen-1,18-dicarbonsäure und 0,0001 bis 5 Gew.-% 4-Isothiocyanato-ethylbutyrat.

Weitere besonders bevorzugte erfindungsgemäße Zusammensetzungen enthalten 0,01 - 5 Gew.-% 9-Octadecen-1,18-dicarbonsäure und 0,0001 bis 5 Gew.-% 1-Isothiocyanato-2-methoxyphenol.

Weitere besonders bevorzugte Zusammensetzungen enthalten 0,01 - 5 Gew.-% 9-Octadecen-1,18-dicarbonsäure und 0,0001 bis 5 Gew.-% Erucin (4-Methylthiobutylisothiocyanat.

Weitere besonders bevorzugte Zusammensetzungen enthalten 0,01 - 5 Gew.-% 9-Octadecen-1,18-dicarbonsäure und 0,0001 bis 5 Gew.-% Iberverin (3-Methylthiopropylisothiocyanat.

Weitere besonders bevorzugte Zusammensetzungen enthalten 0,01 - 5 Gew.-% 9-Octadecen-1,18-dicarbonsäure und 0,0001 bis 5 Gew.-% Berteroin (5-Methylthiopentylisothiocyanat.

Weitere besonders bevorzugte Zusammensetzungen enthalten 0,01 - 5 Gew.-% 9-Octadecen-1,18-dicarbonsäure und 0,0001 bis 5 Gew.-% Lesquerellin (6-Methylthiohexylisothiocyanat).

Weitere besonders bevorzugte Zusammensetzungen enthalten 0,01 - 5 Gew.-% 9-Octadecen-1,18-dicarbonsäure und 0,0001 bis 5 Gew.-% Jirsutin (8-Methylthiooctylisothiocyanat).

Weitere besonders bevorzugte Zusammensetzungen enthalten 0,01 - 5 Gew.-% 9-Octadecen-1,18-dicarbonsäure und 0,0001 bis 5 Gew.-% Arabin (9-Methylthiononylisothiocyanat).

Weitere besonders bevorzugte erfindungsgemäße Zusammensetzungen enthalten 0,01 - 5 Gew.-% 9-Octadecen-1,18-dicarbonsäure und 0,0001 bis 5 Gew.-% Allylisothiocyanat.

Weitere besonders bevorzugte Zusammensetzungen enthalten 0,01 - 5 Gew.-% 9-Octadecen-1,18-dicarbonsäure und 0,0001 bis 5 Gew.-% Brassicin (Indol-3-ylmethylisothiocyanat).

Weitere besonders bevorzugte Zusammensetzungen enthalten 0,01 - 5 Gew.-% 9-Octadecen-1,18-dicarbonsäure und 0,0001 bis 5 Gew.-% Goitrin (2-Hydroxybut-3-enyl-isothiocyanat).

Weitere besonders bevorzugte erfindungsgemäße Zusammensetzungen enthalten 0,01 - 5 Gew.-% 9-Octadecen-1,18-dicarbonsäure und 0,0001 bis 5 Gew.-% Napin (But-3-enyl-isothiocyanat).

Weitere besonders bevorzugte Zusammensetzungen enthalten 0,01 - 5 Gew.-% 9-Octadecen-1,18-dicarbonsäure und 0,0001 bis 5 Gew.-% Neobrassicin (*N*-Methoxyindol-3-ylmethyl-isothiocyanat).

Weitere besonders bevorzugte Zusammensetzungen enthalten 0,01 - 5 Gew.-% 9-Octadecen-1,18-dicarbonsäure und 0,0001 bis 5 Gew.-% *N*-Acetylindol-3-ylmethyl-isothiocyanat.

Weitere besonders bevorzugte Zusammensetzungen enthalten 0,01 - 5 Gew.-% 9-Octadecen-1,18-dicarbonsäure und 0,0001 bis 5 Gew.-% Nasturtiin ((2-Phenylethyl)-isothiocyanat).

Weitere besonders bevorzugte Zusammensetzungen enthalten 0,01 - 5 Gew.-% 9-Octadecen-1,18-dicarbonsäure und 0,0001 bis 5 Gew.-% Barbarin (2-Hydroxy-2-phenylethyl-isothiocyanat).

Weitere besonders bevorzugte erfindungsgemäße Zusammensetzungen enthalten 0,01 - 5 Gew.-% 9-Octadecen-1,18-dicarbonsäure und 0,0001 bis 5 Gew.-% Tropaeolin (Benzylisothiocyanat).

Weitere besonders bevorzugte Zusammensetzungen enthalten 0,01 - 5 Gew.-% 9-Octadecen-1,18-dicarbonsäure und 0,0001 bis 5 Gew.-% Sinalbin (4-Hydroxybenzylisothiocyanat).

Weitere besonders bevorzugte erfindungsgemäße Zusammensetzungen enthalten 0,01 - 5 Gew.-% 9-Octadecen-1,18-dicarbonsäure und 0,0001 bis 5 Gew.-% Capparin (Methylisothiocyanat).

Weitere besonders bevorzugte erfindungsgemäße Zusammensetzungen enthalten 0,01 - 5 Gew.-% 9-Octadecen-1,18-dicarbonsäure und 0,0001 bis 5 Gew.-% Lepidin (Ethylisothiocyanat).

Weitere besonders bevorzugte erfindungsgemäße Zusammensetzungen enthalten 0,01 - 5 Gew.-% 9-Octadecen-1,18-dicarbonsäure und 0,0001 bis 5 Gew.-% Putranjivin (Isopropylisothiocyanat).

Weitere besonders bevorzugte Zusammensetzungen enthalten 0,01 - 5 Gew.-% 9-Octadecen-1,18-dicarbonsäure und 0,0001 bis 5 Gew.-% Jiaputin (2-Methylbutylisothiocyanat).

Weitere besonders bevorzugte Zusammensetzungen enthalten 0,01 - 5 Gew.-% 9-Octadecen-1,18-dicarbonsäure und 0,0001 bis 5 Gew.-% Raphasatin (4-Methylthiobut-3-enyl-isothiocyanat).

Weitere besonders bevorzugte Zusammensetzungen enthalten 0,01 - 5 Gew.-% 9-Octadecen-1,18-dicarbonsäure und 0,0001 bis 5 Gew.-% Cochlearin (1-Methylpropylisothiocyanat).

Weitere besonders bevorzugte Zusammensetzungen enthalten 0,01 - 5 Gew.-% 9-Octadecen-1,18-dicarbonsäure und 0,0001 bis 5 Gew.-% Brassicanapin (4-Pentenylisothiocyanat).

Weitere besonders bevorzugte Zusammensetzungen enthalten 0,01 - 5 Gew.-% 9-Octadecen-1,18-dicarbonsäure und 0,0001 bis 5 Gew.-% Napoleiferin (2-Hydroxypent-4-enylisothiocyanat).

Weitere besonders bevorzugte erfindungsgemäße Zusammensetzungen enthalten 0,01 - 5 Gew.-% 9-Octadecen-1,18-dicarbonsäure und 0,0001 bis 5 Gew.-% Aubrietin.

Weitere besonders bevorzugte erfindungsgemäße Zusammensetzungen enthalten 0,01 - 5 Gew.-% 9-Octadecen-1,18-dicarbonsäure und 0,0001 bis 5 Gew.-% Limnanthin.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1004.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1021.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1022.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1023.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1031.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1032.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1033.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1041.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1042.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1043.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1044.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1045.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1046.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1047.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1048.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1049.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1050.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1051.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1052.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1053.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1062.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1063.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1064.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1065.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1069.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1070.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1072.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1076.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1077.

Weitere besonders bevorzugte Zusammensetzungen enthalten 0,01 - 5 Gew.-% 9-Octadecen-1,18-dicarbonsäure und 0,0001 bis 5 Gew.-% eines Isothiocyanats mit der Struktur GHP 1104.

Besonders bevorzugte erfindungsgemäße Zusammensetzungen enthalten 0,01 - 5 Gew.-% 6-Dodecen-1,12-dicarbonsäure und 0,0001 bis 5 Gew.-% Sulforaphan.

Weitere besonders bevorzugte erfindungsgemäße Zusammensetzungen enthalten 0,01 - 5 Gew.-% 6-Dodecen-1,12-dicarbonsäure und 0,0001 bis 5 Gew.-% Sulforaphen.

Weitere besonders bevorzugte erfindungsgemäße Zusammensetzungen enthalten 0,01 - 5 Gew.-% 6-Dodecen-1,12-dicarbonsäure und 0,0001 bis 5 Gew.-% 6-Isothiocyanato-2-hexanon.

Weitere besonders bevorzugte Zusammensetzungen enthalten 0,01 - 5 Gew.-% 6-Dodecen-1,12-dicarbonsäure und 0,0001 bis 5 Gew.-% exo-2-Acetyl-6-isothiocyanatonorbornan.

Weitere besonders bevorzugte erfindungsgemäße Zusammensetzungen enthalten 0,01 - 5 Gew.-% 6-Dodecen-1,12-dicarbonsäure und 0,0001 bis 5 Gew.-% exo-2-Isothiocyanato-6-methylsulfonylnorbornan.

Weitere besonders bevorzugte erfindungsgemäße Zusammensetzungen enthalten 0,01 - 5 Gew.-% 6-Dodecen-1,12-dicarbonsäure und 0,0001 bis 5 Gew.-% 6-Isothiocyanato-2-hexanol.

Weitere besonders bevorzugte Zusammensetzungen enthalten 0,01 - 5 Gew.-% 6-Dodecen-1,12-dicarbonsäure und 0,0001 bis 5 Gew.-% 1-Isothiocyanato-4-dimethylphosphonylbutan.

Weitere besonders bevorzugte Zusammensetzungen enthalten 0,01 - 5 Gew.-% 6-Dodecen-1,12-dicarbonsäure und 0,0001 bis 5 Gew.-% exo-2-(1'-Hydroxyethyl)-5-isothiocyanatonorbornan.

Weitere besonders bevorzugte Zusammensetzungen enthalten 0,01 - 5 Gew.-% 6-Dodecen-1,12-dicarbonsäure und 0,0001 bis 5 Gew.-% exo-2-Acetyl-5-isothiocyanatonorbornan.

Weitere besonders bevorzugte Zusammensetzungen enthalten 0,01 - 5 Gew.-% 6-Dodecen-1,12-dicarbonsäure und 0,0001 bis 5 Gew.-% 1-Isothiocyanato-8-(methylsulfinyl)-octan (Hirsutin).

Weitere besonders bevorzugte Zusammensetzungen enthalten 0,01 - 5 Gew.-% 6-Dodecen-1,12-dicarbonsäure und 0,0001 bis 5 Gew.-% 1-Isothiocyanato-5-methylsulfonylpentan.

Weitere besonders bevorzugte Zusammensetzungen enthalten 0,01 - 5 Gew.-% 6-Dodecen-1,12-dicarbonsäure und 0,0001 bis 5 Gew.-% 1-Isothiocyanato-5-methylsulfinylpentan (Alyssin).

Weitere besonders bevorzugte Zusammensetzungen enthalten 0,01 - 5 Gew.-% 6-Dodecen-1,12-dicarbonsäure und 0,0001 bis 5 Gew.-% 1-Isothiocyanato-4-methylsulfonylbutan (Erysolin).

Weitere besonders bevorzugte Zusammensetzungen enthalten 0,01 - 5 Gew.-% 6-Dodecen-1,12-dicarbonsäure und 0,0001 bis 5 Gew.-% 1-Isothiocyanato-3-methylsulfinylpropan (Iberin).

Weitere besonders bevorzugte Zusammensetzungen enthalten 0,01 - 5 Gew.-% 6-Dodecen-1,12-dicarbonsäure und 0,0001 bis 5 Gew.-% 1-Isothiocyanato-3-methylsulfonylpropan.

Weitere besonders bevorzugte Zusammensetzungen enthalten 0,01 - 5 Gew.-% 6-Dodecen-1,12-dicarbonsäure und 0,0001 bis 5 Gew.-% cis-3-(Methylsulfonyl)cyclohexylisothiocyanat.

Weitere besonders bevorzugte Zusammensetzungen enthalten 0,01 - 5 Gew.-% 6-Dodecen-1,12-dicarbonsäure und 0,0001 bis 5 Gew.-% cis-3-(Methylsulfonyl)cyclohexylmethylisothiocyanat.

Weitere besonders bevorzugte Zusammensetzungen enthalten 0,01 - 5 Gew.-% 6-Dodecen-1,12-dicarbonsäure und 0,0001 bis 5 Gew.-% trans-3-(Methylsulfonyl)cyclohexylmethylisothiocyanat.

Weitere besonders bevorzugte Zusammensetzungen enthalten 0,01 - 5 Gew.-% 6-Dodecen-1,12-dicarbonsäure und 0,0001 bis 5 Gew.-% 2-Isothiocyanato-4-methylsulfonyl-ethylbutyrat.

Weitere besonders bevorzugte erfindungsgemäße Zusammensetzungen enthalten 0,01 - 5 Gew.-% 6-Dodecen-1,12-dicarbonsäure und 0,0001 bis 5 Gew.-% 9-Isothiocyanato-5-nonanon.

Weitere besonders bevorzugte Zusammensetzungen enthalten 0,01 - 5 Gew.-% 6-Dodecen-1,12-dicarbonsäure und 0,0001 bis 5 Gew.-% 7-Isothiocyanato-3-heptanon (Norcappasalin).

Weitere besonders bevorzugte Zusammensetzungen enthalten 0,01 - 5 Gew.-% 6-Dodecen-1,12-dicarbonsäure und 0,0001 bis 5 Gew.-% 8-Isothiocyanato-4-octanon (Cappasalin).

Weitere besonders bevorzugte Zusammensetzungen enthalten 0,01 - 5 Gew.-% 6-Dodecen-1,12-dicarbonsäure und 0,0001 bis 5 Gew.-% 7-Isothiocyanato-4-heptanon (Capangulin).

Weitere besonders bevorzugte Zusammensetzungen enthalten 0,01 - 5 Gew.-% 6-Dodecen-1,12-dicarbonsäure und 0,0001 bis 5 Gew.-% exo-2-Cyano-5-isothiocyanatonorbornan.

Weitere besonders bevorzugte erfindungsgemäße Zusammensetzungen enthalten 0,01 - 5 Gew.-% 6-Dodecen-1,12-dicarbonsäure und 0,0001 bis 5 Gew.-% 1-Cyano-4-isothiocyanatobutan.

Weitere besonders bevorzugte erfindungsgemäße Zusammensetzungen enthalten 0,01 - 5 Gew.-% 6-Dodecen-1,12-dicarbonsäure und 0,0001 bis 5 Gew.-% 5-Isothiocyanato-methylpentanoat.

Weitere besonders bevorzugte erfindungsgemäße Zusammensetzungen enthalten 0,01 - 5 Gew.-% 6-Dodecen-1,12-dicarbonsäure und 0,0001 bis 5 Gew.-% 5-Isothiocyanatopentansäure.

Weitere besonders bevorzugte erfindungsgemäße Zusammensetzungen enthalten 0,01 - 5 Gew.-% 6-Dodecen-1,12-dicarbonsäure und 0,0001 bis 5 Gew.-% 2-Isothiocyanato-ethylacetat.

Weitere besonders bevorzugte Zusammensetzungen enthalten 0,01 - 5 Gew.-% 6-Dodecen-1,12-dicarbonsäure und 0,0001 bis 5 Gew.-% 4-Isothiocyanato-methylbutyrat (Erypestrin).

Weitere besonders bevorzugte erfindungsgemäße Zusammensetzungen enthalten 0,01 - 5 Gew.-% 6-Dodecen-1,12-dicarbonsäure und 0,0001 bis 5 Gew.-% 4-Isothiocyanato-ethylbutyrat.

Weitere besonders bevorzugte erfindungsgemäße Zusammensetzungen enthalten 0,01 - 5 Gew.-% 6-Dodecen-1,12-dicarbonsäure und 0,0001 bis 5 Gew.-% 1-Isothiocyanato-2-methoxyphenol.

Weitere besonders bevorzugte erfindungsgemäße Zusammensetzungen enthalten 0,01 - 5 Gew.-% 6-Dodecen-1,12-dicarbonsäure und 0,0001 bis 5 Gew.-% Erucin (4-Methylthiobutylisothiocyanat.

Weitere besonders bevorzugte Zusammensetzungen enthalten 0,01 - 5 Gew.-% 6-Dodecen-1,12-dicarbonsäure und 0,0001 bis 5 Gew.-% Iberverin (3-Methylthiopropylisothiocyanat.

Weitere besonders bevorzugte Zusammensetzungen enthalten 0,01 - 5 Gew.-% 6-Dodecen-1,12-dicarbonsäure und 0,0001 bis 5 Gew.-% Berteroin (5-Methylthiopentylisothiocyanat.

Weitere besonders bevorzugte Zusammensetzungen enthalten 0,01 - 5 Gew.-% 6-Dodecen-1,12-dicarbonsäure und 0,0001 bis 5 Gew.-% Lesquerellin (6-Methylthiohexylisothiocyanat).

Weitere besonders bevorzugte Zusammensetzungen enthalten 0,01 - 5 Gew.-% 6-Dodecen-1,12-dicarbonsäure und 0,0001 bis 5 Gew.-% Jirsutin (8-Methylthiooctylisothiocyanat).

Weitere besonders bevorzugte Zusammensetzungen enthalten 0,01 - 5 Gew.-% 6-Dodecen-1,12-dicarbonsäure und 0,0001 bis 5 Gew.-% Arabin (9-Methylthiononylisothiocyanat).

Weitere besonders bevorzugte erfindungsgemäße Zusammensetzungen enthalten 0,01 - 5 Gew.-% 6-Dodecen-1,12-dicarbonsäure und 0,0001 bis 5 Gew.-% Allylisothiocyanat.

Weitere besonders bevorzugte Zusammensetzungen enthalten 0,01 - 5 Gew.-% 6-Dodecen-1,12-dicarbonsäure und 0,0001 bis 5 Gew.-% Brassicin (Indol-3-ylmethylisothiocyanat).

Weitere besonders bevorzugte Zusammensetzungen enthalten 0,01 - 5 Gew.-% 6-Dodecen-1,12-dicarbonsäure und 0,0001 bis 5 Gew.-% Goitrin (2-Hydroxybut-3-enyl-isothiocyanat).

Weitere besonders bevorzugte erfindungsgemäße Zusammensetzungen enthalten 0,01 - 5 Gew.-% 6-Dodecen-1,12-dicarbonsäure und 0,0001 bis 5 Gew.-% Napin (But-3-enyl-isothiocyanat).

Weitere besonders bevorzugte Zusammensetzungen enthalten 0,01 - 5 Gew.-% 6-Dodecen-1,12-dicarbonsäure und 0,0001 bis 5 Gew.-% Neobrassicin (*N*-Methoxyindol-3-ylmethyl-isothiocyanat).

Weitere besonders bevorzugte Zusammensetzungen enthalten 0,01 - 5 Gew.-% 6-Dodecen-1,12-dicarbonsäure und 0,0001 bis 5 Gew.-% *N*-Acetylindol-3-ylmethyl-isothiocyanat.

Weitere besonders bevorzugte Zusammensetzungen enthalten 0,01 - 5 Gew.-% 6-Dodecen-1,12-dicarbonsäure und 0,0001 bis 5 Gew.-% Nasturtiin ((2-Phenylethyl)-isothiocyanat).

Weitere besonders bevorzugte Zusammensetzungen enthalten 0,01 - 5 Gew.-% 6-Dodecen-1,12-dicarbonsäure und 0,0001 bis 5 Gew.-% Barbarin (2-Hydroxy-2-phenylethyl-isothiocyanat).

Weitere besonders bevorzugte erfindungsgemäße Zusammensetzungen enthalten 0,01 - 5 Gew.-% 6-Dodecen-1,12-dicarbonsäure und 0,0001 bis 5 Gew.-% Tropaeolin (Benzylisothiocyanat).

Weitere besonders bevorzugte Zusammensetzungen enthalten 0,01 - 5 Gew.-% 6-Dodecen-1,12-dicarbonsäure und 0,0001 bis 5 Gew.-% Sinalbin (4-Hydroxybenzylisothiocyanat).

Weitere besonders bevorzugte erfindungsgemäße Zusammensetzungen enthalten 0,01 - 5 Gew.-% 6-Dodecen-1,12-dicarbonsäure und 0,0001 bis 5 Gew.-% Capparin (Methylisothiocyanat).

Weitere besonders bevorzugte erfindungsgemäße Zusammensetzungen enthalten 0,01 - 5 Gew.-% 6-Dodecen-1,12-dicarbonsäure und 0,0001 bis 5 Gew.-% Lepidin (Ethylisothiocyanat).

Weitere besonders bevorzugte erfindungsgemäße Zusammensetzungen enthalten 0,01 - 5 Gew.-% 6-Dodecen-1,12-dicarbonsäure und 0,0001 bis 5 Gew.-% Putranjivin (Isopropylisothiocyanat).

Weitere besonders bevorzugte erfindungsgemäße Zusammensetzungen enthalten 0,01 - 5 Gew.-% 6-Dodecen-1,12-dicarbonsäure und 0,0001 bis 5 Gew.-% Jiaputin (2-Methylbutylisothiocyanat).

Weitere besonders bevorzugte Zusammensetzungen enthalten 0,01 - 5 Gew.-% 6-Dodecen-1,12-dicarbonsäure und 0,0001 bis 5 Gew.-% Raphasatin (4-Methylthiobut-3-enyl-isothiocyanat).

Weitere besonders bevorzugte Zusammensetzungen enthalten 0,01 - 5 Gew.-% 6-Dodecen-1,12-dicarbonsäure und 0,0001 bis 5 Gew.-% Cochlearin (1-Methylpropylisothiocyanat).

Weitere besonders bevorzugte Zusammensetzungen enthalten 0,01 - 5 Gew.-% 6-Dodecen-1,12-dicarbonsäure und 0,0001 bis 5 Gew.-% Brassicanapin (4-Pentenylisothiocyanat).

Weitere besonders bevorzugte Zusammensetzungen enthalten 0,01 - 5 Gew.-% 6-Dodecen-1,12-dicarbonsäure und 0,0001 bis 5 Gew.-% Napoleiferin (2-Hydroxypent-4-enylisothiocyanat).

Weitere besonders bevorzugte Zusammensetzungen enthalten 0,01 - 5 Gew.-% 6-Dodecen-1,12-dicarbonsäure und 0,0001 bis 5 Gew.-% Aubrietin.

Weitere besonders bevorzugte Zusammensetzungen enthalten 0,01 - 5 Gew.-% 6-Dodecen-1,12-dicarbonsäure und 0,0001 bis 5 Gew.-% Limnanthin.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1004.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1021.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1022.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1023.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1031.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1032.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1033.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1041.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1042.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1043.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1044.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1045.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1046.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1047.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1048.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1049.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1050.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1051.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1052.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1053.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1062.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1063.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1064.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1065.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1069.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1070.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1072.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1076.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1077.

Weitere besonders bevorzugte Zusammensetzungen enthalten 0,01 - 5 Gew.-% 6-Dodecen-1,12-dicarbonsäure und 0,0001 bis 5 Gew.-% eines Isothiocyanats mit der Struktur GHP 1104.

Besonders bevorzugte erfindungsgemäße Zusammensetzungen enthalten 0,01 - 5 Gew.-% 5-Decen-1,10-dicarbonsäure und 0,0001 bis 5 Gew.-% Sulforaphan.

Weitere besonders bevorzugte erfindungsgemäße Zusammensetzungen enthalten 0,01 - 5 Gew.-% 5-Decen-1,10-dicarbonsäure und 0,0001 bis 5 Gew.-% Sulforaphen.

Weitere besonders bevorzugte erfindungsgemäße Zusammensetzungen enthalten 0,01 - 5 Gew.-% 5-Decen-1,10-dicarbonsäure und 0,0001 bis 5 Gew.-% 6-Isothiocyanato-2-hexanon.

Weitere besonders bevorzugte erfindungsgemäße Zusammensetzungen enthalten 0,01 - 5 Gew.-% 5-Decen-1,10-dicarbonsäure und 0,0001 bis 5 Gew.-% exo-2-Acetyl-6-isothiocyanatonorbornan. Weitere besonders bevorzugte Zusammensetzungen enthalten 0,01 - 5 Gew.-% 5-Decen-1,10-dicarbonsäure und 0,0001 bis 5 Gew.-% exo-2-Isothiocyanato-6-methylsulfonylnorbornan.

Weitere besonders bevorzugte erfindungsgemäße Zusammensetzungen enthalten 0,01 - 5 Gew.-% 5-Decen-1,10-dicarbonsäure und 0,0001 bis 5 Gew.-% 6-Isothiocyanato-2-hexanol.

Weitere besonders bevorzugte Zusammensetzungen enthalten 0,01 - 5 Gew.-% 5-Decen-1,10-dicarbonsäure und 0,0001 bis 5 Gew.-% 1-Isothiocyanato-4-dimethylphosphonylbutan.

Weitere besonders bevorzugte Zusammensetzungen enthalten 0,01 - 5 Gew.-% 5-Decen-1,10-dicarbonsäure und 0,0001 bis 5 Gew.-% exo-2-(1'-Hydroxyethyl)-5-isothiocyanatonorbornan.

Weitere besonders bevorzugte erfindungsgemäße Zusammensetzungen enthalten 0,01 - 5 Gew.-% 5-Decen-1,10-dicarbonsäure und 0,0001 bis 5 Gew.-% exo-2-Acetyl-5-isothiocyanatonorbornan. Weitere besonders bevorzugte Zusammensetzungen enthalten 0,01 - 5 Gew.-% 5-Decen-1,10-dicarbonsäure und 0,0001 bis 5 Gew.-% 1-Isothiocyanato-8-(methylsulfinyl)-octan (Hirsutin).

Weitere besonders bevorzugte erfindungsgemäße Zusammensetzungen enthalten 0,01 - 5 Gew.-% 5-Decen-1,10-dicarbonsäure und 0,0001 bis 5 Gew.-% 1-Isothiocyanato-5-methylsulfonylpentan. Weitere besonders bevorzugte Zusammensetzungen enthalten 0,01 - 5 Gew.-% 5-Decen-1,10-dicarbonsäure und 0,0001 bis 5 Gew.-% 1-Isothiocyanato-5-methylsulfinylpentan (Alyssin).

Weitere besonders bevorzugte Zusammensetzungen enthalten 0,01 - 5 Gew.-% 5-Decen-1,10-dicarbonsäure und 0,0001 bis 5 Gew.-% 1-Isothiocyanato-4-methylsulfonylbutan (Erysolin).

Weitere besonders bevorzugte Zusammensetzungen enthalten 0,01 - 5 Gew.-% 5-Decen-1,10-dicarbonsäure und 0,0001 bis 5 Gew.-% 1-Isothiocyanato-3-methylsulfinylpropan (Iberin).

Weitere besonders bevorzugte Zusammensetzungen enthalten 0,01 - 5 Gew.-% 5-Decen-1,10-dicarbonsäure und 0,0001 bis 5 Gew.-% 1-Isothiocyanato-3-methylsulfonylpropan.

Weitere besonders bevorzugte Zusammensetzungen enthalten 0,01 - 5 Gew.-% 5-Decen-1,10-dicarbonsäure und 0,0001 bis 5 Gew.-% cis-3-(Methylsulfonyl)cyclohexylisothiocyanat.

Weitere besonders bevorzugte Zusammensetzungen enthalten 0,01 - 5 Gew.-% 5-Decen-1,10-dicarbonsäure und 0,0001 bis 5 Gew.-% cis-3-(Methylsulfonyl)cyclohexylmethylisothiocyanat.

Weitere besonders bevorzugte Zusammensetzungen enthalten 0,01 - 5 Gew.-% 5-Decen-1,10-dicarbonsäure und 0,0001 bis 5 Gew.-% trans-3-(Methylsulfonyl)cyclohexylmethylisothiocyanat.

Weitere besonders bevorzugte Zusammensetzungen enthalten 0,01 - 5 Gew.-% 5-Decen-1,10-dicarbonsäure und 0,0001 bis 5 Gew.-% 2-Isothiocyanato-4-methylsulfonyl-ethylbutyrat.

Weitere besonders bevorzugte erfindungsgemäße Zusammensetzungen enthalten 0,01 - 5 Gew.-% 5-Decen-1,10-dicarbonsäure und 0,0001 bis 5 Gew.-% 9-Isothiocyanato-5-nonanon.

Weitere besonders bevorzugte Zusammensetzungen enthalten 0,01 - 5 Gew.-% 5-Decen-1,10-dicarbonsäure und 0,0001 bis 5 Gew.-% 7-Isothiocyanato-3-heptanon (Norcappasalin).

Weitere besonders bevorzugte Zusammensetzungen enthalten 0,01 - 5 Gew.-% 5-Decen-1,10-dicarbonsäure und 0,0001 bis 5 Gew.-% 8-Isothiocyanato-4-octanon (Cappasalin).

Weitere besonders bevorzugte Zusammensetzungen enthalten 0,01 - 5 Gew.-% 5-Decen-1,10-dicarbonsäure und 0,0001 bis 5 Gew.-% 7-Isothiocyanato-4-heptanon (Capangulin).

Weitere besonders bevorzugte erfindungsgemäße Zusammensetzungen enthalten 0,01 - 5 Gew.-% 5-Decen-1,10-dicarbonsäure und 0,0001 bis 5 Gew.-% exo-2-Cyano-5-isothiocyanatonorbornan. Weitere besonders bevorzugte erfindungsgemäße Zusammensetzungen enthalten 0,01 - 5 Gew.-% 5-Decen-1,10-dicarbonsäure und 0,0001 bis 5 Gew.-% 1-Cyano-4-isothiocyanatobutan.

Weitere besonders bevorzugte erfindungsgemäße Zusammensetzungen enthalten 0,01 - 5 Gew.-% 5-Decen-1,10-dicarbonsäure und 0,0001 bis 5 Gew.-% 5-Isothiocyanato-methylpentanoat.

Weitere besonders bevorzugte erfindungsgemäße Zusammensetzungen enthalten 0,01 - 5 Gew.-% 5-Decen-1,10-dicarbonsäure und 0,0001 bis 5 Gew.-% 5-Isothiocyanatopentansäure.

Weitere besonders bevorzugte erfindungsgemäße Zusammensetzungen enthalten 0,01 - 5 Gew.-% 5-Decen-1,10-dicarbonsäure und 0,0001 bis 5 Gew.-% 2-Isothiocyanato-ethylacetat.

Weitere besonders bevorzugte Zusammensetzungen enthalten 0,01 - 5 Gew.-% 5-Decen-1,10-dicarbonsäure und 0,0001 bis 5 Gew.-% 4-Isothiocyanato-methylbutyrat (Erypestrin).

Weitere besonders bevorzugte erfindungsgemäße Zusammensetzungen enthalten 0,01 - 5 Gew.-% 5-Decen-1,10-dicarbonsäure und 0,0001 bis 5 Gew.-% 4-Isothiocyanato-ethylbutyrat.

Weitere besonders bevorzugte erfindungsgemäße Zusammensetzungen enthalten 0,01 - 5 Gew.-% 5-Decen-1,10-dicarbonsäure und 0,0001 bis 5 Gew.-% 1-Isothiocyanato-2-methoxyphenol.

Weitere besonders bevorzugte erfindungsgemäße Zusammensetzungen enthalten 0,01 - 5 Gew.-% 5-Decen-1,10-dicarbonsäure und 0,0001 bis 5 Gew.-% Erucin (4-Methylthiobutylisothiocyanat.

Weitere besonders bevorzugte Zusammensetzungen enthalten 0,01 - 5 Gew.-% 5-Decen-1,10-dicarbonsäure und 0,0001 bis 5 Gew.-% Iberverin (3-Methylthiopropylisothiocyanat.

Weitere besonders bevorzugte Zusammensetzungen enthalten 0,01 - 5 Gew.-% 5-Decen-1,10-dicarbonsäure und 0,0001 bis 5 Gew.-% Berteroin (5-Methylthiopentylisothiocyanat.

Weitere besonders bevorzugte Zusammensetzungen enthalten 0,01 - 5 Gew.-% 5-Decen-1,10-dicarbonsäure und 0,0001 bis 5 Gew.-% Lesquerellin (6-Methylthiohexylisothiocyanat).

Weitere besonders bevorzugte erfindungsgemäße Zusammensetzungen enthalten 0,01 - 5 Gew.-% 5-Decen-1,10-dicarbonsäure und 0,0001 bis 5 Gew.-% Jirsutin (8-Methylthiooctylisothiocyanat).

Weitere besonders bevorzugte erfindungsgemäße Zusammensetzungen enthalten 0,01 - 5 Gew.-% 5-Decen-1,10-dicarbonsäure und 0,0001 bis 5 Gew.-% Arabin (9-Methylthiononylisothiocyanat).

Weitere besonders bevorzugte erfindungsgemäße Zusammensetzungen enthalten 0,01 - 5 Gew.-% 5-Decen-1,10-dicarbonsäure und 0,0001 bis 5 Gew.-% Allylisothiocyanat.

Weitere besonders bevorzugte erfindungsgemäße Zusammensetzungen enthalten 0,01 - 5 Gew.-% 5-Decen-1,10-dicarbonsäure und 0,0001 bis 5 Gew.-% Brassicin (Indol-3-ylmethylisothiocyanat). Weitere besonders bevorzugte Zusammensetzungen enthalten 0,01 - 5 Gew.-% 5-Decen-1,10-dicarbonsäure und 0,0001 bis 5 Gew.-% Goitrin (2-Hydroxybut-3-enyl-isothiocyanat).

Weitere besonders bevorzugte erfindungsgemäße Zusammensetzungen enthalten 0,01 - 5 Gew.-% 5-Decen-1,10-dicarbonsäure und 0,0001 bis 5 Gew.-% Napin (But-3-enyl-isothiocyanat).

Weitere besonders bevorzugte Zusammensetzungen enthalten 0,01 - 5 Gew.-% 5-Decen-1,10-dicarbonsäure und 0,0001 bis 5 Gew.-% Neobrassicin (*N*-Methoxyindol-3-ylmethyl-isothiocyanat).

Weitere besonders bevorzugte erfindungsgemäße Zusammensetzungen enthalten 0,01 - 5 Gew.-% 5-Decen-1,10-dicarbonsäure und 0,0001 bis 5 Gew.-% *N*-Acetylindol-3-ylmethyl-isothiocyanat.

Weitere besonders bevorzugte Zusammensetzungen enthalten 0,01 - 5 Gew.-% 5-Decen-1,10-dicarbonsäure und 0,0001 bis 5 Gew.-% Nasturtiin ((2-Phenylethyl)-isothiocyanat).

Weitere besonders bevorzugte Zusammensetzungen enthalten 0,01 - 5 Gew.-% 5-Decen-1,10-dicarbonsäure und 0,0001 bis 5 Gew.-% Barbarin (2-Hydroxy-2-phenylethyl-isothiocyanat).

Weitere besonders bevorzugte erfindungsgemäße Zusammensetzungen enthalten 0,01 - 5 Gew.-% 5-Decen-1,10-dicarbonsäure und 0,0001 bis 5 Gew.-% Tropaeolin (Benzylisothiocyanat).

Weitere besonders bevorzugte erfindungsgemäße Zusammensetzungen enthalten 0,01 - 5 Gew.-% 5-Decen-1,10-dicarbonsäure und 0,0001 bis 5 Gew.-% Sinalbin (4-Hydroxybenzylisothiocyanat). Weitere besonders bevorzugte erfindungsgemäße Zusammensetzungen enthalten 0,01 - 5 Gew.-% 5-Decen-1,10-dicarbonsäure und 0,0001 bis 5 Gew.-% Capparin (Methylisothiocyanat).

Weitere besonders bevorzugte erfindungsgemäße Zusammensetzungen enthalten 0,01 - 5 Gew.-% 5-Decen-1,10-dicarbonsäure und 0,0001 bis 5 Gew.-% Lepidin (Ethylisothiocyanat).

Weitere besonders bevorzugte erfindungsgemäße Zusammensetzungen enthalten 0,01 - 5 Gew.-% 5-Decen-1,10-dicarbonsäure und 0,0001 bis 5 Gew.-% Putranjivin (Isopropylisothiocyanat).

Weitere besonders bevorzugte erfindungsgemäße Zusammensetzungen enthalten 0,01 - 5 Gew.-% 5-Decen-1,10-dicarbonsäure und 0,0001 bis 5 Gew.-% Jiaputin (2-Methylbutylisothiocyanat).

Weitere besonders bevorzugte Zusammensetzungen enthalten 0,01 - 5 Gew.-% 5-Decen-1,10-dicarbonsäure und 0,0001 bis 5 Gew.-% Raphasatin (4-Methylthiobut-3-enyl-isothiocyanat).

Weitere besonders bevorzugte Zusammensetzungen enthalten 0,01 - 5 Gew.-% 5-Decen-1,10-dicarbonsäure und 0,0001 bis 5 Gew.-% Cochlearin (1-Methylpropylisothiocyanat).

Weitere besonders bevorzugte erfindungsgemäße Zusammensetzungen enthalten 0,01 - 5 Gew.-% 5-Decen-1,10-dicarbonsäure und 0,0001 bis 5 Gew.-% Brassicanapin (4-Pentenylisothiocyanat). Weitere besonders bevorzugte Zusammensetzungen enthalten 0,01 - 5 Gew.-% 5-Decen-1,10-dicarbonsäure und 0,0001 bis 5 Gew.-% Napoleiferin (2-Hydroxypent-4-enylisothiocyanat).

Weitere besonders bevorzugte erfindungsgemäße Zusammensetzungen enthalten 0,01 - 5 Gew.-% 5-Decen-1,10-dicarbonsäure und 0,0001 bis 5 Gew.-% Aubrietin.

Weitere besonders bevorzugte erfindungsgemäße Zusammensetzungen enthalten 0,01 - 5 Gew.-% 5-Decen-1,10-dicarbonsäure und 0,0001 bis 5 Gew.-% Limnanthin.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1004.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1021.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1022.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1023.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1031.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1032.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1033.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1041.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1042.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1043.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1044.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1045.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1046.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1047.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1048.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1049.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1050.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1051.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1052.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1053.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1062.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1063.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1064.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1065.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1069.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1070.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1072.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1076.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1077.

Weitere besonders bevorzugte Zusammensetzungen enthalten 0,01 - 5 Gew.-% 5-Decen-1,10-dicarbonsäure und 0,0001 bis 5 Gew.-% eines Isothiocyanats mit der Struktur GHP 1104.

Besonders bevorzugte erfindungsgemäße Zusammensetzungen enthalten 0,01 - 5 Gew.-% Azelainsäure und 0,0001 bis 5 Gew.-% Sulforaphan.

Weitere besonders bevorzugte erfindungsgemäße Zusammensetzungen enthalten 0,01 - 5 Gew.-% Azelainsäure und 0,0001 bis 5 Gew.-% Sulforaphen.

Weitere besonders bevorzugte erfindungsgemäße Zusammensetzungen enthalten 0,01 - 5 Gew.-% Azelainsäure und 0,0001 bis 5 Gew.-% 6-Isothiocyanato-2-hexanon.

Weitere besonders bevorzugte erfindungsgemäße Zusammensetzungen enthalten 0,01 - 5 Gew.-% Azelainsäure und 0,0001 bis 5 Gew.-% exo-2-Acetyl-6-isothiocyanatonorbornan.

Weitere besonders bevorzugte erfindungsgemäße Zusammensetzungen enthalten 0,01 - 5 Gew.-% Azelainsäure und 0,0001 bis 5 Gew.-% exo-2-Isothiocyanato-6-methylsulfonylnorbornan.

Weitere besonders bevorzugte erfindungsgemäße Zusammensetzungen enthalten 0,01 - 5 Gew.-% Azelainsäure und 0,0001 bis 5 Gew.-% 6-Isothiocyanato-2-hexanol.

Weitere besonders bevorzugte erfindungsgemäße Zusammensetzungen enthalten 0,01 - 5 Gew.-% Azelainsäure und 0,0001 bis 5 Gew.-% 1-Isothiocyanato-4-dimethylphosphonylbutan.

Weitere besonders bevorzugte erfindungsgemäße Zusammensetzungen enthalten 0,01 - 5 Gew.-% Azelainsäure und 0,0001 bis 5 Gew.-% exo-2-(1'-Hydroxyethyl)-5-isothiocyanatonorbornan.

Weitere besonders bevorzugte erfindungsgemäße Zusammensetzungen enthalten 0,01 - 5 Gew.-% Azelainsäure und 0,0001 bis 5 Gew.-% exo-2-Acetyl-5-isothiocyanatonorbornan.

Weitere besonders bevorzugte erfindungsgemäße Zusammensetzungen enthalten 0,01 - 5 Gew.-% Azelainsäure und 0,0001 bis 5 Gew.-% 1-Isothiocyanato-8-(methylsulfinyl)-octan (Hirsutin).

Weitere besonders bevorzugte erfindungsgemäße Zusammensetzungen enthalten 0,01 - 5 Gew.-% Azelainsäure und 0,0001 bis 5 Gew.-% 1-Isothiocyanato-5-methylsulfonylpentan.

Weitere besonders bevorzugte erfindungsgemäße Zusammensetzungen enthalten 0,01 - 5 Gew.-% Azelainsäure und 0,0001 bis 5 Gew.-% 1-Isothiocyanato-5-methylsulfinylpentan (Alyssin).

Weitere besonders bevorzugte erfindungsgemäße Zusammensetzungen enthalten 0,01 - 5 Gew.-% Azelainsäure und 0,0001 bis 5 Gew.-% 1-Isothiocyanato-4-methylsulfonylbutan (Erysolin).

Weitere besonders bevorzugte erfindungsgemäße Zusammensetzungen enthalten 0,01 - 5 Gew.-% Azelainsäure und 0,0001 bis 5 Gew.-% 1-Isothiocyanato-3-methylsulfinylpropan (Iberin).

Weitere besonders bevorzugte erfindungsgemäße Zusammensetzungen enthalten 0,01 - 5 Gew.-% Azelainsäure und 0,0001 bis 5 Gew.-% 1-Isothiocyanato-3-methylsulfonylpropan.

Weitere besonders bevorzugte erfindungsgemäße Zusammensetzungen enthalten 0,01 - 5 Gew.-% Azelainsäure und 0,0001 bis 5 Gew.-% cis-3-(Methylsulfonyl)cyclohexylisothiocyanat.

Weitere besonders bevorzugte erfindungsgemäße Zusammensetzungen enthalten 0,01 - 5 Gew.-% Azelainsäure und 0,0001 bis 5 Gew.-% cis-3-(Methylsulfonyl)cyclohexylmethylisothiocyanat.

Weitere besonders bevorzugte erfindungsgemäße Zusammensetzungen enthalten 0,01 - 5 Gew.-% Azelainsäure und 0,0001 bis 5 Gew.-% trans-3-(Methylsulfonyl)cyclohexylmethylisothiocyanat.

Weitere besonders bevorzugte erfindungsgemäße Zusammensetzungen enthalten 0,01 - 5 Gew.-% Azelainsäure und 0,0001 bis 5 Gew.-% 2-Isothiocyanato-4-methylsulfonyl-ethylbutyrat.

Weitere besonders bevorzugte erfindungsgemäße Zusammensetzungen enthalten 0,01 - 5 Gew.-% Azelainsäure und 0,0001 bis 5 Gew.-% 9-Isothiocyanato-5-nonanon.

Weitere besonders bevorzugte erfindungsgemäße Zusammensetzungen enthalten 0,01 - 5 Gew.-% Azelainsäure und 0,0001 bis 5 Gew.-% 7-Isothiocyanato-3-heptanon (Norcappasalin).

Weitere besonders bevorzugte erfindungsgemäße Zusammensetzungen enthalten 0,01 - 5 Gew.-% Azelainsäure und 0,0001 bis 5 Gew.-% 8-Isothiocyanato-4-octanon (Cappasalin).

Weitere besonders bevorzugte erfindungsgemäße Zusammensetzungen enthalten 0,01 - 5 Gew.-% Azelainsäure und 0,0001 bis 5 Gew.-% 7-Isothiocyanato-4-heptanon (Capangulin).

Weitere besonders bevorzugte erfindungsgemäße Zusammensetzungen enthalten 0,01 - 5 Gew.-% Azelainsäure und 0,0001 bis 5 Gew.-% exo-2-Cyano-5-isothiocyanatonorbornan.

Weitere besonders bevorzugte erfindungsgemäße Zusammensetzungen enthalten 0,01 - 5 Gew.-% Azelainsäure und 0,0001 bis 5 Gew.-% 1-Cyano-4-isothiocyanatobutan.

Weitere besonders bevorzugte erfindungsgemäße Zusammensetzungen enthalten 0,01 - 5 Gew.-% Azelainsäure und 0,0001 bis 5 Gew.-% 5-Isothiocyanato-methylpentanoat.

Weitere besonders bevorzugte erfindungsgemäße Zusammensetzungen enthalten 0,01 - 5 Gew.-% Azelainsäure und 0,0001 bis 5 Gew.-% 5-Isothiocyanatopentansäure.

Weitere besonders bevorzugte erfindungsgemäße Zusammensetzungen enthalten 0,01 - 5 Gew.-% Azelainsäure und 0,0001 bis 5 Gew.-% 2-Isothiocyanato-ethylacetat.

Weitere besonders bevorzugte erfindungsgemäße Zusammensetzungen enthalten 0,01 - 5 Gew.-% Azelainsäure und 0,0001 bis 5 Gew.-% 4-Isothiocyanato-methylbutyrat (Erypestrin).

Weitere besonders bevorzugte erfindungsgemäße Zusammensetzungen enthalten 0,01 - 5 Gew.-% Azelainsäure und 0,0001 bis 5 Gew.-% 4-Isothiocyanato-ethylbutyrat.

Weitere besonders bevorzugte erfindungsgemäße Zusammensetzungen enthalten 0,01 - 5 Gew.-% Azelainsäure und 0,0001 bis 5 Gew.-% 1-Isothiocyanato-2-methoxyphenol.

Weitere besonders bevorzugte erfindungsgemäße Zusammensetzungen enthalten 0,01 - 5 Gew.-% Azelainsäure und 0,0001 bis 5 Gew.-% Erucin (4-Methylthiobutylisothiocyanat.

Weitere besonders bevorzugte erfindungsgemäße Zusammensetzungen enthalten 0,01 - 5 Gew.-% Azelainsäure und 0,0001 bis 5 Gew.-% Iberverin (3-Methylthiopropylisothiocyanat.

Weitere besonders bevorzugte erfindungsgemäße Zusammensetzungen enthalten 0,01 - 5 Gew.-% Azelainsäure und 0,0001 bis 5 Gew.-% Berteroin (5-Methylthiopentylisothiocyanat.

Weitere besonders bevorzugte erfindungsgemäße Zusammensetzungen enthalten 0,01 - 5 Gew.-% Azelainsäure und 0,0001 bis 5 Gew.-% Lesquerellin (6-Methylthiohexylisothiocyanat).

Weitere besonders bevorzugte erfindungsgemäße Zusammensetzungen enthalten 0,01 - 5 Gew.-% Azelainsäure und 0,0001 bis 5 Gew.-% Jirsutin (8-Methylthiooctylisothiocyanat).

Weitere besonders bevorzugte erfindungsgemäße Zusammensetzungen enthalten 0,01 - 5 Gew.-% Azelainsäure und 0,0001 bis 5 Gew.-% Arabin (9-Methylthiononylisothiocyanat).

Weitere besonders bevorzugte erfindungsgemäße Zusammensetzungen enthalten 0,01 - 5 Gew.-% Azelainsäure und 0,0001 bis 5 Gew.-% Allylisothiocyanat.

Weitere besonders bevorzugte erfindungsgemäße Zusammensetzungen enthalten 0,01 - 5 Gew.-% Azelainsäure und 0,0001 bis 5 Gew.-% Brassicin (Indol-3-ylmethylisothiocyanat).

Weitere besonders bevorzugte erfindungsgemäße Zusammensetzungen enthalten 0,01 - 5 Gew.-% Azelainsäure und 0,0001 bis 5 Gew.-% Goitrin (2-Hydroxybut-3-enyl-isothiocyanat).

Weitere besonders bevorzugte erfindungsgemäße Zusammensetzungen enthalten 0,01 - 5 Gew.-% Azelainsäure und 0,0001 bis 5 Gew.-% Napin (But-3-enyl-isothiocyanat).

Weitere besonders bevorzugte erfindungsgemäße Zusammensetzungen enthalten 0,01 - 5 Gew.-% Azelainsäure und 0,0001 bis 5 Gew.-% Neobrassicin (*N*-Methoxyindol-3-ylmethyl-isothiocyanat). Weitere besonders bevorzugte erfindungsgemäße Zusammensetzungen enthalten 0,01 - 5 Gew.-% Azelainsäure und 0,0001 bis 5 Gew.-% *N*-Acetylindol-3-ylmethyl-isothiocyanat.

Weitere besonders bevorzugte erfindungsgemäße Zusammensetzungen enthalten 0,01 - 5 Gew.-% Azelainsäure und 0,0001 bis 5 Gew.-% Nasturtiin ((2-Phenylethyl)-isothiocyanat).

Weitere besonders bevorzugte erfindungsgemäße Zusammensetzungen enthalten 0,01 - 5 Gew.-% Azelainsäure und 0,0001 bis 5 Gew.-% Barbarin (2-Hydroxy-2-phenylethyl-isothiocyanat).

Weitere besonders bevorzugte erfindungsgemäße Zusammensetzungen enthalten 0,01 - 5 Gew.-% Azelainsäure und 0,0001 bis 5 Gew.-% Tropaeolin (Benzylisothiocyanat).

Weitere besonders bevorzugte erfindungsgemäße Zusammensetzungen enthalten 0,01 - 5 Gew.-% Azelainsäure und 0,0001 bis 5 Gew.-% Sinalbin (4-Hydroxybenzylisothiocyanat).

Weitere besonders bevorzugte erfindungsgemäße Zusammensetzungen enthalten 0,01 - 5 Gew.-% Azelainsäure und 0,0001 bis 5 Gew.-% Capparin (Methylisothiocyanat).

Weitere besonders bevorzugte erfindungsgemäße Zusammensetzungen enthalten 0,01 - 5 Gew.-% Azelainsäure und 0,0001 bis 5 Gew.-% Lepidin (Ethylisothiocyanat).

Weitere besonders bevorzugte erfindungsgemäße Zusammensetzungen enthalten 0,01 - 5 Gew.-% Azelainsäure und 0,0001 bis 5 Gew.-% Putranjivin (Isopropylisothiocyanat).

Weitere besonders bevorzugte erfindungsgemäße Zusammensetzungen enthalten 0,01 - 5 Gew.-% Azelainsäure und 0,0001 bis 5 Gew.-% Jiaputin (2-Methylbutylisothiocyanat).

Weitere besonders bevorzugte erfindungsgemäße Zusammensetzungen enthalten 0,01 - 5 Gew.-% Azelainsäure und 0,0001 bis 5 Gew.-% Raphasatin (4-Methylthiobut-3-enyl-isothiocyanat).

Weitere besonders bevorzugte erfindungsgemäße Zusammensetzungen enthalten 0,01 - 5 Gew.-% Azelainsäure und 0,0001 bis 5 Gew.-% Cochlearin (1-Methylpropylisothiocyanat).

Weitere besonders bevorzugte erfindungsgemäße Zusammensetzungen enthalten 0,01 - 5 Gew.-% Azelainsäure und 0,0001 bis 5 Gew.-% Brassicanapin (4-Pentenylisothiocyanat).

Weitere besonders bevorzugte erfindungsgemäße Zusammensetzungen enthalten 0,01 - 5 Gew.-% Azelainsäure und 0,0001 bis 5 Gew.-% Napoleiferin (2-Hydroxypent-4-enylisothiocyanat).

Weitere besonders bevorzugte erfindungsgemäße Zusammensetzungen enthalten 0,01 - 5 Gew.-% Azelainsäure und 0,0001 bis 5 Gew.-% Aubrietin.

Weitere besonders bevorzugte erfindungsgemäße Zusammensetzungen enthalten 0,01 - 5 Gew.-% Azelainsäure und 0,0001 bis 5 Gew.-% Limnanthin.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1004.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1021.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1022.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1023.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1031.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1032.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1033.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1041.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1042.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1043.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1044.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1045.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1046.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1047.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1048.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1049.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1050.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1051.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1052.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1053.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1062.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1063.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1064.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1065.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1069.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1070.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1072.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1076.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1077.

Weitere besonders bevorzugte erfindungsgemäße Zusammensetzungen enthalten 0,01 - 5 Gew.-% Azelainsäure und 0,0001 bis 5 Gew.-% eines Isothiocyanats mit der Struktur GHP 1104.

Besonders bevorzugte erfindungsgemäße Zusammensetzungen enthalten 0,01 - 5 Gew.-% Sebacinsäure und 0,0001 bis 5 Gew.-% Sulforaphan.

Weitere besonders bevorzugte erfindungsgemäße Zusammensetzungen enthalten 0,01 - 5 Gew.-% Sebacinsäure und 0,0001 bis 5 Gew.-% Sulforaphen.

Weitere besonders bevorzugte erfindungsgemäße Zusammensetzungen enthalten 0,01 - 5 Gew.-% Sebacinsäure und 0,0001 bis 5 Gew.-% 6-Isothiocyanato-2-hexanon.

Weitere besonders bevorzugte erfindungsgemäße Zusammensetzungen enthalten 0,01 - 5 Gew.-% Sebacinsäure und 0,0001 bis 5 Gew.-% exo-2-Acetyl-6-isothiocyanatonorbornan.

Weitere besonders bevorzugte erfindungsgemäße Zusammensetzungen enthalten 0,01 - 5 Gew.-% Sebacinsäure und 0,0001 bis 5 Gew.-% exo-2-Isothiocyanato-6-methylsulfonylnorbornan.

Weitere besonders bevorzugte erfindungsgemäße Zusammensetzungen enthalten 0,01 - 5 Gew.-% Sebacinsäure und 0,0001 bis 5 Gew.-% 6-Isothiocyanato-2-hexanol.

Weitere besonders bevorzugte erfindungsgemäße Zusammensetzungen enthalten 0,01 - 5 Gew.-% Sebacinsäure und 0,0001 bis 5 Gew.-% 1-Isothiocyanato-4-dimethylphosphonylbutan.

Weitere besonders bevorzugte erfindungsgemäße Zusammensetzungen enthalten 0,01 - 5 Gew.-% Sebacinsäure und 0,0001 bis 5 Gew.-% exo-2-(1'-Hydroxyethyl)-5-isothiocyanatonorbornan.

Weitere besonders bevorzugte erfindungsgemäße Zusammensetzungen enthalten 0,01 - 5 Gew.-% Sebacinsäure und 0,0001 bis 5 Gew.-% exo-2-Acetyl-5-isothiocyanatonorbornan.

Weitere besonders bevorzugte erfindungsgemäße Zusammensetzungen enthalten 0,01 - 5 Gew.-% Sebacinsäure und 0,0001 bis 5 Gew.-% 1-Isothiocyanato-8-(methylsulfinyl)-octan (Hirsutin).

Weitere besonders bevorzugte erfindungsgemäße Zusammensetzungen enthalten 0,01 - 5 Gew.-% Sebacinsäure und 0,0001 bis 5 Gew.-% 1-Isothiocyanato-5-methylsulfonylpentan.

Weitere besonders bevorzugte erfindungsgemäße Zusammensetzungen enthalten 0,01 - 5 Gew.-% Sebacinsäure und 0,0001 bis 5 Gew.-% 1-Isothiocyanato-5-methylsulfinylpentan (Alyssin).

Weitere besonders bevorzugte erfindungsgemäße Zusammensetzungen enthalten 0,01 - 5 Gew.-% Sebacinsäure und 0,0001 bis 5 Gew.-% 1-Isothiocyanato-4-methylsulfonylbutan (Erysolin).

Weitere besonders bevorzugte erfindungsgemäße Zusammensetzungen enthalten 0,01 - 5 Gew.-% Sebacinsäure und 0,0001 bis 5 Gew.-% 1-Isothiocyanato-3-methylsulfinylpropan (Iberin).

Weitere besonders bevorzugte erfindungsgemäße Zusammensetzungen enthalten 0,01 - 5 Gew.-% Sebacinsäure und 0,0001 bis 5 Gew.-% 1-Isothiocyanato-3-methylsulfonylpropan.

Weitere besonders bevorzugte erfindungsgemäße Zusammensetzungen enthalten 0,01 - 5 Gew.-% Sebacinsäure und 0,0001 bis 5 Gew.-% cis-3-(Methylsulfonyl)cyclohexylisothiocyanat.

Weitere besonders bevorzugte erfindungsgemäße Zusammensetzungen enthalten 0,01 - 5 Gew.-% Sebacinsäure und 0,0001 bis 5 Gew.-% cis-3-(Methylsulfonyl)cyclohexylmethylisothiocyanat.

Weitere besonders bevorzugte erfindungsgemäße Zusammensetzungen enthalten 0,01 - 5 Gew.-% Sebacinsäure und 0,0001 bis 5 Gew.-% trans-3-(Methylsulfonyl)cyclohexylmethylisothiocyanat.

Weitere besonders bevorzugte erfindungsgemäße Zusammensetzungen enthalten 0,01 - 5 Gew.-% Sebacinsäure und 0,0001 bis 5 Gew.-% 2-Isothiocyanato-4-methylsulfonyl-ethylbutyrat.

Weitere besonders bevorzugte erfindungsgemäße Zusammensetzungen enthalten 0,01 - 5 Gew.-% Sebacinsäure und 0,0001 bis 5 Gew.-% 9-Isothiocyanato-5-nonanon.

Weitere besonders bevorzugte erfindungsgemäße Zusammensetzungen enthalten 0,01 - 5 Gew.-% Sebacinsäure und 0,0001 bis 5 Gew.-% 7-Isothiocyanato-3-heptanon (Norcappasalin).

Weitere besonders bevorzugte erfindungsgemäße Zusammensetzungen enthalten 0,01 - 5 Gew.-% Sebacinsäure und 0,0001 bis 5 Gew.-% 8-Isothiocyanato-4-octanon (Cappasalin).

Weitere besonders bevorzugte erfindungsgemäße Zusammensetzungen enthalten 0,01 - 5 Gew.-% Sebacinsäure und 0,0001 bis 5 Gew.-% 7-Isothiocyanato-4-heptanon (Capangulin).

Weitere besonders bevorzugte erfindungsgemäße Zusammensetzungen enthalten 0,01 - 5 Gew.-% Sebacinsäure und 0,0001 bis 5 Gew.-% exo-2-Cyano-5-isothiocyanatonorbornan.

Weitere besonders bevorzugte erfindungsgemäße Zusammensetzungen enthalten 0,01 - 5 Gew.-% Sebacinsäure und 0,0001 bis 5 Gew.-% 1-Cyano-4-isothiocyanatobutan.

Weitere besonders bevorzugte erfindungsgemäße Zusammensetzungen enthalten 0,01 - 5 Gew.-% Sebacinsäure und 0,0001 bis 5 Gew.-% 5-Isothiocyanato-methylpentanoat.

Weitere besonders bevorzugte erfindungsgemäße Zusammensetzungen enthalten 0,01 - 5 Gew.-% Sebacinsäure und 0,0001 bis 5 Gew.-% 5-Isothiocyanatopentansäure.

Weitere besonders bevorzugte erfindungsgemäße Zusammensetzungen enthalten 0,01 - 5 Gew.-% Sebacinsäure und 0,0001 bis 5 Gew.-% 2-Isothiocyanato-ethylacetat.

Weitere besonders bevorzugte erfindungsgemäße Zusammensetzungen enthalten 0,01 - 5 Gew.-% Sebacinsäure und 0,0001 bis 5 Gew.-% 4-Isothiocyanato-methylbutyrat (Erypestrin).

Weitere besonders bevorzugte erfindungsgemäße Zusammensetzungen enthalten 0,01 - 5 Gew.-% Sebacinsäure und 0,0001 bis 5 Gew.-% 4-Isothiocyanato-ethylbutyrat.

Weitere besonders bevorzugte erfindungsgemäße Zusammensetzungen enthalten 0,01 - 5 Gew.-% Sebacinsäure und 0,0001 bis 5 Gew.-% 1-Isothiocyanato-2-methoxyphenol.

Weitere besonders bevorzugte erfindungsgemäße Zusammensetzungen enthalten 0,01 - 5 Gew.-% Sebacinsäure und 0,0001 bis 5 Gew.-% Erucin (4-Methylthiobutylisothiocyanat.

Weitere besonders bevorzugte erfindungsgemäße Zusammensetzungen enthalten 0,01 - 5 Gew.-% Sebacinsäure und 0,0001 bis 5 Gew.-% Iberverin (3-Methylthiopropylisothiocyanat.

Weitere besonders bevorzugte erfindungsgemäße Zusammensetzungen enthalten 0,01 - 5 Gew.-% Sebacinsäure und 0,0001 bis 5 Gew.-% Berteroin (5-Methylthiopentylisothiocyanat.

Weitere besonders bevorzugte erfindungsgemäße Zusammensetzungen enthalten 0,01 - 5 Gew.-% Sebacinsäure und 0,0001 bis 5 Gew.-% Lesquerellin (6-Methylthiohexylisothiocyanat).

Weitere besonders bevorzugte erfindungsgemäße Zusammensetzungen enthalten 0,01 - 5 Gew.-% Sebacinsäure und 0,0001 bis 5 Gew.-% Jirsutin (8-Methylthiooctylisothiocyanat).

Weitere besonders bevorzugte erfindungsgemäße Zusammensetzungen enthalten 0,01 - 5 Gew.-% Sebacinsäure und 0,0001 bis 5 Gew.-% Arabin (9-Methylthiononylisothiocyanat).

Weitere besonders bevorzugte erfindungsgemäße Zusammensetzungen enthalten 0,01 - 5 Gew.-% Sebacinsäure und 0,0001 bis 5 Gew.-% Allylisothiocyanat.

Weitere besonders bevorzugte erfindungsgemäße Zusammensetzungen enthalten 0,01 - 5 Gew.-% Sebacinsäure und 0,0001 bis 5 Gew.-% Brassicin (Indol-3-ylmethylisothiocyanat).

Weitere besonders bevorzugte erfindungsgemäße Zusammensetzungen enthalten 0,01 - 5 Gew.-% Sebacinsäure und 0,0001 bis 5 Gew.-% Goitrin (2-Hydroxybut-3-enyl-isothiocyanat).

Weitere besonders bevorzugte erfindungsgemäße Zusammensetzungen enthalten 0,01 - 5 Gew.-% Sebacinsäure und 0,0001 bis 5 Gew.-% Napin (But-3-enyl-isothiocyanat).

Weitere besonders bevorzugte Zusammensetzungen enthalten 0,01 - 5 Gew.-% Sebacinsäure und 0,0001 bis 5 Gew.-% Neobrassicin (N-Methoxyindol-3-ylmethyl-isothiocyanat).

Weitere besonders bevorzugte erfindungsgemäße Zusammensetzungen enthalten 0,01 - 5 Gew.-% Sebacinsäure und 0,0001 bis 5 Gew.-% N-Acetylindol-3-ylmethyl-isothiocyanat.

Weitere besonders bevorzugte erfindungsgemäße Zusammensetzungen enthalten 0,01 - 5 Gew.-% Sebacinsäure und 0,0001 bis 5 Gew.-% Nasturtiin ((2-Phenylethyl)-isothiocyanat).

Weitere besonders bevorzugte erfindungsgemäße Zusammensetzungen enthalten 0,01 - 5 Gew.-% Sebacinsäure und 0,0001 bis 5 Gew.-% Barbarin (2-Hydroxy-2-phenylethyl-isothiocyanat).

Weitere besonders bevorzugte erfindungsgemäße Zusammensetzungen enthalten 0,01 - 5 Gew.-% Sebacinsäure und 0,0001 bis 5 Gew.-% Tropaeolin (Benzylisothiocyanat).

Weitere besonders bevorzugte erfindungsgemäße Zusammensetzungen enthalten 0,01 - 5 Gew.-% Sebacinsäure und 0,0001 bis 5 Gew.-% Sinalbin (4-Hydroxybenzylisothiocyanat).

Weitere besonders bevorzugte erfindungsgemäße Zusammensetzungen enthalten 0,01 - 5 Gew.-% Sebacinsäure und 0,0001 bis 5 Gew.-% Capparin (Methylisothiocyanat).

Weitere besonders bevorzugte erfindungsgemäße Zusammensetzungen enthalten 0,01 - 5 Gew.-% Sebacinsäure und 0,0001 bis 5 Gew.-% Lepidin (Ethylisothiocyanat).

Weitere besonders bevorzugte erfindungsgemäße Zusammensetzungen enthalten 0,01 - 5 Gew.-% Sebacinsäure und 0,0001 bis 5 Gew.-% Putranjivin (Isopropylisothiocyanat).

Weitere besonders bevorzugte erfindungsgemäße Zusammensetzungen enthalten 0,01 - 5 Gew.-% Sebacinsäure und 0,0001 bis 5 Gew.-% Jiaputin (2-Methylbutylisothiocyanat).

Weitere besonders bevorzugte erfindungsgemäße Zusammensetzungen enthalten 0,01 - 5 Gew.-% Sebacinsäure und 0,0001 bis 5 Gew.-% Raphasatin (4-Methylthiobut-3-enyl-isothiocyanat).

Weitere besonders bevorzugte erfindungsgemäße Zusammensetzungen enthalten 0,01 - 5 Gew.-% Sebacinsäure und 0,0001 bis 5 Gew.-% Cochlearin (1-Methylpropylisothiocyanat).

Weitere besonders bevorzugte erfindungsgemäße Zusammensetzungen enthalten 0,01 - 5 Gew.-% Sebacinsäure und 0,0001 bis 5 Gew.-% Brassicanapin (4-Pentenylisothiocyanat).

Weitere besonders bevorzugte erfindungsgemäße Zusammensetzungen enthalten 0,01 - 5 Gew.-% Sebacinsäure und 0,0001 bis 5 Gew.-% Napoleiferin (2-Hydroxypent-4-enylisothiocyanat).

Weitere besonders bevorzugte erfindungsgemäße Zusammensetzungen enthalten 0,01 - 5 Gew.-% Sebacinsäure und 0,0001 bis 5 Gew.-% Aubrietin.

Weitere besonders bevorzugte erfindungsgemäße Zusammensetzungen enthalten 0,01 - 5 Gew.-% Sebacinsäure und 0,0001 bis 5 Gew.-% Limnanthin.

Weitere besonders bevorzugte erfindungsgemäße Zusammensetzungen enthalten 0,01 - 5 Gew.-% Sebacinsäure und 0,0001 bis 5 Gew.-% eines Isothiocyanats mit der Struktur GHP 1004.

Weitere besonders bevorzugte erfindungsgemäße Zusammensetzungen enthalten 0,01 - 5 Gew.-% Sebacinsäure und 0,0001 bis 5 Gew.-% eines Isothiocyanats mit der Struktur GHP 1021.

Weitere besonders bevorzugte erfindungsgemäße Zusammensetzungen enthalten 0,01 - 5 Gew.-% Sebacinsäure und 0,0001 bis 5 Gew.-% eines Isothiocyanats mit der Struktur GHP 1022.

Weitere besonders bevorzugte erfindungsgemäße Zusammensetzungen enthalten 0,01 - 5 Gew.-% Sebacinsäure und 0,0001 bis 5 Gew.-% eines Isothiocyanats mit der Struktur GHP 1023.

Weitere besonders bevorzugte erfindungsgemäße Zusammensetzungen enthalten 0,01 - 5 Gew.-% Sebacinsäure und 0,0001 bis 5 Gew.-% eines Isothiocyanats mit der Struktur GHP 1031.

Weitere besonders bevorzugte erfindungsgemäße Zusammensetzungen enthalten 0,01 - 5 Gew.-% Sebacinsäure und 0,0001 bis 5 Gew.-% eines Isothiocyanats mit der Struktur GHP 1032.

Weitere besonders bevorzugte erfindungsgemäße Zusammensetzungen enthalten 0,01 - 5 Gew.-% Sebacinsäure und 0,0001 bis 5 Gew.-% eines Isothiocyanats mit der Struktur GHP 1033.

Weitere besonders bevorzugte erfindungsgemäße Zusammensetzungen enthalten 0,01 - 5 Gew.-% Sebacinsäure und 0,0001 bis 5 Gew.-% eines Isothiocyanats mit der Struktur GHP 1041.

Weitere besonders bevorzugte erfindungsgemäße Zusammensetzungen enthalten 0,01 - 5 Gew.-% Sebacinsäure und 0,0001 bis 5 Gew.-% eines Isothiocyanats mit der Struktur GHP 1042.

Weitere besonders bevorzugte erfindungsgemäße Zusammensetzungen enthalten 0,01 - 5 Gew.-% Sebacinsäure und 0,0001 bis 5 Gew.-% eines Isothiocyanats mit der Struktur GHP 1043.

Weitere besonders bevorzugte erfindungsgemäße Zusammensetzungen enthalten 0,01 - 5 Gew.-% Sebacinsäure und 0,0001 bis 5 Gew.-% eines Isothiocyanats mit der Struktur GHP 1044.

Weitere besonders bevorzugte erfindungsgemäße Zusammensetzungen enthalten 0,01 - 5 Gew.-% Sebacinsäure und 0,0001 bis 5 Gew.-% eines Isothiocyanats mit der Struktur GHP 1045.

Weitere besonders bevorzugte erfindungsgemäße Zusammensetzungen enthalten 0,01 - 5 Gew.-% Sebacinsäure und 0,0001 bis 5 Gew.-% eines Isothiocyanats mit der Struktur GHP 1046.

Weitere besonders bevorzugte erfindungsgemäße Zusammensetzungen enthalten 0,01 - 5 Gew.-% Sebacinsäure und 0,0001 bis 5 Gew.-% eines Isothiocyanats mit der Struktur GHP 1047.

Weitere besonders bevorzugte erfindungsgemäße Zusammensetzungen enthalten 0,01 - 5 Gew.-% Sebacinsäure und 0,0001 bis 5 Gew.-% eines Isothiocyanats mit der Struktur GHP 1048.

Weitere besonders bevorzugte erfindungsgemäße Zusammensetzungen enthalten 0,01 - 5 Gew.-% Sebacinsäure und 0,0001 bis 5 Gew.-% eines Isothiocyanats mit der Struktur GHP 1049.

Weitere besonders bevorzugte erfindungsgemäße Zusammensetzungen enthalten 0,01 - 5 Gew.-% Sebacinsäure und 0,0001 bis 5 Gew.-% eines Isothiocyanats mit der Struktur GHP 1050.

Weitere besonders bevorzugte erfindungsgemäße Zusammensetzungen enthalten 0,01 - 5 Gew.-% Sebacinsäure und 0,0001 bis 5 Gew.-% eines Isothiocyanats mit der Struktur GHP 1051.

Weitere besonders bevorzugte erfindungsgemäße Zusammensetzungen enthalten 0,01 - 5 Gew.-% Sebacinsäure und 0,0001 bis 5 Gew.-% eines Isothiocyanats mit der Struktur GHP 1052.

Weitere besonders bevorzugte erfindungsgemäße Zusammensetzungen enthalten 0,01 - 5 Gew.-% Sebacinsäure und 0,0001 bis 5 Gew.-% eines Isothiocyanats mit der Struktur GHP 1053.

Weitere besonders bevorzugte erfindungsgemäße Zusammensetzungen enthalten 0,01 - 5 Gew.-% Sebacinsäure und 0,0001 bis 5 Gew.-% eines Isothiocyanats mit der Struktur GHP 1062.

Weitere besonders bevorzugte erfindungsgemäße Zusammensetzungen enthalten 0,01 - 5 Gew.-% Sebacinsäure und 0,0001 bis 5 Gew.-% eines Isothiocyanats mit der Struktur GHP 1063.

Weitere besonders bevorzugte erfindungsgemäße Zusammensetzungen enthalten 0,01 - 5 Gew.-% Sebacinsäure und 0,0001 bis 5 Gew.-% eines Isothiocyanats mit der Struktur GHP 1064.

Weitere besonders bevorzugte erfindungsgemäße Zusammensetzungen enthalten 0,01 - 5 Gew.-% Sebacinsäure und 0,0001 bis 5 Gew.-% eines Isothiocyanats mit der Struktur GHP 1065.

Weitere besonders bevorzugte erfindungsgemäße Zusammensetzungen enthalten 0,01 - 5 Gew.-% Sebacinsäure und 0,0001 bis 5 Gew.-% eines Isothiocyanats mit der Struktur GHP 1069.

Weitere besonders bevorzugte erfindungsgemäße Zusammensetzungen enthalten 0,01 - 5 Gew.-% Sebacinsäure und 0,0001 bis 5 Gew.-% eines Isothiocyanats mit der Struktur GHP 1070.

Weitere besonders bevorzugte erfindungsgemäße Zusammensetzungen enthalten 0,01 - 5 Gew.-% Sebacinsäure und 0,0001 bis 5 Gew.-% eines Isothiocyanats mit der Struktur GHP 1072.

Weitere besonders bevorzugte erfindungsgemäße Zusammensetzungen enthalten 0,01 - 5 Gew.-% Sebacinsäure und 0,0001 bis 5 Gew.-% eines Isothiocyanats mit der Struktur GHP 1076.

Weitere besonders bevorzugte erfindungsgemäße Zusammensetzungen enthalten 0,01 - 5 Gew.-% Sebacinsäure und 0,0001 bis 5 Gew.-% eines *Isothiocyanats mit der Struktur GHP 1077.

Weitere besonders bevorzugte erfindungsgemäße Zusammensetzungen enthalten 0,01 - 5 Gew.-% Sebacinsäure und 0,0001 bis 5 Gew.-% eines Isothiocyanats mit der Struktur GHP 1104.

Vorzugsweise werden die mindestens eine Säure, ausgewählt aus 8-Hexadecen-1,16-dicarbonsäure, 7-Tetradecen-1,14-dicarbonsäure, 9-Octadecen-1,18-dicarbonsäure, 6-Dodecen-1,12-dicarbonsäure, 5-Decen-1,10-dicarbonsäure, Decandisäure (Sebacinsäure) und Nonandisäure (Azelainsäure), und die mindestens eine Substanz, ausgewählt aus Sulforaphan, Sulforaphen, Sulforaphan-Analoga und Sulforaphen-Analoga, in bestimmten Mengenverhältnissen zueinander eingesetzt. Hier sind erfindungsgemäße kosmetische oder dermatologische Zusammensetzungen bevorzugt, bei denen das Gewichtsverhältnis von Gesamtmenge an Disäure (= 8-Hexadecen-1,16-dicarbonsäure, 7-Tetradecen-1,14-dicarbonsäure, 9-Octadecen-1,18-dicarbonsäure, 6-Dodecen-1,12-dicarbonsäure, 5-Decen-1,10-dicarbonsäure, Decandisäure (Sebacinsäure) und Nonandisäure (Azelainsäure)), zur Gesamtmenge an Sulforaphan, Sulforaphen, Sulforaphan-Analoga und

Sulforaphen-Analoga 1000 : 1 bis 1: 1, vorzugsweise 500 : 1 bis 10 : 1 und insbesondere 300 : 1 bis 50 : 1, beträgt.

In einer bevorzugten Ausführungsform enthalten die erfindungsgemäßen Zusammensetzungen zur Unterstützung der aufhellenden Wirkung der Kombination aus mindestens einer Säure, ausgewählt aus 8-Hexadecen-1,16-dicarbonsäure, 7-Tetradecen-1,14-dicarbonsäure, 9-Octadecen-1,18-dicarbonsäure, 6-Dodecen-1,12-dicarbonsäure, 5-Decen-1,10-dicarbonsäure, Decandisäure (Sebacinsäure) und Nonandisäure (Azelainsäure), in einer Gesamtmenge von 0,01 - 5 Gew.-%, mit 0,0001 bis 5 Gew.-% Gesamtgehalt an mindestens einer Substanz, ausgewählt aus der Gruppe, bestehend aus Sulforaphan, Sulforaphen, Sulforaphan-Analoga und Sulforaphen-Analoga, weiterhin insgesamt 0,05 bis 10 Gew.-%, vorzugsweise 0,5 bis 5 Gew.-% und insbesondere 1 bis 3 Gew.-%, an mindestens einer weiteren hautaufhellenden Substanz enthält, die ausgewählt ist aus der Gruppe, bestehend aus Ascorbinsäure, Ascorbinsäuresalzen, Ascorbinsäureglycosiden, Ascorbinsäureglycosidsalzen, Ascorbinsäureestern von organischen Säuren, den Salzen der Ascorbinsäureester von organischen Säuren, Ascorbinsäureestern von anorganischen Säuren, den Salzen der Ascorbinsäureester von anorganischen Säuren, Extrakten aus der Wurzel von Glycyrrhiza glabra, Glycyrrhetin, Glycyrrhetinsäure und ihren Salzen, Extrakten aus verschiedenen Teilen des Maulbeerbaums (Morus spp., insbesondere aus Morus alba und hier insbesondere Extrakte aus der Baumrinde, dem Stamm, den Blättern und den Früchten), Extrakten aus der Schale von Citrus unshiu, Phytinsäure, Diacetylboldin (O,O-Diacetylboldin), Hexapeptide-2, Extrakten aus dem Pilz Nigrospora sphaerica, Hydrochinon, Kojisäure, Arbutin, Extrakten aus Scutellaria spp. (Helmkraut), insbesondere aus Scutellaria baicalensis (Baikal-Helmkraut) und hier insbesondere Extrakte aus der Wurzel, Extrakten aus *Waltheria indica* ("Sleepy Morning") und hier insbesondere Extrakte aus den Blättern, Extrakten aus Bärentraube (*Arctostaphylos uva-ursi* (L.), Ericaceae) und hier insbesondere Extrakte aus den Blättern, Extrakten aus Preiselbeere (Blätter und Blüten), Extrakten aus Heidelbeere (Früchte), Extrakten aus Blattknospen von Birnbäumen, Anissamenöl, Extrakten aus Brombeerblättern, weiterhin Extrakten aus *Pyrola rotundifolia* (Rundblättriges Wintergrün), Gurke und/oder Limone, weiterhin Cumarinsäure ((Z)-2-Hydroxyzimtsäure), Hydroxystilbenen, deren Estern, Salzen und Oligomeren, insbesondere Resveratrol, alpha-Viniferin und epsilon-Viniferin, Extrakten aus diversen Bestandteilen der Weinreben (Traubenfrüchte, Traubenkerne, Traubenschalen, Stiele), sowie Mischungen der vorgenannten Substanzen.

Besonders bevorzugte erfindungsgemäße kosmetische oder dermatologische Zusammensetzungen sind **dadurch gekennzeichnet, dass** sie bezogen auf ihr Gewicht 0,05 bis 3,0 Gew.-%, vorzugsweise 0,07 bis 2,0 Gew.-% und insbesondere 0,1 bis 1,0 Gew.-% Ascorbinsäure und/oder Ascorbinsäuresalze und/oder Ascorbinsäurederivate enthalten. Als Ascorbinsäuredervivate kommen insbesondere die Salze der Ascorbinsäure, d.h. Hydroascorbate bzw. Ascorbate in Frage, wobei unter diesen die Alkali- und Erdalkalisalze bevorzugt sind. Besonders bevorzugte Salze der Ascorbinsäure, die erfindungsgemäß eingesetzt werden können, sind Natriumhydroascorbat, Natriumascorbat, Kaliumhydroascorbat, Kaliumascorbat, Ammoniumascorbat, Ammoniumhydroascorbat, Calciumascorbat, Calciumhydroascorbat, Magnesiumascorbat und Magnesiumhydroascorbat.

Mit besonderem Vorzug können auch die Ester der Ascorbinsäure eingesetzt werden (R ≠ H in der vorstehend aufgeführten Formel). Unter den Estern der Ascorbinsäure sind insbesondere Ascorbylmethanoat, Ascorbylethanoat, Ascorbyl-n-propanoat, Ascorbyl-iso-propanoat, Ascorbyl-n-butanoat, usw. bevorzugt. Mit besonderem Vorzug enthalten die erfindungsgemäßen Zusammensetzungen Ester von Ascorbinsäure mit Fettsäuren, insbesondere Ascorbyllinoleat, Ascorbyloleat, Ascorbyloleinat, Ascorbylpalmitat, Ascorbylstearat, Ascorbyloleat (6-Palmitoyl-L-ascorbinsäure). 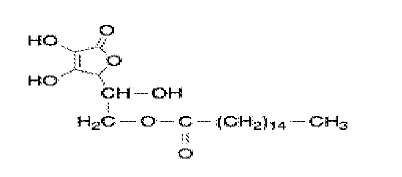 ist erfindungsgemäß besonders bevorzugt, ebenso Ascorbyltetraisopalmitat.

Auch "anorganische" Säuren können mit Ascorbinsäure verestert werden. Unter den "anorganischen" Estern der Ascorbinsäure ist insbesondere das Ascorbylphosphat bevorzugt.

Auch Ascorbinsäurederivate mit glycosidisch gebundenen Zuckern sind erfindungsgemäß mit besonderem Vorzug einsetzbar. Bewährt hat sich hier insbesondere das Ascorbylglucosid.

Bevorzugt sind erfindungsgemäße kosmetische oder dermatologische Zusammensetzungen, die Ascorbylphosphat und/oder Ascorbylglucosid und/oder Ascorbyltetraisopalmitat enthalten.

Die erfindungsgemäßen Zusammensetzungen können weitere Inhaltstoffe von Kosmetika enthalten, die weiter unten beschrieben werden. Erfindungsgemäß bevorzugt ist es aber auch, bestimmte Inhaltsstoffe nicht in die erfindungsgemäßen Zusammensetzungen zu inkorporieren. Hier sind insbesondere erfindungsgemäße kosmetische oder dermatologische Zusammensetzungen bevorzugt, die kein Retinol und keine Retinolderivate enthalten.

Das Retinol (Vitamin A₁) gehört gemeinsam mit dem 3,4-Didehydroretinol (Vitamin A₂) zur Gruppe der als Vitamin A bezeichneten Substanzen. Das β-Carotin ist das Provitamin des Retinols. Vorzugsweise sind die erfindungsgemäßen Zusammensetzungen frei von diesen Substanzen, enthalten also weder Retinol, noch 3,4-Didehydroretinol, noch beta-Carotin, noch Vitamin A-Säure und deren Ester, Vitamin A-Aldehyd und Vitamin A-Alkohol sowie dessen Ester wie das Palmitat und das Acetat.

Eine weitere Gruppe von bevorzugten Inhaltsstoffen der erfindungsgemäßen kosmetischen oder dermatologischen Zusammensetzungen sind Vitamine, Provitamine oder Vitaminvorstufen, wobei - wie vorstehend beschrieben - Retinol vorzugsweise nicht enthalten ist. Diese werden nachfolgend beschrieben:
Zur Vitamin B-Gruppe oder zu dem Vitamin B-Komplex gehören u. a.
   - Vitamin B₁ (Thiamin)
   - Vitamin B₂ (Riboflavin)
   - Vitamin B₃. Unter dieser Bezeichnung werden häufig die Verbindungen Nicotinsäure und
      Nicotinsäureamid (Niacinamid) geführt. Erfindungsgemäß bevorzugt ist das Nicotinsäureamid, das in den erfindungsgemäß verwendetenen Mitteln bevorzugt in Mengen von 0,05 bis 1 Gew.-%, bezogen auf das gesamte Mittel, enthalten ist.
   - Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der
      Panthenolmonoethylether und dessen Monoacetat sowie die in der WO 92/13829 offenbarten kationischen Panthenolderivate. Die genannten Verbindungen des Vitamin B₅-Typs sind in den erfindungsgemäßen Mitteln bevorzugt in Mengen von 0,05 - 10 Gew.-%, bezogen auf das gesamte Mittel, enthalten. Mengen von 0,1 - 5 Gew.-% sind besonders bevorzugt.
   - Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Erfindungsgemäß besonders bevorzugte Zusammensetzungen sind **dadurch gekennzeichnet, dass** sie mindestens eine Vitamin B₆-Komponente in einer
      Gesamtmenge von 0,0001 bis 1,0 Gew.-%, insbesondere in Mengen von 0,001 bis 0,01 Gew.-%, enthalten.
   - Vitamin B₇ (Biotin), auch als Vitamin H oder "Hautvitamin" bezeichnet. Bei Biotin handelt es sich um (3a*S*,4*S*, 6a*R*)-2-Oxohexahydrothienol[3,4-*d*]-imidazol-4-valeriansäure. Erfindungsgemäß besonders bevorzugte Zusammensetzungen sind **dadurch gekennzeichnet, dass** sie mindestens eine Komponente, ausgewählt aus Biotin und den Biotinestern, in einer Gesamtmenge von 0,0001 bis 1,0 Gew.-%, insbesondere 0,001 bis 0,01 Gew.-%, enthalten.
   - Folsäure (Vitamin B₉, Vitamin B_{c}). Internationaler Freiname für N-[4-(2-Amino-3,4-dihydro-4-oxo-6-pteridinylmethylamino)-benzoyl]-L-glutaminsäure (N-Pteroyl-L-glutaminsäure, PteGlu). Folat wird synonym zu Pteroylglutamat gebraucht, Folate ist der Sammelbegriff für alle Folsäure-wirksamen Verbindungen und bezeichnet eine Substanzklasse, die einen mit 4-Aminobenzoesäure und L-Glutaminsäure verbundenen Pteridin-Ring enthält. Folsäure ist ein Wachstumsfaktor für verschiedene Mikroorganismen und eine Verbindung mit Vitamincharakter, die in der Natur meist als Polyglutamat und in reduzierter Form (7,8-Dihydrofolsäure, H₂Folat, DHF; Tetrahydrofolsäure, H₄Folat, THF; 5'-Methyl-Tetrahydrofolsäure, CH₃-H₄Folat, MeTHF) vorkommt. Erfindungsgemäß besonders bevorzugte Zusammensetzungen sind dadurch gekennzeichnet, dass sie mindestens eine Komponente, ausgewählt aus Folsäure, Folaten und deren
      Estern, in einer Gesamtmenge von 0,0001 bis 1,0 Gew.-%, insbesondere 0,01 bis 0,5 Gew.-%, bezogen auf die Zusammensetzung, enthalten.
   - Orotsäure (Vitamin B₁₃, 1,2,3,6-Tetrahydro-2,6-dioxo-4-pyrimidin-carbonsäure, Uracil-6-carbonsäure, Molkensäure). Orotsäure, ihr Cholinester oder Orotsäure-Metallsalze (Orotate von Ca,
      Cr, Fe, K, Co, Cu, Li, Mg, Mn, Na, Zn, Sn) sind erfindungsgemäß besonders bevorzugt. Erfindungsgemäß besonders bevorzugte Zusammensetzungen sind **dadurch gekennzeichnet, dass** sie mindestens eine Komponente, ausgewählt aus Orotsäure, Orotaten und deren Estern, in einer Gesamtmenge von 0,0001 - 1,0 Gew.-%, insbesondere 0,01 - 0,5 Gew.-%, bezogen auf die Zusammensetzung, enthalten.
   Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten.
   Vitamin F. Unter dem Begriff "Vitamin F" werden üblicherweise essentielle Fettsäuren, insbesondere Linolsäure, Linolensäure und Arachidonsäure, verstanden.
   Zusammenfassend sind erfindungsgemäße kosmetische oder dermatologische Zusammensetzungen bevorzugt , die Vitamine, Pro-Vitamine und Vitaminvorstufen enthalten, die den Gruppen B, C, E und F zugeordnet werden, wobei bevorzugte Zusammensetzungen die genannten Verbindungen in Mengen von 0,1 bis 5 Gew.-%, vorzugsweise von 0,25 bis 4 Gew.-% und insbesondere von 0,5 bis 2,5 Gew.-%, jeweils bezogen auf das gesamte Mittel, enthalten.
   In einer weiteren bevorzugten Ausführungsform enthalten die erfindungsgemäßen Zusammensetzungen mindestens eine Substanz, ausgewählt aus der Gruppe, bestehend aus Biotin, Biotinestern, Folsäure und Folsäureestern, in einer Gesamtmenge von 0,0001 bis 2 Gew.-%, vorzugsweise 0,01 bis 1 Gew.-%, jeweils bezogen auf das Gewicht der gesamten Zusammensetzung.
   Ebenfalls bevorzugt ist der Einsatz von Taurin. Hier sind erfindungsgemäße kosmetische oder dermatologische Zusammensetzungen bevorzugt, die zusätzlich 0,5 bis 2,5 Gew.-%, vorzugsweise 0,75 bis 1,5 Gew.-% und insbesondere 1 bis 1,25 Gew.-% 2-Aminoethansulfonsäure (Taurin) enthalten.
   Es ist auch vorteilhaft im Sinne der vorliegenden Erfindung, kosmetische oder dermatologische Zusammensetzungen bereitzustellen, mindestens eine UV-Filtersubstanz enthalten. Die erfindungsgemäß bevorzugten UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)-oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.
   Bevorzugt sind solche UV-Filter, deren molarer Extinktionskoeffizient am Absorptionsmaximum oberhalb von 15 000, insbesondere oberhalb von 20000, liegt.
   Erfindungsgemäß besonders bevorzugte Zusammensetzungen sind **dadurch gekennzeichnet, dass** sie mindestens eine UV-Filtersubstanz aus den nachfolgend dargestellten Gruppen (i) = Alkyl-und/oder Alkoxy-substituierte Dibenzoylmethanderivate, (ii) = Polysilicone-15 oder (iii) = Derivate der Benzimidazolsulfonsäure enthalten.
   i) Alkyl- und/oder Alkoxy-substituiertes Dibenzoylmethanderivat
      Erfindungsgemäß bevorzugte Alkyl- und/oder Alkoxy-substituierte Dibenzoylmethanderivate sind **dadurch gekennzeichnet, dass** einer der Phenylreste mit mindestens einer Alkylgruppe, ausgewählt aus Methyl, Ethyl, n-Propyl, Isopropyl, n-Butyl, sec-Butyl, tert.-Butyl, n-Pentyl, Isopentyl, Neopentyl, 2-Ethylhexyl, und der andere Phenylrest mit mindestens einer Alkoxy-Gruppe, ausgewählt aus Methoxy, Ethoxy, n-Propoxy, Isopropoxy, n-Butoxy, sec-Butoxy, tert.Butoxy, n-Pentoxy, Isopentoxy, Neopentoxyl, 2-Ethylhexoxy, substituiert ist. Besonders bevorzugte Substituenten sind Isopropyl, tert.-Butyl und Methoxy. Erfindungsgemäß bevorzugte Alkyl- und/oder Alkoxy-substituierte Dibenzoylmethanderivate sind ausgewählt aus 2-Methyldibenzoylmethan, 4-Methyldibenzoylmethan, 4-Isopropyldibenzoylmethan, 4-tert-Butyldibenzoylmethan, 2,4-Dimethyldibenzoylmethan, 2,5-Dimethyldibenzoylmethan, 4,4'-Diisopropyldibenzoylmethan, 4,4'-Dimethoxydibenzoylmethan, 4-tert-Butyl-4'-Methoxydibenzoylmethan, 2-Methyl-5-isopropyl-4'-methoxydibenzoyl-methan, 2-Methyl-5-tert-butyl-4'-methoxydibenzoylmethan, 2,4-Dimethyl-4'-methoxydibenzoyl-methan, 2,6-Dimethyl-4-tert-butyl-4'-methoxydibenzoylmethan. Besonders bevorzugt ist 4-tert-Butyl-4'-Methoxydibenzoylmethan mit der INCI-Bezeichnung Butyl Methoxydibenzoylmethane (auch als 1-(4'-tert-Butylphenyl)-3-(4'-methoxyphenyl)propan-1,3-dion bezeichnet), die z. B. als PARSOL^{®} 1789 von DSM oder als Eusolex^{®} 9020 von Merck KGaA erhältlich ist.
   ii) Polysilicone-15
      Die UV-B-Filtersubstanz mit der INCI-Bezeichnung Polysilicone-15 wird auch als 3-(4-(2,2-Bis-Eth-oxycarbonylvinyl)-phenoxy)propenyl)-methoxysiloxan/Dimethylsiloxan-Copolymer mit der Parsol^{®} SLX), Dimethicodiethylbenzalmalonat, Diethylbenzylidene Malonate Dimethicone oder Diethylmalonylbenzylidene Oxypropene Dimethicone bezeichnet und ist unter dem Handelsnamen Parsol^{®} SLX (INCI-Bezeichnung Dimethicodiethylbenzal malonate (CAS-Nr. 207574-74-1)) von DSM erhältlich. Die chemische Struktur ist beispielsweise in EP 709080 A2 beschrieben.
   iii) Derivat der Benzimidazolsulfonsäure Bevorzugte UV-Filtersubstanz(en) der Komponente (iii) der erfindungsgemäßen Zusammensetzungen ist bzw. sind die Phenylen-1,4-bis-(2-benzimidazyl)-3,3'-5,5'-tetrasulfonsäure (UV-A) und ihre Salze, insbesondere die Alkali-, Erdalkali-, Ammonium-, Alkylammonium-, Alkanolammonium- und
   Glucammoniumsalze, bevorzugt die entsprechenden Natrium-, Kalium-, Trialkylammonium- oder Triethanolamin-Salze, insbesondere das Phenylen-1,4-bis-(2-benzimidazyl)-3,3'-5,5'-tetrasulfon-säure-bis-natriumsalz mit der INCI-Bezeichnung "Disodium Phenyl Dibenzimidazole Tetrasulfonate" (CAS-Nr.: 180898-37-7), das beispielsweise unter der Handelsbezeichnung Neo Heliopan AP von Symrise erhältlich ist. Weitere bevorzugte UV-Filtersubstanz(en) der Komponente (iii) der erfindungsgemäßen Zusammensetzungen ist bzw. sind die 2-Phenylbenzimidazol-5-sulfonsäure (UV-B) und ihre Salze, insbesondere die Alkali-, Erdalkali-, Ammonium-, Alkylammonium-, Alkanolammonium- und Glucammoniumsalze, bevorzugt die entsprechenden Natrium-, Kalium-, Trialkylammonium- oder Triethanolamin-Salze, insbesondere aber die 2-Phenylbenzimidazol-5-sulfonsäure selbst mit der INCI-Bezeichnung "Phenylbenzimidazole sulfonic acid" (CAS.-Nr. 27503-81-7), die beispielsweise unter den Handelsnamen Neo Heliopan Hydro von Symrise oder Eusolex 232 von Merck KGaA erhältlich ist.
   In einer erfindungsgemäß besonders bevorzugten Ausführungsform sind die Salze der 2-Phenylbenzimidazol-5-sulfonsäure ausgewählt aus den Salzen dieser Säure mit basischen Aminosäuren, insbesondere mit Lysin, Arginin und/oder Histidin, wobei Arginin besonders bevorzugt ist.
   In einer weiteren erfindungsgemäß besonders bevorzugten Ausführungsform sind die Salze der Phenylen-1,4-bis-(2-benzimidazyl)-3,3'-5,5'-tetrasulfonsäure ausgewählt aus den Salzen dieser Säure mit basischen Aminosäuren, insbesondere mit Ornithin, Lysin, Arginin und/oder Histidin, wobei Arginin besonders bevorzugt ist.
   Erfindungsgemäß besonders bevorzugte Lichtschutzfilter-Kombinationen (i), (ii) und (iii) umfassen 4-tert-Butyl-4'-Methoxydibenzoylmethan, Polysilicone-15 und 2-Phenylbenzimidazol-5-sulfonsäure. Weitere erfindungsgemäß besonders bevorzugte Lichtschutzfilter-Kombinationen (i), (ii) und (iii) umfassen 4-tert-Butyl-4'-Methoxydibenzoylmethan, Polysilicone-15 und Dinatriumphenylbenzimidazoltetrasulfonat.
   Weitere erfindungsgemäß besonders bevorzugte Lichtschutzfilter-Kombinationen (i), (ii) und (iii) umfassen 4-tert-Butyl-4'-Methoxydibenzoylmethan, Polysilicone-15 und ein 2-Phenylbenzimidazol-5-sulfonsäure-Arginin-Salz.
   Die oben genannten UV-Filtersubstanzen der Komponente (i sind bevorzugt in einer Gesamtmenge von 0,5 Gew.-% bis 20 Gew.-%, besonders bevorzugt 1 bis 15 Gew.-%, insbesondere 2 bis 10 Gew.-% und außerordentlich bevorzugt 3 - 5 Gew.-%, enthalten, jeweils bezogen auf das Gesamtgewicht der erfindungsgemäßen Zusammensetzungen.
   Polysilicone-15, die oben genannte Komponente (ii), ist bevorzugt in einer Gesamtmenge von 0,5 Gew.-% bis 10 Gew.-%, besonders bevorzugt 1 bis 7 Gew.-%, insbesondere 2 bis 5 Gew.-% und außerordentlich bevorzugt 3 - 4 Gew.-%, enthalten, jeweils bezogen auf das Gesamtgewicht der erfindungsgemäßen Zusammensetzungen.
   Die oben genannten UV-Filtersubstanzen der Komponente (iii) sind bevorzugt in einer Gesamtmenge von 0,5 Gew.-% bis 15 Gew.-%, besonders bevorzugt 1 bis 10 Gew.-%, insbesondere 2 bis 5 Gew.-% und außerordentlich bevorzugt 3 - 4 Gew.-%, enthalten, jeweils bezogen auf das Gesamtgewicht der erfindungsgemäßen Zusammensetzungen.
   Bevorzugte erfindungsgemäße Zusammensetzungen sind **dadurch gekennzeichnet, dass** die UV-Filter (i), (ii) und (iii) in einem Gewichtsverhältnis zueinander von (i) : (ii): (iii) wie (0,8 - 1,5) : (0,8 - 1,5) : (0,8 - 1,5), bevorzugt wie (0,9 - 1,2) : (0,9 - 1,2) : (0,9 - 1,2), besonders bevorzugt wie (1 - 1,1) : (1 - 1,1) : (1 - 1,1), enthalten sind.
   Um den Lichtschutzfaktor weiter zu erhöhen, können die erfindungsgemäßen Zusammensetzungen bevorzugt mindestens eine weitere organische und/oder anorganische UV-Filtersubstanz enthalten. Weitere bevorzgte UV-Filtersubstanzen, die neben denen der Komponenten (i), (ii) und (iii) in den erfindungsgemäßen Zusammensetzungen eingesetzt werden, sind im Folgenden genannt.
   Triazinderivate
   UV-Filtersubstanzen auf Basis von Triazinderivaten, die das nachfolgende Strukturmotiv 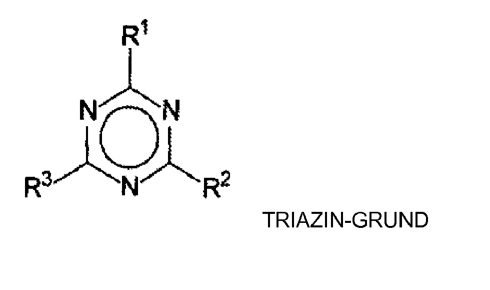 aufweisen, sind im Stand der Technik bekannt, beispielsweise aus EP 775698, EP 878469 und EP 1027881.
   Hinsichtlich der C₃-Achse des Triazin-Grundkörpers dieser Verbindungen sind sowohl symmetrische Substitution als auch unsymmetrische Substitution denkbar.
   Hinsichtlich der C₃-Achse des Triazin-Grundkörpers symmetrische Triazinderivate sind bevorzugt solche der allgemeinen Formel TRIAZIN-GRUND mit R¹ = R² = R³ = -NH-Phe-COOR, mit Phe = Phenyl-Rest, bei dem die Substituenten -NH und -COOR in para-Position zueinander stehen. Besonders bevorzugte derartige symmetrische Triazinderivate sind 4,4',4"-(1,3,5-Triazin-2,4,6-triyltriimino)-tris-benzoesäure-tris(alkylester). Ein besonders bevorzugter derartiger Ester ist 4,4',4"-(1,3,5-Triazin-2,4,6-triyltrümino)-tris-benzoesäure-tris(2-ethylhexylester) [INCI: Ethylhexyl Triazone, vormals Octyl Triazone], das als Einzelsubstanz von BASF unter dem Handelsnamen UVINUL® T 150 vertrieben wird, aber auch in diversen kommerziellen UV-Filtermischungen erhältlich ist.
   Erfindungsgemäß besonders bevorzugt ist Ethylhexyl Triazone.
   Erfindungsgemäß bevorzugte unsymmetrische Triazinderivate sind beispielsweise solche, die in EP 775698 offenbart sind: 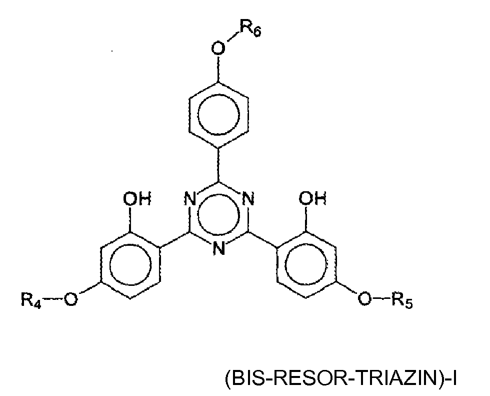 und/oder

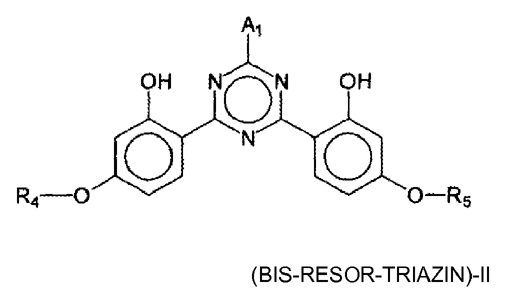

Alle in EP 775698 erwähnten so genannten Bis-Resorcinyltriazine, seien sie durch generische oder durch konkrete Formeln offenbart, sind vorteilhaft im Sinne der vorliegenden Erfindung.

Ganz besonders bevorzugt werden R₄ und R₅ aus der Gruppe der verzweigten und unverzweigten Alkylgruppen von 1 bis 18 Kohlenstoffatomen gewählt. Auch können die Alkylgruppen wiederum vorteilhaft mit Silyloxygruppen substituiert sein. A₁ stellt bevorzugt einen substituierten homo- oder heterocyclischen aromatischen Fünfring oder Sechsring dar. Ganz besonders bevorzugte optionale UV-Filtersubstanzen sind ausgewählt aus unsymmetrisch substituierten s-Triazin-Verbindungen der allgemeinen Formel (BIS-RESOR-TRIAZIN)-I, in der R₆ ein Wasserstoffatom oder eine verzweigte oder unverzweigte Alkylgruppe mit 1 bis 10 Kohlenstoffatomen darstellt. Eine besonders bevorzugte Verbindung der allgemeinen Formel (BIS-RESOR-TRIAZIN)-I, in der R₄ und R₅ jeweils eine 2-Ethylhexyl-Gruppe und R₆ eine Methylgruppe darstellen, ist 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazine), unter dem Handelsnamen Tinosorb^{®} S von CIBA erhältlich.

Eine weitere, erfindungsgemäß bevorzugte optionale unsymmetrisch substituierte s-Triazin-Verbindung ist eine Verbindung mit der INCI-Bezeichnung Diethylhexyl Butamido Triazone, mit der allgemeinen Formel TRIAZIN-GRUND mit R¹ = R² -NH-Phe-COOR, mit Phe = Phenyl-Rest, bei dem die Substituenten -NH und -COOR in para-Position zueinander stehen, R = 2-Ethylhexyl und R³ = -NH-Phe-CONH-tert-Butyl, mit Phe = Phenyl-Rest, bei dem die Substituenten -NH und -CONH-tert.-Butyl in para-Position zueinander stehen. Diese UV-Filtersubstanz, ein effektiver UV-A-Filter, ist unter der Handelsbezeichnung UVASORB HEB bei Sigma 3V erhältlich ist. Weitere, erfindungsgemäß bevorzugte optionale UV-Filtersubstanzen auf Basis von s-Triazin-Verbindungen sind: 2,4,6-Tris([1,1'-Biphenyl]-4-yl)-1,3,5-Triazine, INCI: Tris-Biphenyl Triazine, erhältlich unter dem Handelsnamen Tinosorb A2B von CIBA, 2,4-bis-[5-1(di-methylpropyl)benzoxazol-2-yl-(4-phenyl)-imino]-6-(2-ethylhexyl)-imino-1,3,5-triazin (CAS Nr. 288254-16-0, Uvasorb^{®} K2A von 3V Sigma, INCI: Ethylhexyl Bis-Isopentylbenzoxazolylphenyl Melamine), 2,4-Bis-{[4-(3-sulfona-to)-2-hydroxy-propyloxy)-2-hydroxy]-phenyl}-6-(4-methoxyphenyl)-1,3,5-triazin-Natriumsalz, 2,4-Bis-{[4-(3-(2-propyloxy)-2-hydroxy-propyloxy)-2-hydroxy]-phenyl}-6-(4-methoxyphenyl)-1,3,5-triazin, 2,4-Bis-{[4-(2-ethyl-hexyloxy)-2-hydroxy]-phenyl}-6-[4-(2-methoxyethyl-carboxyl)-phenylamino]-1,3,5-triazin, 2,4-Bis-{[4-(3-(2-propyloxy)-2-hydroxy-propyloxy)-2-hydroxy]-phenyl}-6-[4-(ethylcarboxyl)-phenylamino]-1, 3,5-triazin, 2,4-Bis-{[4-(2-ethyl-hexyloxy)-2-hydroxy]-phenyl}-6-(1-methyl-pyrrol-2-yl)-1,3,5-triazin, 2,4-Bis-{[4-tris(trimethylsiloxy-silylpropyloxy)-2-hydroxy]-phenyl}-6-(4-methoxyphenyl)-1,3,5-triazin, 2,4-Bis-([4-(2-methylpropenyloxy)-2-hydroxy]-phenyl}-6-(4-methoxyphenyl)-1,3 ,5-triazin und 2,4-Bis-{[4-(1',1',1',3',5',5',5'-Heptamethylsiloxy-2-methyl-propyloxy)-2-hydroxy]-phenyl}-6-(4-methoxyphenyl)-1,3,5-triazin,2-[4,6-Bis(2,4-dimethylphenyl)-1,3,5-triazin-2-yl]-5-(ethylhexyloxy)phenol. Die s-Triazinderivate werden bevorzugt in die Ölphase der erfindungsgemäßen kosmetischen oder dermatologischen Zusammensetzungen eingearbeitet. Benzotriazole Eine weitere bevorzugte optionale UV-Filtersubstanz ist 2,2'-Methylen-bis-(6-(2H-benzotriazol-2-yl)-4-(1,1,3,3-tetramethylbutyl)-phenol) (INCI: Methylene Bis-Benzotriazolyl Tetramethylbutylphenol, MBBT) mit folgender Strukturformel 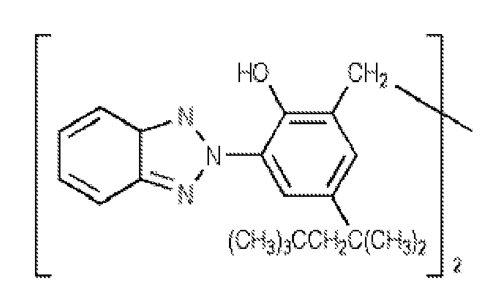 , unter der Handelsbezeichnung Tinosorb^{®} M von CIBA erhältlich. Hierbei handelt es sich um einen so genannten Breitbandfilter, der sowohl UV-A- als auch UV-B-Strahlung absorbiert.

Eine weitere bevorzugte optionale Breitband-UV-Filtersubstanz ist 2-(2H-Benzotriazol-2-yl)-4-methyl-6-[2-methyl-3-[1,3,3,3-tetramethyl-1-((trimethylsilyl)oxy]disiloxanyl]propyl]-phenol (CAS-Nr.: 155633-54-8) mit der INCI-Bezeichnung Drometrizole Trisiloxane.

Weitere bevorzugte optionale Benzotriazol-Filter sind 2,2'-Methyl-bis-[6(2H-benzotriazol-2-yl)-4-(methyl)phenol] (MIXXIM BB/200 der Firma Fairmount Chemical), 2-(2'-Hydroxy-3',5'-di-t-amyl-phenyl)benzotriazol (CAS- Nr.: 025973-551), 2-(2'-Hydroxy-5'-octylphenyl)-benzotriazol (CAS-Nr. 003147-75-9), 2-(2'-Hydroxy-5'-methylphenyl)benzotriazol (CAS-Nr. 2440-22-4) und 2-(2H-benzotriazol-2-yl)-4-methyl-6-[2-methyl-3-[1,3,3,3-tetramethyl-1-((trimethylsilyl)oxy]disiloxanyl)propyl]-phenol (CAS-Nr.: 155633-54-8) mit der INCI-Bezeichnung Drometrizole Trisiloxane.

Weitere bevorzugte UV-Filtersubstanzen, die neben denen der Komponenten (i), (ii) und (iii) in den erfindungsgemäßen Zusammensetzungen eingesetzt werden, sind im Folgenden genannt: Derivate des Camphers, insbesondere 3-Benzylidencampher-Derivate, die keine ionisierbaren funktionellen Gruppen im Molekül aufweisen können, bevorzugt 3-(4'-Methylbenzyliden)-D,L-campher [INCI: 4-Methylbenzylidene Camphor], das von Merck unter der Warenbezeichnung Eusolex 6300 vertrieben wird; Derivate des Camphers, die ionisierbare funktionelle Gruppen im Molekül aufweisen können, bevorzugt Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze; das 1,4-di(2-oxo-10-Sulfo-3-bornylidenmethyl)-Benzol und dessen Salze (besonders die entsprechenden 10-Sulfato-verbindungen, insbesondere das entsprechende Natrium-, Kalium-oder Triethanolammonium-Salz), das auch als Benzol-1,4-di(2-oxo-3-bornylidenmethyl-10-sulfonsäure) bezeichnet wird, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze mit der INCI-Bezeichnung Terephthalylidene Dicamphor Sulfonic Acid (CAS.-Nr.: 92761-26-7, als Mexoryl SX von der Firma Chimex erhältlich). 4-Aminobenzoësäure-Derivate, bevorzugt 4-(Dimethylamino)-benzoësäure(2-ethylhexyl) ester, 4-(Dimethylamino)benzoësäureamylester; 2-Aminobenzoesäure-Derivate, Ester der Zimtsäure, bevorzugt 4-Methoxyzimtsäure(2-ethylhexyl)-ester, 4-Methoxyzimtsäurepropylester, 4-Methoxyzimtsäureisopentylester; und 2-Cyano-3,3-phenylzimtsäure-2-ethylhexylester (= Ethylhexyl-2-cyano-3,3-diphenylacrylat (Octocrylene)), Ester der Salicylsäure, bevorzugt Salicylsäure(2-ethylhexyl)ester (2-Ethylhexylsalicylat (= Octylsalicylat)), Salicylsäure(4-isopropylbenzyl)ester (4-Isopropylbenzylsalicylat), Salicylsäurehomomenthylester (Homomenthylsalicylat, Homosalate), Derivate des Benzophenons, die keine ionisierbaren funktionellen Gruppen im Molekül aufweisen, bevorzugt 2-Hydroxy-4-methoxy-4'-methylbenzophenon, 2,2'-Dihydroxy-4-methoxybenzophenon, 2-Hydroxy-4-methoxybenzophenon, 2-Hydroxy-4-methoxy-4'-methylbenzophenon, 2,2'-Dihydroxy-4-methoxybenzophenon; 2-(4'-Diethylamino-2'-hydroxybenzoyl)-benzoesäurehexylester (auch: Aminobenzophenon, unter der Bezeichnung Uvinul A Plus bei der Firma BASF erhältlich), Derivate des Benzophenons, die ionisierbare funktionelle Gruppen im Molekül aufweisen, bevorzugt Sulfonsäurederivate von Benzophenonen, besonders bevorzugt 2-Hydroxy-4-methoxybenzophenon-5-sulfonsäure und ihre Salze, Ester der Benzalmalonsäure, bevorzugt 4-Methoxybenzalmalonsäuredi(2-ethylhexyl)ester, sowie an Polymere gebundene UV-Filter, die von Komponente (b) verschieden sind, Derivate von Benzoxazol, bevorzugt 2,2'(Naphthalene-1,4-Diyl)bis(benzoxazole) (INCI: Dibenzoxazoyl Naphthalene) und 2,4-bis-[5-1(di-methylpropyl)benzoxazol-2-yl-(4-phenyl)-imino]-6-(2-ethylhexyl)-imino-1,3,5-triazin (Uvasorb^{®} K2A von 3V Sigma, INCI: Ethylhexyl Bis-Isopentylbenzoxazolylphenyl Melamine). Die Benzoxazol-Derivate liegen bevorzugt in gelöster Form in den erfindungsgemäßen kosmetischen Zusammensetzungen vor. Es kann ggf. aber auch von Vorteil sein, wenn die Benzoxazol-Derivate in pigmentärer, d. h. ungelöster Form - beispielsweise in Partikelgrößen von 10 nm bis zu 300 nm - vorliegen.

Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'methoxyphenyl)propan-1,3-dion (z. B. Parsol^{®} 1789) in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.

Die Gesamtmenge an der mindestens einen optionalen organischen UV-Filtersubstanz in den erfindungsgemäßen kosmetischen oder dermatologischen Zusammensetzungen beträgt bevorzugt 0,5 - 20 Gew.-%, besonders bevorzugt 1 - 15 Gew.-%, außerordentlich bevorzugt 2 - 10 Gew.-%, jeweils bezogen auf das Gesamtgewicht der erfindungsgemäßen Zusammensetzung.

Bei den erfindungsgemäß bevorzugten optionalen anorganischen UV-Filtersubstanzen handelt es sich bevorzugt um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und

Bariumsulfat. Besonders bevorzugt sind Titandioxid und Zinkoxid. Die Partikel sollten dabei einen mittleren Durchmesser von weniger als 100 nm, bevorzugt zwischen 5 und 50 nm und insbesondere zwischen 15 und 30 nm aufweisen, also so genannte Nanopigmente darstellen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex^{®} T2000 (Merck). Als hydrophobe Coatingmittel kommen dabei vor allem Silicone und dabei speziell Trialkoxyoctylsilane, bevorzugt Triethoxy Caprylylsilane, oder Simethicone in Frage. Weitere bevorzugte Coatingmittel sind Aluminiumoxide. Weitere bevorzugte Coatingmittel ist Siliciumdioxid, z. B. bei dem Handelsprodukt Eusolex T AVO von Merck KGaA. Ein weiteres bevorzugtes Coatingmittel ist Stearinsäure. Ein weiteres bevorzugtes Coatingmittel ist Polyhydroxystearinsäure. Ein weiteres bevorzugtes Coatingmittel ist Aluminiumstearat. Ein besonders bevorzugter anorganischer UV-Filter ist ein mit Triethoxy Caprylylsilane hydrophob beschichtetes Titandioxid, erhältlich unter der Bezeichnung Titan M 265 von Kemira. Erfindungsgemäß bevorzugt ist mindestens eine anorganische UV-Filtersubstanz in einer Gesamtmenge von 0,1 - 15 Gew.-%, besonders bevorzugt 0,5 - 10 Gew.-%, außerordentlich bevorzugt 1,0 - 5 Gew.-% und weiter bevorzugt 2,0 - 4,0 Gew.-%, jeweils bezogen auf die gesamte Zusammensetzung, enthalten.

Besonders bevorzugte Zusammensetzungen enthalten 0,01 - 5 Gew.-% 8-Hexadecen-1,16-dicarbonsäure, 0,0001 bis 5 Gew.-% Sulforaphan und Butyl Methoxydibenzoylmethane.

Weitere besonders bevorzugte Zusammensetzungen enthalten 0,01 - 5 Gew.-% 8-Hexadecen-1,16-dicarbonsäure, 0,0001 bis 5 Gew.-% Sulforaphen und Butyl Methoxydibenzoylmethane.

Weitere besonders bevorzugte Zusammensetzungen enthalten 0,01 - 5 Gew.-% 8-Hexadecen-1,16-dicarbonsäure, 0,0001 bis 5 Gew.-% Allylisothiocyanat und Butyl Methoxydibenzoylmethane.

Weitere besonders bevorzugte Zusammensetzungen enthalten 0,01 - 5 Gew.-% 8-Hexadecen-1,16-dicarbonsäure, 0,0001 bis 5 Gew.-% Benzylisothiocyanat und Butyl Methoxydibenzoylmethane.

Weitere besonders bevorzugte Zusammensetzungen enthalten 0,01 - 5 Gew.-% 8-Hexadecen-1,16-dicarbonsäure, 0,0001 bis 5 Gew.-% Phenylethylisothiocyanat und Butyl Methoxydibenzoylmethane.

Weitere besonders bevorzugte Zusammensetzungen enthalten 0,01 - 5 Gew.-% 8-Hexadecen-1,16-dicarbonsäure, 0,0001 bis 5 Gew.-% Ethylisothiocyanat und Butyl Methoxydibenzoylmethane.

Weitere besonders bevorzugte Zusammensetzungen enthalten 0,01 - 5 Gew.-% 8-Hexadecen-1,16-dicarbonsäure, 0,0001 bis 5 Gew.-% Barbarin und Butyl Methoxydibenzoylmethane.

Weitere besonders bevorzugte Zusammensetzungen enthalten 0,01 - 5 Gew.-% 8-Hexadecen-1,16-dicarbonsäure, 0,0001 bis 5 Gew.-% Goitrin und Butyl Methoxydibenzoylmethane.

Weitere besonders bevorzugte Zusammensetzungen enthalten 0,01 - 5 Gew.-% 8-Hexadecen-1,16-dicarbonsäure, 0,0001 bis 5 Gew.-% Iberin und Butyl Methoxydibenzoylmethane.

Weitere besonders bevorzugte Zusammensetzungen enthalten 0,01 - 5 Gew.-% 8-Hexadecen-1,16-dicarbonsäure, 0,0001 bis 5 Gew.-% Brassicin und Butyl Methoxydibenzoylmethane.

Weitere besonders bevorzugte Zusammensetzungen enthalten 0,01 - 5 Gew.-% 8-Hexadecen-1,16-dicarbonsäure, 0,0001 bis 5 Gew.-% Nasturtiin und Butyl Methoxydibenzoylmethane.

Besonders bevorzugte erfindungsgemäße Zusammensetzungen enthalten 0,01 - 5 Gew.-% 8-Hexadecen-1,16-dicarbonsäure, 0,0001 bis 5 Gew.-% Sulforaphan und Polysilicone-15.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Sulforaphen und Polysilicone-15.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Allylisothiocyanat und Polysilicone-15.

Weitere besonders bevorzugte Zusammensetzungen enthalten 0,01 - 5 Gew.-% 8-Hexadecen-1,16-dicarbonsäure, 0,0001 bis 5 Gew.-% Benzylisothiocyanat und Polysilicone-15.

Weitere besonders bevorzugte Zusammensetzungen enthalten 0,01 - 5 Gew.-% 8-Hexadecen-1,16-dicarbonsäure, 0,0001 bis 5 Gew.-% Phenylethylisothiocyanat und Polysilicone-15.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Ethylisothiocyanat und Polysilicone-15.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Barbarin und Polysilicone-15.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Goitrin und Polysilicone-15.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Iberin und Polysilicone-15.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Brassicin und Polysilicone-15.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Nasturtiin und Polysilicone-15.

Besonders bevorzugte Zusammensetzungen enthalten 0,01 - 5 Gew.-% 8-Hexadecen-1,16-dicarbonsäure, 0,0001 bis 5 Gew.-% Sulforaphan und 2-Phenylbenzimidazol-5-sulfonsäure.

Weitere besonders bevorzugte Zusammensetzungen enthalten 0,01 - 5 Gew.-% 8-Hexadecen-1,16-dicarbonsäure, 0,0001 bis 5 Gew.-% Sulforaphen und 2-Phenylbenzimidazol-5-sulfonsäure.

Weitere besonders bevorzugte Zusammensetzungen enthalten 0,01 - 5 Gew.-% 8-Hexadecen-1,16-dicarbonsäure, 0,0001 bis 5 Gew.-% Allylisothiocyanat und 2-Phenylbenzimidazol-5-sulfonsäure.

Weitere besonders bevorzugte Zusammensetzungen enthalten 0,01 - 5 Gew.-% 8-Hexadecen-1,16-dicarbonsäure, 0,0001 bis 5 Gew.-% Benzylisothiocyanat und 2-Phenylbenzimidazol-5-sulfonsäure.

Weitere besonders bevorzugte Zusammensetzungen enthalten 0,01 - 5 Gew.-% 8-Hexadecen-1,16-dicarbonsäure, 0,0001 bis 5 Gew.-% Phenylethylisothiocyanat und 2-Phenylbenzimidazol-5-sulfonsäure.

Weitere besonders bevorzugte Zusammensetzungen enthalten 0,01 - 5 Gew.-% 8-Hexadecen-1,16-dicarbonsäure, 0,0001 bis 5 Gew.-% Ethylisothiocyanat und 2-Phenylbenzimidazol-5-sulfonsäure.

Weitere besonders bevorzugte Zusammensetzungen enthalten 0,01 - 5 Gew.-% 8-Hexadecen-1,16-dicarbonsäure, 0,0001 bis 5 Gew.-% Barbarin und 2-Phenylbenzimidazol-5-sulfonsäure.

Weitere besonders bevorzugte Zusammensetzungen enthalten 0,01 - 5 Gew.-% 8-Hexadecen-1,16-dicarbonsäure, 0,0001 bis 5 Gew.-% Goitrin und 2-Phenylbenzimidazol-5-sulfonsäure.

Weitere besonders bevorzugte Zusammensetzungen enthalten 0,01 - 5 Gew.-% 8-Hexadecen-1,16-dicarbonsäure, 0,0001 bis 5 Gew.-% Iberin und 2-Phenylbenzimidazol-5-sulfonsäure.

Weitere besonders bevorzugte Zusammensetzungen enthalten 0,01 - 5 Gew.-% 8-Hexadecen-1,16-dicarbonsäure, 0,0001 bis 5 Gew.-% Brassicin und 2-Phenylbenzimidazol-5-sulfonsäure.

Weitere besonders bevorzugte Zusammensetzungen enthalten 0,01 - 5 Gew.-% 8-Hexadecen-1,16-dicarbonsäure, 0,0001 bis 5 Gew.-% Nasturtiin und 2-Phenylbenzimidazol-5-sulfonsäure.

Besonders bevorzugte Zusammensetzungen enthalten 0,01 - 5 Gew.-% 8-Hexadecen-1,16-dicarbonsäure, 0,0001 bis 5 Gew.-% Sulforaphan, Butyl Methoxydibenzoylmethane und Polysilicone-15. Weitere besonders bevorzugte Zusammensetzungen enthalten 0,01 - 5 Gew.-% 8-Hexadecen-1,16-dicarbonsäure, 0,0001 bis 5 Gew.-% Sulforaphen, Butyl Methoxydibenzoylmethane und

Polysilicone-15.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Allylisothiocyanat, Butyl Methoxydibenzoylmethane und Polysilicone-15.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Benzylisothiocyanat, Butyl Methoxydibenzoylmethane und Polysilicone-15.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Phenylethylisothiocyanat, Butyl Methoxydibenzoylmethane und Polysilicone-15.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Ethylisothiocyanat, Butyl Methoxydibenzoylmethane und Polysilicone-15.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Barbarin, Butyl Methoxydibenzoylmethane und Polysilicone-15.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Goitrin, Butyl Methoxydibenzoylmethane und Polysilicone-15.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Iberin, Butyl Methoxydibenzoylmethane und Polysilicone-15.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Brassicin, Butyl Methoxydibenzoylmethane und Polysilicone-15.

Weitere besonders bevorzugte Zusammensetzungen enthalten 0,01 - 5 Gew.-% 8-Hexadecen-1,16-dicarbonsäure, 0,0001 bis 5 Gew.-% Nasturtiin, Butyl Methoxydibenzoylmethane und

Polysilicone-15.

Besonders bevorzugte erfindungsgemäße Zusammensetzungen enthalten 0,01 - 5 Gew.-% 8-Hexadecen-1,16-dicarbonsäure, 0,0001 bis 5 Gew.-% Sulforaphan, Butyl Methoxydibenzoylmethane, Polysilicone-15 und 2-Phenylbenzimidazol-5-sulfonsäure.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Sulforaphen, Butyl Methoxydibenzoylmethane, Polysilicone-15 und 2-Phenylbenzimidazol-5-sulfonsäure.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Allylisothiocyanat, Butyl Methoxydibenzoylmethane, Polysilicone-15 und 2-Phenylbenzimidazol-5-sulfonsäure.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Benzylisothiocyanat, Butyl Methoxydibenzoylmethane, Polysilicone-15 und 2-Phenylbenzimidazol-5-sulfonsäure.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Phenylethylisothiocyanat, Butyl Methoxydibenzoylmethane, Polysilicone-15 und 2-Phenylbenzimidazol-5-sulfonsäure.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Ethylisothiocyanat, Butyl Methoxydibenzoylmethane, Polysilicone-15 und 2-Phenylbenzimidazol-5-sulfonsäure.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Barbarin, Butyl Methoxydibenzoylmethane, Polysilicone-15 und 2-Phenylbenzimidazol-5-sulfonsäure.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Goitrin, Butyl Methoxydibenzoylmethane, Polysilicone-15 und 2-Phenylbenzimidazol-5-sulfonsäure.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Iberin, Butyl Methoxydibenzoylmethane, Polysilicone-15 und 2-Phenylbenzimidazol-5-sulfonsäure.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Brassicin, Butyl Methoxydibenzoylmethane, Polysilicone-15 und 2-Phenylbenzimidazol-5-sulfonsäure.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Nasturtiin, Butyl Methoxydibenzoylmethane, Polysilicone-15 und 2-Phenylbenzimidazol-5-sulfonsäure.

Besonders bevorzugte Zusammensetzungen enthalten 0,01 - 5 Gew.-% 9-Octadecen-1,18-dicarbonsäure, 0,0001 bis 5 Gew.-% Sulforaphan und Butyl Methoxydibenzoylmethane.

Weitere besonders bevorzugte Zusammensetzungen enthalten 0,01 - 5 Gew.-% 9-Octadecen-1,18-dicarbonsäure, 0,0001 bis 5 Gew.-% Sulforaphen und Butyl Methoxydibenzoylmethane.

Weitere besonders bevorzugte Zusammensetzungen enthalten 0,01 - 5 Gew.-% 9-Octadecen-1,18-dicarbonsäure, 0,0001 bis 5 Gew.-% Allylisothiocyanat und Butyl Methoxydibenzoylmethane.

Weitere besonders bevorzugte Zusammensetzungen enthalten 0,01 - 5 Gew.-% 9-Octadecen-1,18-dicarbonsäure, 0,0001 - 5 Gew.-% Benzylisothiocyanat und Butyl Methoxydibenzoylmethane.

Weitere besonders bevorzugte Zusammensetzungen enthalten 0,01 - 5 Gew.-% 9-Octadecen-1,18-dicarbonsäure, 0,0001 - 5 Gew.-% Phenylethylisothiocyanat und Butyl Methoxydibenzoylmethane.

Weitere besonders bevorzugte Zusammensetzungen enthalten 0,01 - 5 Gew.-% 9-Octadecen-1,18-dicarbonsäure, 0,0001 bis 5 Gew.-% Ethylisothiocyanat und Butyl Methoxydibenzoylmethane.

Weitere besonders bevorzugte Zusammensetzungen enthalten 0,01 - 5 Gew.-% 9-Octadecen-1,18-dicarbonsäure, 0,0001 bis 5 Gew.-% Barbarin und Butyl Methoxydibenzoylmethane.

Weitere besonders bevorzugte Zusammensetzungen enthalten 0,01 - 5 Gew.-% 9-Octadecen-1,18-dicarbonsäure, 0,0001 bis 5 Gew.-% Goitrin und Butyl Methoxydibenzoylmethane.

Weitere besonders bevorzugte Zusammensetzungen enthalten 0,01 - 5 Gew.-% 9-Octadecen-1,18-dicarbonsäure, 0,0001 bis 5 Gew.-% Iberin und Butyl Methoxydibenzoylmethane.

Weitere besonders bevorzugte Zusammensetzungen enthalten 0,01 - 5 Gew.-% 9-Octadecen-1,18-dicarbonsäure, 0,0001 bis 5 Gew.-% Brassicin und Butyl Methoxydibenzoylmethane.

Weitere besonders bevorzugte Zusammensetzungen enthalten 0,01 - 5 Gew.-% 9-Octadecen-1,18-dicarbonsäure, 0,0001 bis 5 Gew.-% Nasturtiin und Butyl Methoxydibenzoylmethane.

Besonders bevorzugte erfindungsgemäße Zusammensetzungen enthalten 0,01 - 5 Gew.-% 9-Octadecen-1,18-dicarbonsäure, 0,0001 bis 5 Gew.-% Sulforaphan und Polysilicone-15.

Weitere besonders bevorzugte erfindungsgemäße Zusammensetzungen enthalten 0,01 - 5 Gew.-% 9-Octadecen-1,18-dicarbonsäure, 0,0001 bis 5 Gew.-% Sulforaphen und Polysilicone-15.

Weitere besonders bevorzugte erfindungsgemäße Zusammensetzungen enthalten 0,01 - 5 Gew.-% 9-Octadecen-1,18-dicarbonsäure, 0,0001 bis 5 Gew.-% Allylisothiocyanat und Polysilicone-15.

Weitere besonders bevorzugte Zusammensetzungen enthalten 0,01 - 5 Gew.-% 9-Octadecen-1,18-dicarbonsäure, 0,0001 bis 5 Gew.-% Benzylisothiocyanat und Polysilicone-15.

Weitere besonders bevorzugte Zusammensetzungen enthalten 0,01 - 5 Gew.-% 9-Octadecen-1,18-dicarbonsäure, 0,0001 - 5 Gew.-% Phenylethylisothiocyanat und Polysilicone-15.

Weitere besonders bevorzugte erfindungsgemäße Zusammensetzungen enthalten 0,01 - 5 Gew.-% 9-Octadecen-1,18-dicarbonsäure, 0,0001 bis 5 Gew.-% Ethylisothiocyanat und Polysilicone-15.

Weitere besonders bevorzugte erfindungsgemäße Zusammensetzungen enthalten 0,01 - 5 Gew.-% 9-Octadecen-1,18-dicarbonsäure, 0,0001 bis 5 Gew.-% Barbarin und Polysilicone-15.

Weitere besonders bevorzugte erfindungsgemäße Zusammensetzungen enthalten 0,01 - 5 Gew.-% 9-Octadecen-1,18-dicarbonsäure, 0,0001 bis 5 Gew.-% Goitrin und Polysilicone-15.

Weitere besonders bevorzugte erfindungsgemäße Zusammensetzungen enthalten 0,01 - 5 Gew.-% 9-Octadecen-1,18-dicarbonsäure, 0,0001 bis 5 Gew.-% Iberin und Polysilicone-15.

Weitere besonders bevorzugte erfindungsgemäße Zusammensetzungen enthalten 0,01 - 5 Gew.-% 9-Octadecen-1,18-dicarbonsäure, 0,0001 bis 5 Gew.-% Brassicin und Polysilicone-15.

Weitere besonders bevorzugte erfindungsgemäße Zusammensetzungen enthalten 0,01 - 5 Gew.-% 9-Octadecen-1,18-dicarbonsäure, 0,0001 bis 5 Gew.-% Nasturtiin und Polysilicone-15.

Besonders bevorzugte Zusammensetzungen enthalten 0,01 - 5 Gew.-% 9-Octadecen-1,18-dicarbonsäure, 0,0001 bis 5 Gew.-% Sulforaphan und 2-Phenylbenzimidazol-5-sulfonsäure.

Weitere besonders bevorzugte Zusammensetzungen enthalten 0,01 - 5 Gew.-% 9-Octadecen-1,18-dicarbonsäure, 0,0001 bis 5 Gew.-% Sulforaphen und 2-Phenylbenzimidazol-5-sulfonsäure.

Weitere besonders bevorzugte Zusammensetzungen enthalten 0,01 - 5 Gew.-% 9-Octadecen-1,18-dicarbonsäure, 0,0001 - 5 Gew.-% Allylisothiocyanat und 2-Phenylbenzimidazol-5-sulfonsäure. Weitere besonders bevorzugte Zusammensetzungen enthalten 0,01 - 5 Gew.-% 9-Octa-decen-1,18-dicarbonsäure, 0,0001 - 5 Gew.-% Benzylisothiocyanat und 2-Phenylbenzimidazol-5-sulfonsäure. Weitere besonders bevorzugte Zusammensetzungen enthalten 0,01 - 5 Gew.-% 9-Octadecen-1,18-dicarbonsäure, 0,0001 bis 5 Gew.-% Phenylethylisothiocyanat und 2-Phenylbenzimidazol-5-sulfonsäure. Weitere besonders bevorzugte Zusammensetzungen enthalten 0,01 - 5 Gew.-% 9-Octadecen-1,18-dicarbonsäure, 0,0001 bis 5 Gew.-% Ethylisothiocyanat und 2-Phenylbenzimidazol-5-sulfonsäure. Weitere besonders bevorzugte Zusammensetzungen enthalten 0,01 - 5 Gew.-% 9-Octadecen-1,18-dicarbonsäure, 0,0001 bis 5 Gew.-% Barbarin und 2-Phenylbenzimidazol-5-sulfonsäure. Weitere besonders bevorzugte Zusammensetzungen enthalten 0,01 - 5 Gew.-% 9-Octadecen-1,18-dicarbonsäure, 0,0001 bis 5 Gew.-% Goitrin und 2-Phenylbenzimidazol-5-sulfonsäure. Weitere besonders bevorzugte Zusammensetzungen enthalten 0,01 - 5 Gew.-% 9-Octadecen-1,18-dicarbonsäure, 0,0001 bis 5 Gew.-% Iberin und 2-Phenylbenzimidazol-5-sulfonsäure. Weitere besonders bevorzugte Zusammensetzungen enthalten 0,01 - 5 Gew.-% 9-Octa-decen-1,18-dicarbonsäure, 0,0001 bis 5 Gew.-% Brassicin und 2-Phenylbenzimidazol-5 sulfonsäure. Weitere besonders bevorzugte Zusammensetzungen enthalten 0,01 - 5 Gew.-% 9-Octa-decen-1,18-dicarbonsäure, 0,0001 bis 5 Gew.-% Nasturtiin und 2-Phenylbenzimidazol-5-sulfonsäure.

Besonders bevorzugte Zusammensetzungen enthalten 0,01 - 5 Gew.-% 9-Octadecen-1,18-dicarbonsäure, 0,0001 - 5 Gew.-% Sulforaphan, Butyl Methoxydibenzoylmethane und Polysilicone-15.

Weitere besonders bevorzugte Zusammensetzungen enthalten 0,01 - 5 Gew.-% 9-Octadecen-1,18-dicarbonsäure, 0,0001 - 5 Gew.-% Sulforaphen, Butyl Methoxydibenzoylmethane und Polysilicone-15. Weitere besonders bevorzugte Zusammensetzungen enthalten 0,01 - 5 Gew.-% 9-Octa-decen-1,18-dicarbonsäure, 0,0001 bis 5 Gew.-% Allylisothiocyanat, Butyl Methoxydibenzoylmethane und Polysilicone-15. Weitere besonders bevorzugte erfindungsgemäße Zusammensetzungen enthalten 0,01 - 5 Gew.-% 9-Octadecen-1,18-dicarbonsäure, 0,0001 bis 5 Gew.-% Benzylisothiocyanat, Butyl Methoxydibenzoylmethane und Polysilicone-15. Weitere besonders bevorzugte Zusammensetzungen enthalten 0,01 - 5 Gew.-% 9-Octadecen-1,18-dicarbonsäure, 0,0001 - 5 Gew.-% Phenylethylisothiocyanat, Butyl Methoxydibenzoylmethane und Polysilicone-15.

Weitere besonders bevorzugte Zusammensetzungen enthalten 0,01 - 5 Gew.-% 9-Octadecen-1,18-dicarbonsäure, 0,0001 bis 5 Gew.-% Ethylisothiocyanat, Butyl Methoxydibenzoylmethane und

Polysilicone-15. Weitere besonders bevorzugte Zusammensetzungen enthalten 0,01 - 5 Gew.-% 9-Octadecen-1,18-dicarbonsäure, 0,0001 bis 5 Gew.-% Barbarin, Butyl Methoxydibenzoylmethane und Polysilicone-15. Weitere besonders bevorzugte Zusammensetzungen enthalten 0,01 - 5 Gew.-% 9-Octadecen-1,18-dicarbonsäure, 0,0001 bis 5 Gew.-% Goitrin, Butyl Methoxydibenzoylmethane und Polysilicone-15. Weitere besonders bevorzugte Zusammensetzungen enthalten 0,01 - 5 Gew.-% 9-Octadecen-1,18-dicarbonsäure, 0,0001 bis 5 Gew.-% Iberin, Butyl Methoxydibenzoylmethane und Polysilicone-15. Weitere besonders bevorzugte Zusammensetzungen enthalten 0,01 - 5 Gew.-% 9-Octadecen-1,18-dicarbonsäure, 0,0001 bis 5 Gew.-% Brassicin, Butyl Methoxydibenzoylmethane und Polysilicone-15. Weitere besonders bevorzugte Zusammensetzungen enthalten 0,01 - 5 Gew.-% 9-Octadecen-1,18-dicarbonsäure, 0,0001 bis 5 Gew.-% Nasturtiin, Butyl Methoxydibenzoylmethane und Polysilicone-15. Besonders bevorzugte Zusammensetzungen enthalten 0,01 - 5 Gew.-% 9-Octadecen-1,18-dicarbonsäure, 0,0001 bis 5 Gew.-% Sulforaphan, Butyl Methoxydibenzoylmethane, Polysilicone-15 und 2-Phenylbenzimidazol-5-sulfonsäure. Weitere besonders bevorzugte Zusammensetzungen enthalten 0,01 - 5 Gew.-% 9-Octadecen-1,18-dicarbonsäure, 0,0001 bis 5 Gew.-% Sulforaphen, Butyl Methoxydibenzoylmethane, Polysilicone-15 und 2-Phenylbenzimidazol-5-sulfonsäure. Weitere besonders bevorzugte Zusammensetzungen enthalten 0,01 - 5 Gew.-% 9-Octadecen-1,18-dicarbonsäure, 0,0001 bis 5 Gew.-% Allylisothiocyanat, Butyl Methoxydibenzoylmethane, Polysilicone-15 und 2-Phenylbenzimidazol-5-sulfonsäure. Weitere besonders bevorzugte Zusammensetzungen enthalten 0,01 - 5 Gew.-% 9-Octadecen-1,18-dicarbonsäure, 0,0001 bis 5 Gew.-% Benzylisothiocyanat, Butyl Methoxydibenzoylmethane, Polysilicone-15 und 2-Phenylbenzimidazol-5-sulfonsäure. Weitere besonders bevorzugte Zusammensetzungen enthalten 0,01 - 5 Gew.-% 9-Octadecen-1,18-dicarbonsäure, 0,0001 bis 5 Gew.-% Phenylethylisothiocyanat, Butyl Methoxydibenzoylmethane, Polysilicone-15 und 2-Phenylbenzimidazol-5-sulfonsäure. Weitere besonders bevorzugte Zusammensetzungen enthalten 0,01 - 5 Gew.-% 9-Octadecen-1,18-dicarbonsäure, 0,0001 bis 5 Gew.-% Ethylisothiocyanat, Butyl Methoxydibenzoylmethane, Polysilicone-15 und 2-Phenylbenzimidazol-5-sulfonsäure. Weitere besonders bevorzugte Zusammensetzungen enthalten 0,01 - 5 Gew.-% 9-Octadecen-1,18-dicarbonsäure, 0,0001 bis 5 Gew.-% Barbarin, Butyl Methoxydibenzoylmethane, Polysilicone-15 und 2-Phenylbenzimidazol-5-sulfonsäure. Weitere besonders bevorzugte erfindungsgemäße Zusammensetzungen enthalten 0,01 - 5 Gew.-% 9-Octadecen-1,18-dicarbonsäure, 0,0001 bis 5 Gew.-% Goitrin, Butyl Methoxydibenzoylmethane, Polysilicone-15 und 2-Phenylbenzimidazol-5-sulfonsäure. Weitere besonders bevorzugte erfindungsgemäße Zusammensetzungen enthalten 0,01 - 5 Gew.-% 9-Octadecen-1,18-dicarbonsäure, 0,0001 bis 5 Gew.-% Iberin, Butyl Methoxydibenzoylmethane, Polysilicone-15 und 2-Phenylbenzimidazol-5-sulfonsäure. Weitere besonders bevorzugte Zusammensetzungen enthalten 0,01 - 5 Gew.-% 9-Octadecen-1,18-dicarbonsäure, 0,0001 bis 5 Gew.-% Brassicin, Butyl Methoxydibenzoylmethane, Polysilicone-15 und 2-Phenylbenzimidazol-5-sulfonsäure.

Weitere besonders bevorzugte erfindungsgemäße Zusammensetzungen enthalten 0,01 - 5 Gew.-% 9-Octadecen-1,18-dicarbonsäure, 0,0001 bis 5 Gew.-% Nasturtiin, Butyl Methoxydibenzoylmethane, Polysilicone-15 und 2-Phenylbenzimidazol-5-sulfonsäure.

Besonders bevorzugte Zusammensetzungen enthalten 0,01 - 5 Gew.-% 7-Tetradecen-1,14-dicarbonsäure, 0,0001 bis 5 Gew.-% Sulforaphan und Butyl Methoxydibenzoylmethane.

Weitere besonders bevorzugte Zusammensetzungen enthalten 0,01 - 5 Gew.-% 7-Tetradecen-1,14-dicarbonsäure, 0,0001 bis 5 Gew.-% Sulforaphen und Butyl Methoxydibenzoylmethane.

Weitere besonders bevorzugte Zusammensetzungen enthalten 0,01 - 5 Gew.-% 7-Tetradecen-1,14-dicarbonsäure, 0,0001 bis 5 Gew.-% Allylisothiocyanat und Butyl Methoxydibenzoylmethane.

Weitere besonders bevorzugte Zusammensetzungen enthalten 0,01 - 5 Gew.-% 7-Tetradecen-1,14-dicarbonsäure, 0,0001 - 5 Gew.-% Benzylisothiocyanat und Butyl Methoxydibenzoylmethane.

Weitere besonders bevorzugte Zusammensetzungen enthalten 0,01 - 5 Gew.-% 7-Tetradecen-1,14-dicarbonsäure, 0,0001 - 5 Gew.-% Phenylethylisothiocyanat und Butyl Methoxydibenzoylmethane. Weitere besonders bevorzugte Zusammensetzungen enthalten 0,01 - 5 Gew.-% 7-Tetradecen-1,14-dicarbonsäure, 0,0001 bis 5 Gew.-% Ethylisothiocyanat und Butyl Methoxydibenzoylmethane. Weitere besonders bevorzugte Zusammensetzungen enthalten 0,01 - 5 Gew.-% 7-Tetradecen-1,14-dicarbonsäure, 0,0001 - 5 Gew.-% Barbarin und Butyl Methoxydibenzoylmethane. Weitere besonders bevorzugte Zusammensetzungen enthalten 0,01 - 5 Gew.-% 7-Tetradecen-1,14-dicarbonsäure, 0,0001 bis 5 Gew.-% Goitrin und Butyl Methoxydibenzoylmethane.

Weitere besonders bevorzugte Zusammensetzungen enthalten 0,01 - 5 Gew.-% 7-Tetradecen-1,14-dicarbonsäure, 0,0001 bis 5 Gew.-% Iberin und Butyl Methoxydibenzoylmethane.

Weitere besonders bevorzugte Zusammensetzungen enthalten 0,01 - 5 Gew.-% 7-Tetradecen-1,14-dicarbonsäure, 0,0001 bis 5 Gew.-% Brassicin und Butyl Methoxydibenzoylmethane.

Weitere besonders bevorzugte Zusammensetzungen enthalten 0,01 - 5 Gew.-% 7-Tetradecen-1,14-dicarbonsäure, 0,0001 bis 5 Gew.-% Nasturtiin und Butyl Methoxydibenzoylmethane.

Besonders bevorzugte erfindungsgemäße Zusammensetzungen enthalten 0,01 - 5 Gew.-% 7-Tetradecen-1,14-dicarbonsäure, 0,0001 bis 5 Gew.-% Sulforaphan und Polysilicone-15.

Weitere besonders bevorzugte erfindungsgemäße Zusammensetzungen enthalten 0,01 - 5 Gew.-% 7-Tetradecen-1,14-dicarbonsäure, 0,0001 bis 5 Gew.-% Sulforaphen und Polysilicone-15.

Weitere besonders bevorzugte erfindungsgemäße Zusammensetzungen enthalten 0,01 - 5 Gew.-% 7-Tetradecen-1,14-dicarbonsäure, 0,0001 bis 5 Gew.-% Allylisothiocyanat und Polysilicone-15.

Weitere besonders bevorzugte erfindungsgemäße Zusammensetzungen enthalten 0,01 - 5 Gew.-% 7-Tetradecen-1,14-dicarbonsäure, 0,0001 - 5 Gew.-% Benzylisothiocyanat und Polysilicone-15.

Weitere besonders bevorzugte Zusammensetzungen enthalten 0,01 - 5 Gew.-% 7-Tetradecen-1,14-dicarbonsäure, 0,0001 - 5 Gew.-% Phenylethylisothiocyanat und Polysilicone-15.

Weitere besonders bevorzugte erfindungsgemäße Zusammensetzungen enthalten 0,01 - 5 Gew.-% 7-Tetradecen-1,14-dicarbonsäure, 0,0001 bis 5 Gew.-% Ethylisothiocyanat und Polysilicone-15.

Weitere besonders bevorzugte erfindungsgemäße Zusammensetzungen enthalten 0,01 - 5 Gew.-% 7-Tetradecen-1,14-dicarbonsäure, 0,0001 bis 5 Gew.-% Barbarin und Polysilicone-15.

Weitere besonders bevorzugte erfindungsgemäße Zusammensetzungen enthalten 0,01 - 5 Gew.-% 7-Tetradecen-1,14-dicarbonsäure, 0,0001 bis 5 Gew.-% Goitrin und Polysilicone-15.

Weitere besonders bevorzugte erfindungsgemäße Zusammensetzungen enthalten 0,01 - 5 Gew.-% 7-Tetradecen-1,14-dicarbonsäure, 0,0001 bis 5 Gew.-% Iberin und Polysilicone-15.

Weitere besonders bevorzugte erfindungsgemäße Zusammensetzungen enthalten 0,01 - 5 Gew.-% 7-Tetradecen-1,14-dicarbonsäure, 0,0001 bis 5 Gew.-% Brassicin und Polysilicone-15.

Weitere besonders bevorzugte erfindungsgemäße Zusammensetzungen enthalten 0,01 - 5 Gew.-% 7-Tetradecen-1,14-dicarbonsäure, 0,0001 bis 5 Gew.-% Nasturtiin und Polysilicone-15.

Besonders bevorzugte Zusammensetzungen enthalten 0,01 - 5 Gew.-% 7-Tetradecen-1,14-dicarbonsäure, 0,0001 - 5 Gew.-% Sulforaphan und 2-Phenylbenzimidazol-5-sulfonsäure.

Weitere besonders bevorzugte Zusammensetzungen enthalten 0,01 - 5 Gew.-% 7-Tetradecen-1,14-dicarbonsäure, 0,0001 bis 5 Gew.-% Sulforaphen und 2-Phenylbenzimidazol-5-sulfonsäure.

Weitere besonders bevorzugte Zusammensetzungen enthalten 0,01 - 5 Gew.-% 7-Tetradecen-1,14-dicarbonsäure, 0,0001 - 5 Gew.-% Allylisothiocyanat und 2-Phenylbenzimidazol-5-sulfonsäure. Weitere besonders bevorzugte erfindungsgemäße Zusammensetzungen enthalten 0,01 - 5 Gew.-% 7-Tetradecen-1,14-dicarbonsäure, 0,0001 bis 5 Gew.-% Benzylisothiocyanat und 2-Phenylbenzimidazol-5-sulfonsäure. Weitere besonders bevorzugte Zusammensetzungen enthalten 0,01 - 5 Gew.-% 7-Tetradecen-1,14-dicarbonsäure, 0,0001 bis 5 Gew.-% Phenylethylisothiocyanat und 2-Phenylbenzimidazol-5-sulfonsäure. Weitere besonders bevorzugte Zusammensetzungen enthalten 0,01 - 5 Gew.-% 7-Tetradecen-1,14-dicarbonsäure, 0,0001 - 5 Gew.-% Ethylisothiocyanat und 2-Phenylbenzimidazol-5-sulfonsäure. Weitere besonders bevorzugte erfindungsgemäße Zusammensetzungen enthalten 0,01 - 5 Gew.-% 7-Tetradecen-1,14-dicarbonsäure, 0,0001 bis 5 Gew.-% Barbarin und 2-Phenylbenzimidazol-5-sulfonsäure. Weitere besonders bevorzugte Zusammensetzungen enthalten 0,01 - 5 Gew.-% 7-Tetradecen-1,14-dicarbonsäure, 0,0001 bis 5 Gew.-% Goitrin und 2-Phenylbenzimidazol-5-sulfonsäure. Weitere besonders bevorzugte Zusammensetzungen enthalten 0,01 - 5 Gew.-% 7-Tetradecen-1,14-dicarbonsäure, 0,0001 bis 5 Gew.-% Iberin und 2-Phenylbenzimidazol-5-sulfonsäure. Weitere besonders bevorzugte Zusammensetzungen enthalten 0,01 - 5 Gew.-% 7-Tetradecen-1,14-dicarbonsäure, 0,0001 bis 5 Gew.-% Brassicin und

2-Phenylbenzimidazol-5-sulfonsäure. Weitere besonders bevorzugte Zusammensetzungen enthalten 0,01 - 5 Gew.-% 7-Tetradecen-1,14-dicarbonsäure, 0,0001 bis 5 Gew.-% Nasturtiin und

2-Phenylbenzimidazol-5-sulfonsäure.

Besonders bevorzugte Zusammensetzungen enthalten 0,01 - 5 Gew.-% 7-Tetradecen-1,14-dicarbonsäure, 0,0001 bis 5 Gew.-% Sulforaphan, Butyl Methoxydibenzoylmethane und

Polysilicone-15. Weitere besonders bevorzugte Zusammensetzungen enthalten 0,01 - 5 Gew.-% 7-Tetradecen-1,14-dicarbonsäure, 0,0001 bis 5 Gew.-% Sulforaphen, Butyl Methoxydibenzoylmethane und Polysilicone-15. Weitere besonders bevorzugte erfindungsgemäße Zusammensetzungen enthalten 0,01 - 5 Gew.-% 7-Tetradecen-1,14-dicarbonsäure, 0,0001 bis 5 Gew.-% Allylisothiocyanat, Butyl Methoxydibenzoylmethane und Polysilicone-15. Weitere besonders bevorzugte erfindungsgemäße Zusammensetzungen enthalten 0,01 - 5 Gew.-% 7-Tetradecen-1,14-dicarbonsäure, 0,0001 bis 5 Gew.-% Benzylisothiocyanat, Butyl Methoxydibenzoylmethane und Polysilicone-15. Weitere besonders bevorzugte Zusammensetzungen enthalten 0,01 - 5 Gew.-% 7-Tetradecen-1,14-dicarbonsäure, 0,0001 bis 5 Gew.-% Phenylethylisothiocyanat, Butyl Methoxydibenzoylmethane und Polysilicone-15. Weitere besonders bevorzugte Zusammensetzungen enthalten 0,01 - 5 Gew.-% 7-Tetradecen-1,14-dicarbonsäure, 0,0001 bis 5 Gew.-% Ethylisothiocyanat, Butyl Methoxydibenzoylmethane und Polysilicone-15. Weitere besonders bevorzugte Zusammensetzungen enthalten 0,01 - 5 Gew.-% 7-Tetradecen-1,14-dicarbonsäure, 0,0001 - 5 Gew.-% Barbarin, Butyl Methoxydibenzoylmethane und Polysilicone-15. Weitere besonders bevorzugte Zusammensetzungen enthalten 0,01 - 5 Gew.-% 7-Tetradecen-1,14-dicarbonsäure, 0,0001 bis 5 Gew.-% Goitrin, Butyl Methoxydibenzoylmethane und Polysilicone-15. Weitere besonders bevorzugte Zusammensetzungen enthalten 0,01 - 5 Gew.-% 7-Tetradecen-1,14-dicarbonsäure, 0,0001 bis 5 Gew.-% Iberin, Butyl Methoxydibenzoylmethane und Polysilicone-15. Weitere besonders bevorzugte Zusammensetzungen enthalten 0,01 - 5 Gew.-% 7-Tetradecen-1,14-dicarbonsäure, 0,0001 bis 5 Gew.-% Brassicin, Butyl Methoxydibenzoylmethane und Polysilicone-15. Weitere besonders bevorzugte Zusammensetzungen enthalten 0,01 - 5 Gew.-% 7-Tetradecen-1,14-dicarbonsäure, 0,0001 bis 5 Gew.-% Nasturtiin, Butyl Methoxydibenzoylmethane und Polysilicone-15.

Besonders bevorzugte Zusammensetzungen enthalten 0,01 - 5 Gew.-% 7-Tetradecen-1,14-dicarbonsäure, 0,0001 bis 5 Gew.-% Sulforaphan, Butyl Methoxydibenzoylmethane, Polysilicone-15 und 2-Phenylbenzimidazol-5-sulfonsäure. Weitere besonders bevorzugte Zusammensetzungen enthalten 0,01 - 5 Gew.-% 7-Tetradecen-1,14-dicarbonsäure, 0,0001 bis 5 Gew.-% Sulforaphen, Butyl Methoxydibenzoylmethane, Polysilicone-15 und 2-Phenylbenzimidazol-5-sulfonsäure.

Weitere besonders bevorzugte Zusammensetzungen enthalten 0,01 - 5 Gew.-% 7-Tetradecen-1,14-dicarbonsäure, 0,0001 bis 5 Gew.-% Allylisothiocyanat, Butyl Methoxydibenzoylmethane, Polysilicone-15 und 2-Phenylbenzimidazol-5-sulfonsäure. Weitere besonders bevorzugte Zusammensetzungen enthalten 0,01 - 5 Gew.-% 7-Tetradecen-1,14-dicarbonsäure, 0,0001 bis 5 Gew.-% Benzylisothiocyanat, Butyl Methoxydibenzoylmethane, Polysilicone-15 und 2-Phenylbenzimidazol-5-sulfonsäure. Weitere besonders bevorzugte Zusammensetzungen enthalten 0,01 - 5 Gew.-% 7-Tetradecen-1,14-dicarbonsäure, 0,0001 bis 5 Gew.-% Phenylethylisothiocyanat, Butyl Methoxydibenzoylmethane, Polysilicone-15 und 2-Phenylbenzimidazol-5-sulfonsäure. Weitere besonders bevorzugte Zusammensetzungen enthalten 0,01 - 5 Gew.-% 7-Tetradecen-1,14-dicarbonsäure, 0,0001 bis 5 Gew.-% Ethylisothiocyanat, Butyl Methoxydibenzoylmethane, Polysilicone-15 und 2-Phenylbenzimidazol-5-sulfonsäure. Weitere besonders bevorzugte Zusammensetzungen enthalten 0,01 - 5 Gew.-% 7-Tetradecen-1,14-dicarbonsäure, 0,0001 bis 5 Gew.-% Barbarin, Butyl Methoxydibenzoylmethane, Polysilicone-15 und 2-Phenylbenzimidazol-5-sulfonsäure. Weitere besonders bevorzugte erfindungsgemäße Zusammensetzungen enthalten 0,01 - 5 Gew.-% 7-Tetradecen-1,14-dicarbonsäure, 0,0001 bis 5 Gew.-% Goitrin, Butyl Methoxydibenzoylmethane, Polysilicone-15 und 2-Phenylbenzimidazol-5-sulfonsäure.

Weitere besonders bevorzugte Zusammensetzungen enthalten 0,01 - 5 Gew.-% 7-Tetradecen-1,14-dicarbonsäure, 0,0001 bis 5 Gew.-% Iberin, Butyl Methoxydibenzoylmethane, Polysilicone-15 und 2-Phenylbenzimidazol-5-sulfonsäure. Weitere besonders bevorzugte Zusammensetzungen enthalten 0,01 - 5 Gew.-% 7-Tetradecen-1,14-dicarbonsäure, 0,0001 bis 5 Gew.-% Brassicin, Butyl Methoxydibenzoylmethane, Polysilicone-15 und 2-Phenylbenzimidazol-5-sulfonsäure.

Weitere besonders bevorzugte Zusammensetzungen enthalten 0,01 - 5 Gew.-% 7-Tetradecen-1,14-dicarbonsäure, 0,0001 bis 5 Gew.-% Nasturtiin, Butyl Methoxydibenzoylmethane, Polysilicone-15 und 2-Phenylbenzimidazol-5-sulfonsäure. Besonders bevorzugte Zusammensetzungen enthalten 0,01 - 5 Gew.-% 8-Hexadecen-1,16-dicarbonsäure, 0,0001 - 5 Gew.-% Sulforaphan und Ethylhexyl Triazone. Weitere besonders bevorzugte Zusammensetzungen enthalten 0,01 - 5 Gew.-% 8-Hexadecen-1,16-dicarbonsäure, 0,0001 bis 5 Gew.-% Sulforaphen und Ethylhexyl Triazone.

Weitere besonders bevorzugte Zusammensetzungen enthalten 0,01 - 5 Gew.-% 8-Hexadecen-1,16-dicarbonsäure, 0,0001 bis 5 Gew.-% Allylisothiocyanat und Ethylhexyl Triazone.

Weitere besonders bevorzugte Zusammensetzungen enthalten 0,01 - 5 Gew.-% 8-Hexadecen-1,16-dicarbonsäure, 0,0001 bis 5 Gew.-% Benzylisothiocyanat und Ethylhexyl Triazone.

Weitere besonders bevorzugte Zusammensetzungen enthalten 0,01 - 5 Gew.-% 8-Hexadecen-1,16-dicarbonsäure, 0,0001 bis 5 Gew.-% Phenylethylisothiocyanat und Ethylhexyl Triazone.

Weitere besonders bevorzugte Zusammensetzungen enthalten 0,01 - 5 Gew.-% 8-Hexadecen-1,16-dicarbonsäure, 0,0001 bis 5 Gew.-% Ethylisothiocyanat und Ethylhexyl Triazone.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Barbarin und Ethylhexyl Triazone.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Goitrin und Ethylhexyl Triazone.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Iberin und Ethylhexyl Triazone.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Brassicin und Ethylhexyl Triazone.

Weitere besonders bevorzugte erfindungsgemäße Zusammensetzungen enthalten 0,01 - 5 Gew.-% 8-Hexadecen-1,16-dicarbonsäure, 0,0001 bis 5 Gew.-% Nasturtiin und Ethylhexyl Triazone.

Neben den erfindungsgemäß obligatorischen Dicarbonsäuren und den bevorzugten zusätzlichen Säuren (z. B. Ascorbinsäure, wasserlösliche UV-Filter in Säureform) selbst können auch deren physiologisch verträgliche Salze erfindungsgemäß eingesetzt werden. Beispiele für solche Salze sind die Alkali-, Erdalkali-, Zinksalze sowie Ammoniumsalze, worunter im Rahmen der vorliegenden Anmeldung auch die Mono-, Di- und Trimethyl-, -ethyl- und -hydroxyethyl-Ammoniumsalze zu verstehen sind. Ganz besonders bevorzugt können im Rahmen der Erfindung jedoch mit alkalisch reagierenden Aminosäuren, wie beispielsweise Arginin, Lysin, Ornithin und Histidin, neutralisierte Säuren eingesetzt werden.

Die erfindungsgemäßen Zusammensetzungen können in allen für die topische Applikation auf die Haut geeigneten Darreichungsformen konfektioniert sein. Bevorzugte Darreichungsformen sind eine Lösung, eine Emulsion vom Typ Wasser-in-Öl (W/O), eine Emulsion vom Typ Öl-in-Wasser (O/W), eine multiple Emulsion, beispielsweise vom Typ Wasser-in-Öl-in-Wasser (W/O/W) oder Öl-in-Wasser-in-Öl (O/W/O), eine Hydrodispersion, eine Lipodispersion, ein Gel, ein Hydrodispersionsgel, ein Lipodispersionsgel, ein fester Stift, ein Gelpflaster (Gelpatch), ein Pflaster, ein Kataplasma, Wirkstoff-haltige Liposomen oder ein transdermales therapeutisches System. Niedrigviskose oder mittelviskose Darreichungsformen können auch in einem treibgasfreien Pump- oder Sprühspender oder zusammen mit einem Treibmittel in einem Aerosolbehälter konfektioniert sein. Weiterhin ist bevorzugt, die erfindungsgemäße Wirkstoffkombination in einem Mehrkammerspender, bevorzugt in einem Zweikammerspender, zu verpacken. Dabei ist in einer ersten bevorzugten Ausführungsform die erfindungsgemäße Wirkstoffkombination in einer Kammer enthalten. In einer weiteren bevorzugten Ausführungsform liegen die beiden Bestandteile a) und b) der erfindungsgemäßen Wirkstoffkombination in getrennten Kammern eines Mehrkammerspenders, bevorzugt eines Zweikammerspenders, vor. In einer weiteren bevorzugten Ausführungsform liegen die beiden Bestandteile a) und b) der erfindungsgemäßen Wirkstoffkombination in getrennten Streifen eines Pastenstrangs beziehungsweise analog hierzu in getrennten Phasen einer mehrphasigen Zusammensetzung mit fester oder hochviskoser Konsistenz vor, wie es beispielsweise von Deodorantstiften bekannt ist. In einer weiteren bevorzugten Ausführungsform liegt mindestens einer beiden Bestandteile a) und b) der erfindungsgemäßen Wirkstoffkombination in verkapselter Form vor. Die Verkapselung kann beispielsweise in Form von Gelatinekapseln, Polyamidkapseln, Polyvinylalkoholkapseln oder Kapseln aus anderen geeigneten Polymermaterialien erfolgen. Bevorzugt erfolgt die Verkapselung in Form von Liposomen, besonders bevorzugt in Form von aus Phospholipiden, wie bevorzugt Lecithin, gehärtetem Lecithin oder Lecithinfraktionen, wie Phosphatidylcholin, und/oder aus Sphingolipiden, wie Ceramiden, gebildeten Liposomen. Die Liposomen können überwiegend als unilamellare Vesikel, paucilamellare Vesikel oder multilamellare Vesikel vorliegen.

Erfindungsgemäße Emulsionen im Sinne der vorliegenden Erfindung, z. B. in Form einer Creme, einer Lotion oder einer kosmetischen Milch, sind vorteilhaft und enthalten z. B. Fette, Öle, Wachse und/oder andere Fettkörper, sowie Wasser und einen oder mehrere Emulgatoren, wie sie üblicherweise für einen solchen Typ der Formulierung verwendet werden.

Es ist auch möglich und vorteilhaft im Sinne der vorliegenden Erfindung, die erfindungsgemäßen Zusammensetzungen als wässrige Systeme bzw. Tensidzusammensetzungen zur Reinigung und

Pflege der Haut bereitzustellen. Dies umfasst bevorzugt Duschgels, Kopfhauttonics, Sprays etc.

Als besonders vorteilhaft hat sich der Einsatz von Tensiden (E) in den erfindungsgemäßen Mitteln erwiesen. In einer weiteren bevorzugten Ausführungsform enthalten die erfindungsgemäßen Mittel daher Tenside. Unter dem Begriff Tenside werden grenzflächenaktive Substanzen, die an Ober-und Grenzflächen Adsorptionsschichten bilden oder in Volumenphasen zu Mizellkolloiden oder lyotropen Mesophasen aggregieren können, verstanden. Man unterscheidet Aniontenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wässriger Lösung stark hydratisiert sind.

Als anionische Tenside (E1) eignen sich in erfindungsgemäßen Zusammensetzungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 C-Atomen. Zusätzlich können im Molekül Glykol- oder Polyglykolether-Gruppen, Ester-, Ether-und Amidgruppen sowie Hydroxylgruppen enthalten sein. Beispiele für geeignete anionische Tenside sind, jeweils in Form der Natrium-, Kalium- und Ammonium- sowie der Mono-, Di- und

Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- sulfatierte Hydroxyalkylpolyethylen- und/oder Hydroxyalkylenpropylenglykolether
- Sulfonate ungesättigter Fettsäuren mit 8 bis 24 C-Atomen und 1 bis 6 Doppelbindungen
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (E1-I), R¹(OCH₂CH₂)ₙ-O-P(O)(OX)-OR² (E1-I) in der R¹ bevorzugt für einen aliphatischen Kohlenwasserstoffrest mit 8 bis 30 Kohlenstoffatomen, R² für Wasserstoff, einen Rest (CH₂CH₂O)ₙR² oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C₁ bis C₄ - Kohlenwasserstoffrest, steht,
- sulfatierte Fettsäurealkylenglykolester der Formel (E1-II) R⁷CO(AlkO)ₙSO₃M (E1-II) in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht,
- Monoglyceridsulfate und Monoglyceridethersulfate der Formel (E1-III)

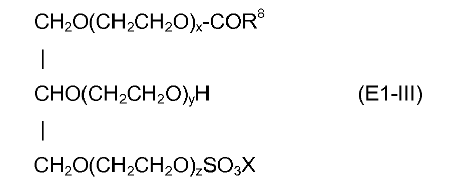

in der R⁸CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali-oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder

Chlorsulfonsäure in Form ihrer Natriumsalze. Vorzugsweise werden Monoglyceridsulfate der Formel (E1-III) eingesetzt, in der R⁸CO für einen linearen Acylrest mit 8 bis 18 Kohlenstoffatomen steht,
- Amidethercarbonsäuren,
- Kondensationsprodukte aus C₈ - C₃₀ - Fettalkoholen mit Proteinhydrolysaten und/oder Aminosäuren und deren Derivaten,welche dem Fachmann als Eiweissfettsäurekondensate bekannt sind, wie beispielsweise die Lamepon^{®} - Typen, Gluadin^{®} - Typen, Hostapon^{®} KCG oder die Amisoft^{®} - Typen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen, Monoglycerdisulfate, Alkyl- und Alkenyletherphosphate sowie Eiweißfettsäurekondensate.

Als zwitterionische Tenside (E2) werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden (E3) werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Besonders bevorzugte ampholytische Tenside sind das N-Kokosalkylaminopropionat, das Kokosacylaminoethylaminopropionat und das C₁₂ - C₁₈ - Acylsarcosin.

Nichtionische Tenside (E4) enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol - Typen (Cognis),
- alkoxylierte Triglyceride,
- alkoxylierte Fettsäurealkylester der Formel (E4-I) R¹CO-(OCH₂CHR²)_{w}OR³ (E4-I) in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II), R⁴O-[G]ₚ (E4-II) in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Die Alkyl- und Alkenyloligoglykoside können sich von Aldosen bzw. Ketosen mit 5 oder 6 Kohlenstoffatomen, vorzugsweise von Glucose, ableiten. Die bevorzugten Alkyl- und/oder Alkenyloligoglykoside sind somit Alkyl- und/oder Alkenyloligoglucoside. Die Indexzahl p in der allgemeinen Formel (E4-II) gibt den Oligomerisierungsgrad (DP), d. h. die Verteilung von Mono- und Oligoglykosiden an und steht für eine Zahl zwischen 1 und 10. Während p im einzelnen Molekül stets ganzzahlig sein muss und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R⁴ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹⁵ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.
- Zuckertenside vom Typ der Fettsäure-N-alkylpolyhydroxyalkylamide, ein nichtionisches Tensid der Formel (E4-III), R⁵CO-NR⁶-[Z] (E4-III) in der R⁵CO für einen aliphatischen Acylrest mit 6 bis 22 Kohlenstoffatomen, R⁶ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht. Bei den Fettsäure-N-alkylpolyhydroxyalkylamiden handelt es sich um bekannte Stoffe, die üblicherweise durch reduktive Aminierung eines reduzierenden Zuckers mit

Ammoniak, einem Alkylamin oder einem Alkanolamin und nachfolgende Acylierung mit einer

Fettsäure, einem Fettsäurealkylester oder einem Fettsäurechlorid erhalten werden. Vorzugsweise leiten sich die Fettsäure-N-alkylpolyhydroxyalkylamide von reduzierenden Zuckern mit 5 oder 6 Kohlenstoffatomen, insbesondere von der Glucose ab. Die bevorzugten Fettsäure-N-alkylpolyhydroxyalkylamide stellen daher Fettsäure-N-alkylglucamide dar, wie sie durch die Formel (E4-IV) wiedergegeben werden:

R⁷CO-NR⁸-CH₂-(CHOH)₄-CH₂OH (E4-IV)

Vorzugsweise werden als Fettsäure-N-alkylpolyhydroxyalkylamide Glucamide der Formel (E4-IV) eingesetzt, in der R⁸ für Wasserstoff oder eine Alkylgruppe steht und R⁷CO für den Acylrest der Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Arachinsäure, Gadoleinsäure, Behensäure oder Erucasäure bzw. derer technischer Mischungen steht. Besonders bevorzugt sind Fettsäure-N-alkylglucamide der Formel (E4-IV), die durch reduktive Aminierung von Glucose mit Methylamin und anschließende Acylierung mit Laurinsäure oder C12/14-Kokosfettsäure bzw. einem entsprechenden Derivat erhalten werden. Weiterhin können sich die Polyhydroxyalkylamide auch von Maltose und Palatinose ableiten.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zusammensetzungen mit hervorragenden Eigenschaften werden auch erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Weiterhin sind ganz besonders bevorzugte nichtionische Tenside die Zuckertenside. Diese können in den erfindungsgemäß verwendeten Mitteln bevorzugt in Mengen von 0,1 - 20 Gew.-%, bezogen auf das gesamte Mittel, enthalten sein. Mengen von 0,5 - 15 Gew.-% sind bevorzugt, und ganz besonders bevorzugt sind Mengen von 0,5 - 7,5 Gew.-%.

Erfindungsgemäß einsetzbar sind kationische Tenside vom Typ der quarternären Ammoniumverbindungen, der Esterquats und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf.

Bevorzugt einsetzbar sind erfindungsgemäß QAV mit Behenylresten, insbesondere die unter der Bezeichnung Behentrimoniumchlorid bzw. -bromid (Docosanyltrimethylammonium Chlorid bzw. -Bromid) bekannten Substanzen. Andere bevorzugte QAV weisen mindestens zwei Behenylreste auf, wobei QAV, welche zwei Behenylreste an einem Imidazoliniumrückgrat besonders bevorzugt sind. Kommerziell erhältlich sind diese Substanzen beispielsweise unter den Bezeichnungen Genamin^{®} KDMP (Clariant) und Crodazosoft^{®} DBQ (Crodauza).

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Die kationischen Tenside sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Die Tenside (E) werden vorzugsweise in Mengen von 0,1 - 45 Gew.%, bevorzugt 0,5 - 30 Gew.% und ganz besonders bevorzugt von 0,5 - 25 Gew.%, bezogen auf das gesamte erfindungsgemäße Mittel, eingesetzt.

Anionische, nichtionische, zwitterionische und/oder amphotere Tenside sowie deren Mischungen können erfindungsgemäß bevorzugt sein.

Zusammenfassend sind erfindungsgemäße kosmetische oder dermatologische Zusammensetzungen bevorzugt, die - bezogen auf ihr Gewicht - 0,5 bis 70 Gew.-%, vorzugsweise 1 bis 60 Gew.-% und insbesondere 5 bis 25 Gew.-% anionische(s) und/oder nichtionische(s) und/oder kationische(s) und/oder amphotere(s) Tensid(e), enthalten.

In einer weiteren bevorzugten Ausführungsform können die erfindungsgemäßen Mittel Emulgatoren (F) enthalten. Emulgatoren bewirken an der Phasengrenzfläche die Ausbildung von wasser- bzw. ölstabilen Adsorptionsschichten, welche die dispergierten Tröpfchen gegen Koaleszenz schützen und damit die Emulsion stabilisieren. Emulgatoren sind daher wie Tenside aus einem hydrophoben und einem hydrophilen Molekülteil aufgebaut. Hydrophile Emulgatoren bilden bevorzugt O/W - Emulsionen und hydrophobe Emulgatoren bilden bevorzugt W/O - Emulsionen. Unter einer Emulsion ist eine tröpfchenförmige Verteilung (Dispersion) einer Flüssigkeit in einer anderen Flüssigkeit unter Aufwand von Energie zur Schaffung von stabilisierenden Phasengrenzflächen mittels Tensiden zu verstehen. Die Auswahl dieser emulgierenden Tenside oder Emulgatoren richtet sich dabei nach den zu dispergierenden Stoffen und der jeweiligen äußeren Phase sowie der Feinteiligkeit der Emulsion. Erfindungsgemäß verwendbare Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phosphatidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- Lineare und verzweigte Fettsäuren mit 8 bis 30 C - Atomen und deren Na-, K-, Ammonium-, Ca-, Mg- und Zn - Salze.

Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel. Bevorzugt können die erfindungsgemäßen Zusammensetzungen mindestens einen nichtionogenen Emulgator mit einem HLB-Wert von 8 bis 18 enthalten. Nichtionogene Emulgatoren mit einem HLB-Wert von 10 - 15 können erfindungsgemäß besonders bevorzugt sein.

Als weiterhin vorteilhaft hat es sich gezeigt, wenn Polymere (G) in den erfindungsgemäßen Mitteln enthalten sind. In einer bevorzugten Ausführungsform werden den erfindungsgemäßen Mitteln daher Polymere zugesetzt, wobei sich sowohl kationische, anionische, amphotere als auch nichtionische Polymere als wirksam erwiesen haben.

Erfindungsgemäß einsetzbar sind vorzugsweise kationische bzw. amphotere Polymere. Unter kationischen bzw. amphoteren Polymeren sind Polymere zu verstehen, welche in der Haupt- und/ oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C1-4-Kohlenwasserstoffgruppe an eine aus Acrylsäure, Methacrylsäure oder deren Derivaten aufgebaute Polymerhauptkette gebunden sind, haben sich als besonders geeignet erwiesen.

Homopolymere der allgemeinen Formel (G1-I), 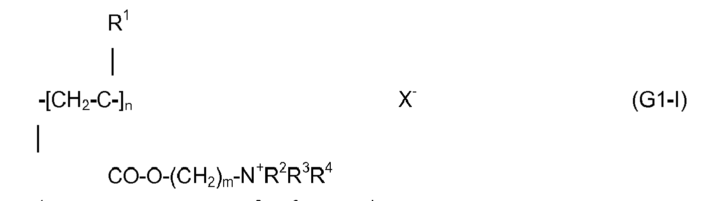 in der R¹= -H oder -CH₃ ist, R², R³ und R⁴ unabhängig voneinander ausgewählt sind aus C1-4-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (G1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt: R¹ steht für eine Methylgruppe, R², R³ und R⁴ stehen für Methylgruppen, m hat den Wert 2. Als physiologisch verträgliches Gegenionen X⁻ kommen beispielsweise Halogenidionen, Sulfationen, Phosphationen, Methosulfationen sowie organische Ionen wie Lactat-, Citrat- , Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Solche Produkte sind beispielsweise unter den Bezeichnungen Rheocare^{®} CTH (Cosmetic Rheologies) und Synthalen^{®} CR (Ethnichem) im Handel erhältlich. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwässrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (G1-I) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwässrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß der DE-PS 44 13 686,
- kationisierter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette.
   Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere in den erfindungsgemäßen Mitteln einsetzbare kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel verfügbar sind.

Erfindungsgemäß bevorzugte kationische Polymere sind kationische Cellulose-Derivate und Chitosan und dessen Derivate, insbesondere die Handelsprodukte Polymer^{®}JR 400, Hydagen^{®} HCMF und Kytamer^{®} PC, kationische Guar-Derivate, kationische Honig-Derivate, insbesondere das Handelsprodukt Honeyquat^{®} 50, kationische Alkylpolyglycodside gemäß der DE-PS 44 13 686 und

Polymere vom Typ Polyquaternium-37.

Weiterhin sind kationiserte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann.

Ganz besonders bevorzugt sind die kationischen Proteinhydrolysate und -derivate auf pflanzlicher Basis.

Zusätzlich zu kationischen Polymerisaten oder an ihrer Stelle können die erfindungsgemäßen Mittel auch amphotere Polymere enthalten. Diese weisen zusätzlich mindestens eine negativ geladene Gruppe im Molekül auf und werden auch als zwitterionische Polymere bezeichnet. Im Rahmen der vorliegenden Erfindung bevorzugt einsetzbare zwitterionische Polymerisate setzen sich im wesentlichen zusammen aus
A) Monomeren mit quartären Ammoniumgruppen der allgemeinen Formel (Z-I),

   R¹-CH=CR²-CO-Z-(CₙH₂ₙ)-N(⁺)R³R⁴R⁵ A⁽⁻⁾ (Z-I)

   In der R¹ und R² unabhängig voneinander stehen für Wasserstoff oder eine Methylgruppe und R³, R⁴ und R⁵ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist und
B) monomeren Carbonsäuren der allgemeinen Formel (Z-II),

   R⁶-CH=CR⁷-COOH (II)

in denen R⁶ und R⁷ unabhängig voneinander Wasserstoff oder Methylgruppen sind.

Geeignete Ausgangsmonomere sind z. B. Dimethylaminoethylacrylamid, Dimethylaminoethylmethacrylamid, Dimethylaminopropylacrylamid, Dimethylaminopropylmethacrylamid und Diethylaminoethylacrylamid, wenn Z eine NH-Gruppe bedeutet oder Dimethylaminoethylacrylat, Dimethylaminoethylmethacrylat und Diethylaminoethylacrylat, wenn Z ein Sauerstoffatom ist. Die eine tertiäre Aminogruppe enthaltenden Monomeren werden dann in bekannter Weise quaterniert, wobei als Alkylierungsreagenzien Methylchlorid, Dimethylsulfat oder Diethylsulfat besonders geeignet sind. Die Quaternisierungsreaktion kann in wässriger Lösung oder im Lösungsmittel erfolgen.

Vorteilhafterweise werden solche Monomere der Formel (Z-I) verwendet, die Derivate des Acrylamids oder Methacrylamids darstellen. Weiterhin bevorzugt sind solche Monomeren, die als Gegenionen Halogenid-, Methoxysulfat- oder Ethoxysulfat-Ionen enthalten. Ebenfalls bevorzugt sind solche Monomeren der Formel (Z-I), bei denen R³, R⁴ und R⁵ Methylgruppen sind. Das Acrylamidopropyl-trimethylammoniumchlorid ist ein ganz besonders bevorzugtes Monomer der Formel (Z-I). Als monomere Carbonsäuren der Formel (Z-II) eignen sich Acrylsäure, Methacrylsäure, Crotonsäure und 2-Methyl-crotonsäure. Bevorzugt werden Acryl- oder Methacrylsäure, insbesondere Acrylsäure, eingesetzt.

Die kationischen bzw. amphoteren Polymere sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Bei den anionischen Polymeren (G2) handelt es sich um anionische Polymere, welche Carboxylat-und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure. Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann. Besonders bevorzugt sind Homopolymere der 2-Acrylamido-2-methylpropansulfonsäure oder ihrer Salze, wie sie z. B. als Simulgel^{®} 800 oder Viscolam AT 100 P erhältlich sind.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Dimethylacrylamid Methacrylamid, Acrylsäureester, Methacrylsäureester, Vinylpyrrolidon, Vinylether und Vinylester.

Bevorzugte erfindungsgemäße Zusammensetzungen enthalten mindestens ein anionisches verdickendes Polymer in Form eines inversen, auto-inversiblen Polymerlatex, enthaltend eine Ölphase, eine Wasserphase, mindestens einen Öl-in-Wasser-Emulgator und mindestens einen verzweigten oder vernetzten Polyelektrolyten, der ein Copolymer darstellt, das mindestens ein Monomer mit einer starken Säurefunktion sowie mindestens ein weiteres Monomer aufweist, das neutral ist oder eine schwache Säurefunktion enthält.

Bevorzugte Polyelektrolyten sind aus zwei, drei, vier, fünf oder sechs verschiedenen Monomeren aufgebaut, von denen mindestens ein Monomer eine starke Säurefunktion aufweist und das mindestens eine weitere Monomer neutral ist oder eine schwache Säurefunktion aufweist.

Bevorzugt sind die Monomerkombinationen
starke Säurefunktion/neutral,
starke Säurefunktion/schwache Säurefunktion,
starke Säurefunktion/schwache Säurefunktion/neutral
starke Säurefunktion/schwache Säurefunktion 1/schwache Säurefunktion 2/neutral,
starke Säurefunktion/schwache Säurefunktion/neutral 1/neutral 2,
starke Säurefunktion/schwache Säurefunktion 1/schwache Säurefunktion 2/neutral 1/neutral 2.

Die oben angegebene Reihenfolge der Auflistung der Monomerbausteine gibt dabei nicht notwendigerweise die tatsächliche Reihenfolge der Monomerbausteine im Polyelektrolytmolekül wieder.

In einer bevorzugten Ausführungsform der Erfindung sind die Polyelektrolytmonomeren, die eine schwache Säurefunktion enthalten, ausgewählt aus den Monomeren, die eine Carboxylgruppe -COOH enthalten, die teilweise oder vollständig neutralisiert ist.

Aus der partiellen Neutralisation der schwachen Säurefunktion resultieren der neutralisierte Monomerbaustein und der nicht-neutralisierte, saure Monomerbaustein, beispielsweise Acrylsäure und

Natriumacrylat. Beide Monomere werden im Sinne der vorliegenden Anmeldung als gleich angesehen. Dementsprechend werden beide Monomere im Sinne der vorliegenden Anmeldung als "Monomer mit schwacher Säurefunktion" angesehen.

Besonders bevorzugte Beispiele für solche schwach sauren Polyelektrolytmonomeren sind Acrylsäure, Methacrylsäure, Itaconsäure und Maleinsäure. Bevorzugte erfindungsgemäße Zusammensetzungen sind daher **dadurch gekennzeichnet, dass** die schwache Säurefunktion des Polyelektrolytmonomeren eine Carboxylgruppe -COOH darstellt, die teilweise oder vollständig neutralisiert ist, wobei weiter bevorzugte erfindungsgemäße Zusammensetzungen dadurch gekennzeichnet sind, dass die Monomere des Polyelektrolyten, die mit der schwach sauren -COOH-Gruppe funktionalisiert sind, ausgewählt sind aus Acrylsäure, Methacrylsäure, Itaconsäure und Maleinsäure, die teilweise oder vollständig neutralisiert sind.

In einer weiteren bevorzugten Ausführungsform der vorliegenden Erfindung sind die Polyelektrolytmonomere mit einer starken Säuregruppe ausgewählt aus Monomeren, die mit einer Sulfonsäuregruppe -SO₃H oder einer Phosphonsäuregruppe funktionalisiert sind. Ein besonders bevorzugtes Polyelektrolytmonomer mit starker Säurefunktion ist 2-Methyl-2[(1-oxo-2-propenyl)amino]-1-pro-pansulfonsäure, die teilweise oder vollständig neutralisiert ist.

Aus der partiellen Neutralisation der starken Säurefunktion resultieren der neutralisierte Monomerbaustein und der nicht-neutralisierte, saure Monomerbaustein, beispielsweise 2-Methyl-2[(1-oxo-2-propenyl)amino]-1-propansulfonsäure und Natrium-2-methyl-2[(1-oxo-2-propenyl)amino]-1-pro-pansulfonat. Beide Monomere werden im Sinne der vorliegenden Anmeldung als gleich angesehen. Dementsprechend werden beide Monomere im Sinne der vorliegenden Anmeldung als "Monomer mit starker Säurefunktion" angesehen.

Bevorzugte erfindungsgemäße Zusammensetzungen sind daher **dadurch gekennzeichnet, dass** die starke Säurefunktion des Polyelektrolytmonomeren ausgewählt ist aus einer Sulfonsäuregruppe -SO₃H und einer Phosphonsäuregruppe, die teilweise oder vollständig neutralisiert sind, wobei weiter bevorzugte erfindungsgemäße Zusammensetzungen dadurch gekennzeichnet sind, dass das Polyelektrolytmonomer mit starker Säurefunktion ausgewählt ist aus 2-Methyl-2[(1-oxo-2-propenyl)amino]-1-propansulfonsäure, die teilweise oder vollständig neutralisiert ist.

Im Sinne der vorliegenden Erfindung sind alle Polyelektrolytmonomere, die mit einer schwachen oder starken Säuregruppe funktionalisiert sind, teilweise oder vollständig neutralisiert als Natrium-, Kalium-, Ammonium-, Ethanolamin- oder Aminosäure-Salz. Bevorzugte erfindungsgemäße Zusammensetzungen sind daher **dadurch gekennzeichnet, dass** die Polyelektrolytmonomere, die mit einer schwachen oder starken Säuregruppe funktionalisiert sind, teilweise oder vollständig neutralisiert sind als Natrium-, Kalium-, Ammonium-, Ethanolamin- oder Aminosäure-Salz.

In einer weiteren bevorzugten Ausführungsform der vorliegenden Erfindung ist das neutrale Polyelektrolytmonomer ausgewählt aus 2-Hydroxyethylacrylat, 2,3-Dihydroxypropylacrylat, 2-Hydroxyethylmethacrylat, 2,3-Dihydroxypropylmethacrylat, den ethoxylierten Derivaten der vorstehend genannten Ester mit einem Molekulargewicht zwischen 400 und 1000 g/mol, Acrylamid, Alkylacrylamiden, wie beispielsweise Methylacrylamid, Ethylacrylamid, n-Propylacrylamid und Isopropylacrylamid, Dialkylamiden, wie beispielsweise Dimethylacrylamid, Diethylacrylamid, Di-n-propylacrylamid und Diisopropylacrylamid, sowie Polyvinylpyrrolidon. In bevorzugten erfindungsgemäßen Zusammensetzungen ist das neutrale Polyelektrolytmonomer ausgewählt aus 2-Hydroxyethylacrylat, 2,3-Dihydroxypropylacrylat, 2-Hydroxyethylmethacrylat, 2,3-Dihydroxypropylmethacrylat, den ethoxylierten Derivaten der genannten Ester mit einem Molekulargewicht zwischen 400 und 1000 g/mol, Acrylamid, Dimethylacrylamid, Diethylacrylamid, Di-n-propylacrylamid, Diisopropylacrylamid sowie Polyvinylpyrrolidon. Besonders bevorzugte neutrale Polyelektrolytmonomere sind ausgewählt aus 2-Hydroxyethylacrylat, Acrylamid, Dimethylacrylamid und Polyvinylpyrrolidon. Vernetzte Copolymere aus 2-Methyl-2[(1-oxo-2-propenyl)amino]-1-propansulfonsäure (AMPS) und Hydroxyethylacrylat, in Form eines inversen, auto-inversiblen Latex sind kommerziell erhältlich, z. B. unter den Namen Simulgel^{®}NS, Simulgel^{®}I-NS 100, Simulgel^{®}FL und Sepiplus^{®} S von der Firma Seppic.

Weiterhin besonders bevorzugt sind teilweise oder vollständig neutralisierte, vernetzte Copolymere aus 2-Methyl-2[(1-oxo-2-propenyl)amino]-1-propansulfonsäure (AMPS) und Acrylamid, in Form eines inversen, auto-inversiblen Latex kommerziell erhältlich, z. B. unter dem Handelsnamen Simulgel^{®}600 von der Firma Seppic. Weiterhin besonders bevorzugt sind teilweise oder vollständig neutralisierte, vernetzte Copolymere aus 2-Methyl-2[(1-oxo-2-propenyl)amino]-1-propansulfon-säure (AMPS) und Acrylsäure, in Form eines inversen, auto-inversiblen Latex erhältlich, z. B. unter den Namen Simulgel^{®}EG und Simulgel^{®}EPG von Seppic. Weiterhin besonders bevorzugt sind teilweise oder vollständig neutralisierte, vernetzte Copolymere aus 2-Methyl-2[(1-oxo-2-propenyl)amino]-1-propansulfonsäure (AMPS), Acrylsäure bzw. Natriumacrylat, und (als drittem Monomerbaustein) Dimethylacrylamid, in Form eines inversen, autoinversiblen Latex erhältlich, z. B. unter dem Namen Simulgel^{®}SMS 88 von Seppic.

Allen vorgenannten, bevorzugt verwendeten inversen Polymerlatices ist gemein, dass sie mindestens ein Öl, bevorzugt ausgewählt aus weißen Mineralölen, Squalan, Isohexadecan und (gegebenenfalls hydriertem) Polyisobuten, sowie mindestens einen Öl-in-Wasser-Emulgator, ausgewählt aus den Ethylenoxid-Addukten von Sorbitanoleat, Ricinusöl, das gewünschtenfalls gehärtet ist, Sorbitanlaurat und Laurylalkohol, sowie weiterhin ausgewählt aus der Polyethylenoxid-freien Öl-in-Wasser-Emulgatorklasse der C6-C22-Fettalkohol-Glucoseether, insbesondere Caprinic Glucoside, Caprylic Glucoside, Capric Glucoside, Lauric Glucoside, Myristic Glucoside, Cetyl Glucoside, Stearyl Glucoside, Arachidyl Glucoside, Behenyl Glucoside, besonders bevorzugt Caprylic Glucoside und Capric Glucoside, enthalten. Demnach sind bevorzugte erfindungsgemäße Zusammensetzungen **dadurch gekennzeichnet, dass** die Ölphase des inversen Polymerlatex mindestens ein Öl, ausgewählt aus weißen Mineralölen, Squalan, Isohexadecan und (gegebenenfalls hydriertem) Polyisobuten, enthält. Weiter bevorzugte erfindungsgemäße Zusammensetzungen sind **dadurch gekennzeichnet, dass** der in dem inversen Polymerlatex enthaltene Öl-in-Wasser-Emulgator ausgewählt ist aus den Ethylenoxid-Addukten von Sorbitanoleat, Ricinusöl, das gewünschtenfalls gehärtet ist, Sorbitanlaurat und Laurylalkohol, sowie aus Caprylic Glucoside und Capric Glucoside. Die erfindungsgemäß bevorzugten verdickenden Polymerlatices weisen vorzugsweise einen Polymergehalt von 30 - 90 Gew.-%, bevorzugt 35 - 75 Gew.-% und besonders bevorzugt 40 - 60 Gew.-%, jeweils bezogen auf den gesamten Latex, auf. Der Polymergehalt des Latex hat dabei jedoch eher nur eine Bedeutung für die Herstellung der erfindungsgemäßen Zusammensetzungen, z. B. in Bezug auf die Mischbarkeit bzw. Dosierbarkeit. Für die erfindungsgemäßen Zusammensetzungen selbst ist eher der Polymergehalt an sich, bezogen auf die erfindungsgemäße Zusammensetzung, bedeutsam.

Bezogen auf die gesamte erfindungsgemäße kosmetische Zusammensetzung, wird das verdickende Polymer in Mengen von 0,1 - 5 Gew.-%, bevorzugt 0,3 - 3 Gew.-% und besonders bevorzugt 0,5 - 2,5 Gew.-%, bezogen auf die gesamte erfindungsgemäße kosmetische Zusammensetzung, eingesetzt, wobei Polymergehalte von 0,4, 0,6, 0,7, 0,8, 0,9, 1,0, 1,1, 1,2, 1,3, 1,4, 1,5, 1,6, 1,7, 1,8, 1,9, 2,0, 2,1, 2,2, 2,3 und 2,4 Gew.-% besonders bevorzugt und Polymergehalte von 0,3, 0,4, 0,5, 0,6, 0,7, 0,8, 0,9, 1,0 und 1,1 Gew.-% außerordentlich bevorzugt sein können.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol^{®} im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze^{®} QM im Handel erhältlich.

Weiterhin können als Polymere zur Steigerung der Wirkung der erfindungsgemäßen Wirkstoffkombination amphotere Polymere (G3) verwendet werden. Unter dem Begriff amphotere Polymere werden sowohl solche Polymere, die im Molekül sowohl freie Aminogruppen als auch freie -COOH- oder SO₃H-Gruppen enthalten und zur Ausbildung innerer Salze befähigt sind, als auch zwitterionische Polymere, die im Molekül quartäre Ammoniumgruppen und -COO⁻- oder -SO₃⁻-Gruppen enthalten, und solche Polymere zusammengefaßt, die -COOH- oder SO₃H-Gruppen und quartäre Ammoniumgruppen enthalten.

Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Bevorzugt eingesetzte amphotere Polymere sind solche Polymerisate, die sich im wesentlichen zusammensetzen aus
(a) Monomeren mit quartären Ammoniumgruppen der allgemeinen Formel (G3-I),

   R¹-CH=CR²-CO-Z-(CₙH₂ₙ)-N⁽⁺⁾R³R⁴R⁵ A⁽⁻⁾ (G3-I)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoffatomen, Z eine NH- Gruppe oder ein Sauerstoffatom, n eine ganze Zahl von 2 bis 5 und A⁽⁻⁾ das Anion einer organischen oder anorganischen Säure ist, und
(b) monomeren Carbonsäuren der allgemeinen Formel (G3-II),

   R⁶-CH=CR⁷-COOH (G3-II)
in denen R⁶ und R⁷ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Ganz besonders bevorzugt sind solche Polymerisate, bei denen Monomere des Typs (a) eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-Ion ist; Acrylamidopropyl-trimethyl-ammoniumchlorid ist ein besonders bevorzugtes Monomeres (a). Als Monomeres (b) für die genannten Polymerisate wird bevorzugt Acrylsäure verwendet.

Die erfindungsgemäßen Mittel können in einer weiteren bevorzugten Ausführungsform nichtionogene Polymere (G4) enthalten. Geeignete nichtionogene Polymere sind beispielsweise:
- Vinylpyrrolidon/Vinylester-Copolymere, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) und Natrosol^{®}-Typen (Hercules) vertrieben werden.
- Stärke und deren Derivate, insbesondere Stärkealkylether;
- Polyvinylpyrrolidone, wie sie beispielsweise unter der Bezeichnung Luviskol^{®} (BASF) vertrieben werden.
   Es ist erfindungsgemäß auch möglich, dass die verwendeten Zusammensetzungen mehrere, insbesondere zwei verschiedene Polymere gleicher Ladung und/oder jeweils ein ionisches und ein amphoteres und/oder nicht ionisches Polymer enthalten.

Die Polymere (G) sind in den erfindungsgemäßen Mitteln bevorzugt in Mengen von 0,05 bis 10 Gew.-%, bezogen auf das gesamte Mittel, enthalten. Mengen von 0,1 bis 5, insbesondere von 0,1 bis 3 Gew.-%, sind besonders bevorzugt.

Eine weitere Gruppe von Pflegestoffen, die in den erfindungsgemäßen Mitteln enthalten sein kann, sind die Proteinhydrolysate und deren Derivate (P) enthalten. Proteinhydrolysate sind Produktgemische, die durch sauer, basisch oder enzymatisch katalysierten Abbau von Proteinen erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch β-Aminosäuren und deren Derivate wie β-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton.

Erfindungsgemäß können Proteinhydrolysate sowohl pflanzlichen als auch tierischen oder marinen oder synthetischen Ursprungs eingesetzt werden. Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Selbstverständlich umfasst die erfindungsgemäße Lehre alle isomeren Formen, wie cis - trans - Isomere, Diastereomere und chirale Isomere. Erfindungsgemäß ist es auch bevorzugt, eine Mischung aus mehreren Proteinhydrolysaten (P) einzusetzen. Die Proteinhydrolysate (P) sind in den Mitteln bevorzugt in Konzentrationen von 0,01 Gew.-% bis zu 20 Gew.-%, besonders bevorzugt von 0,05 Gew.- bis zu 15 Gew.-% und ganz besonders bevorzugt in Mengen von 0,05 Gew.-% bis zu 5 Gew.-% enthalten.

Die erfindungsgemäßen Mittel können weiterhin bevorzugt eine 2-Pyrrolidinon-5-carbonsäure und deren Derivate (J) enthalten. Besonders bevorzugt sind die Natrium-, Kalium-, Calcium-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäßen Mitteln betragen vorzugsweise 0,05 bis 10 Gew.-%, bezogen auf das gesamte Mittel, besonders bevorzugt 0,1 bis 5, und insbesondere 0,1 bis 3 Gew.-%.

Zusätzlich kann es sich als vorteilhaft erweisen, wenn in den erfindungsgemäßen Zusammensetzungen mindestens ein wasserlösliches mehrwertiges C₂ - C₉-Alkanol mit 2 - 6 Hydroxylgruppen und/oder mindestens ein wasserlösliches Polyethylenglycol mit 3 - 20 Ethylenoxid-Einheiten sowie Mischungen hiervon enthalten ist. Derartige Polyole können ein besonders ebenmäßiges Ergebnis der Hautaufhellung begünstigen.

Erfindungsgemäß wird unter Wasserlöslichkeit eine Löslichkeit von wenigstens 5 Gew.-% bei 20 °C verstanden, das heißt, dass Mengen von wenigstens 5 g des mehrwertigen C₂ - C₉-Alkanols mit 2 - 6 Hydroxylgruppen bzw. des Polyethylenglycols mit 3 - 20 Ethylenoxid-Einheiten in 95 g Wasser bei 20 °C löslich sind.

Bevorzugt sind diese Komponenten ausgewählt aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,2-Hexandiol und 1,6-Hexandiol, Hexantriolen wie 1,2,6-Hexantriol, 2-Ethyl-2-hydroxymethyl-1,3-propandiol, 1,2-Octandiol, 1,8-Octandiol, Diethylenglycol, Triethylenglycol, Dipropylenglycol, Tripropylenglycol, Diglycerin, Triglycerin, Erythrit, Sorbit sowie Mischungen der vorgenannten Substanzen. Geeignete wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind. Auch Zucker und bestimmte Zuckerderivate wie Fructose, Glucose, Maltose, Maltitol, Mannit, Inosit, Sucrose, Trehalose und Xylose sind erfindungsgemäß geeignet.

Besonders bevorzugte erfindungsgemäße Zubereitungen sind **dadurch gekennzeichnet, dass** das mindestens eine wasserlösliche mehrwertige C₂ - C₉-Alkanol mit 2 - 6 Hydroxylgruppen und/ oder mindestens eine wasserlösliche Polyethylenglycol mit 3 - 20 Ethylenoxid-Einheiten insgesamt in Mengen von 3 - 25 Gew.-%, bevorzugt 5 - 18 Gew.-%, besonders bevorzugt 10 - 15 Gew.-%, außerordentlich bevorzugt 6, 7, 8, 9, 10, 11, 12, 13, 14, 15, 16 oder 17 Gew.-%, jeweils bezogen auf die Gesamtzusammensetzung, enthalten ist.

Die erfindungsgemäßen Zusammensetzungen können Parfüme, Parfümöle oder Parfümölbestandteile enthalten.

Die erfindungsgemäßen Zusammensetzungen eignen sich zur Aufhellung der Haut und können dementsprechend eingesetzt werden. Dabei kann die Haut lokal begrenzt (z.B. auf Sommersprossen, Altersflecken oder Fehlpigmentierungen) oder großflächig (zur Hautaufhellung) behandelt werden.

Weitere Gegenstände der vorliegenden Erfindung sind daher die Verwendung von erfindungsgemäßen kosmetischen oder dermatologischen Zusammensetzungen zur Aufhellung der Hautfarbe, zur Reduzierung der Pigmentierung der Haut, zum Ausgleich des Erscheinungsbildes einer ungleichmäßigen Hautpigmentierung und/oder zur Aufhellung von Altersflecken der Haut.

Ein weiterer Gegenstand der vorliegenden Erfindung ist die Verwendung von erfindungsgemäßen kosmetischen und dermatologischen Zusammensetzungen zur Behandlung postinflammatorischer Hyperpigmentierung.

Bezüglich bevorzugter Ausführungsformen der erfindungsgemäßen Verwendungen gilt mutatis mutandis das zu den erfindungsgemäßen Zusammensetzungen Gesagte.

Die nachfolgenden Beispiele sollen den Gegenstand der Erfindung näher erläutern, ohne ihn einzuschränken.

**1. Beispielserie: Hautaufhellende lamellare Öl-in-Wasser-Emulsionen**

| | 1.1 | 2.1 | 3.1 |
|---|---|---|---|
| Distelöl | 3,00 | 3,00 | 3,00 |
| Myritol 318 | 5,00 | 5,00 | 5,00 |
| Novata AB | 2,00 | 2,00 | 2,00 |
| Lanette 22 | 1,00 | 1,00 | 1,00 |
| Cutina MD | 2,00 | 2,00 | 2,00 |
| Stenol 1618 | 1,00 | 1,00 | 1,00 |
| Isopropylstearat | 4,00 | 4,00 | 4,00 |
| Schibutter | 2,00 | 2,00 | 2,00 |
| Dimethicone (350 cSt) | 1,00 | 1,00 | 1,00 |
| Controx KS | 0,05 | 0,05 | 0,05 |
| Propylparaben | 0,20 | 0,20 | 0,20 |
| Dow Corning Fluid 1501 | 1,00 | 1,00 | 1,00 |
| Dry Flo Plus | 1,00 | 1,00 | 1,00 |
| Kronos 1171 (CI 77891) | 0,50 | 0,50 | 0,50 |
| 1,6-Hexandiol | 6,00 | 3,00 | - |
| Propylenglycol | 5,00 | 5,00 | 5,00 |
| Glycerin | 5,00 | 3,00 | 3,00 |
| Methylparaben | 0,20 | 0,20 | 0,20 |
| Tego Carbomer | 0,40 | 0,40 | 0,40 |
| DSH-CN | 5,00 | 5,00 | 5,00 |
| Biotin | - | 0,05 | 0,1 |
| Arlatone Dioic Acid | 1,00 | 0,50 | 1,20 |
| Sulforaphan | 0,05 | 0,10 | 0,20 |
| Parfum | 0,10 | 0,10 | 0,10 |
| Aqua | ad 100 | ad 100 | ad 100 |

**2. Beispielserie: Hautaufhellende Öl-in-Wasser-Emulsionen**

| | 2.1 | 2.2 | 2.3 |
|---|---|---|---|
| Cetiol SN | 4,00 | 4,00 | 4,00 |
| Mineral oil | 6,00 | 6,00 | 6,00 |
| Cutina CBS | 2,00 | 2,00 | 2,00 |
| Edenor L2 SM | 1,50 | 1,50 | 1,50 |
| Emulgin B3 | 1,00 | 1,00 | 1,00 |
| Baysilon M 350 | 1,00 | 1,00 | 1,00 |
| Tocopherylacetat | 0,50 | 0,50 | 0,50 |
| Propylparaben | 0,30 | 0,30 | 0,30 |
| Mandelöl | 2,00 | 2,00 | 2,00 |
| Pemulen TR-1 | 0,27 | 0,27 | 0,27 |
| Glycerin | 5,00 | 3,00 | 3,00 |
| Milchsäure 80% | 0,26 | 0,26 | 0,26 |
| Propylenglycol | 5,00 | 5,00 | 5,00 |
| Methylparaben | 0,30 | 0,30 | 0,30 |
| Phenoxyethanol | 0,90 | 0,90 | 0,90 |
| Panthenol | 0,50 | - | - |
| Seanergilium BG | 1,00 | - | - |
| Aqua (Water), Propylene Glycol, Nelumbo Nucifera Flower Extract, Nelumbo Nucifera Root Extract | - | 2,00 | - |
| Simulgel EPG | 0,50 | 0,50 | 0,50 |
| Silk Protein | 0,05 | 0,05 | - |
| Keltrol SF | 0,20 | 0,20 | 0,20 |
| Arlatone Dioic Acid | 1,00 | 0,50 | 0,20 |
| Sulforaphan | 0,1 | 0,1 | 0,05 |
| Allylisothiocyanat | - | 0,05 | 0,05 |
| Magnesiumascorbylphosphat | - | 0,50 | - |
| Resveratrol | - | - | 0,1 |
| Parfum | 0,30 | 0,30 | 0,30 |
| Aqua | ad 100 | ad 100 | ad 100 |

**3. Hautaufhellende Antifalten-Cremes in Form von Öl-in-Wasser-Emulsionen mit Liposomen**

| | 3.1 | 3.2 | 3.3 |
|---|---|---|---|
| Lipoid S 75-3 | 0,50 | 0,50 | 0,50 |
| Isopropylstearat | 4,00 | 4,00 | 4,00 |
| Cetiol B | 2,00 | 2,00 | 2,00 |
| Tocopherylacetat | 0,50 | 0,50 | 0,50 |
| Cutina MD | 1,00 | 1,00 | 1,00 |
| Lanette 22 | 2,00 | 2,00 | 2,00 |
| Dimethicone (350 cSt) | 0,50 | 0,50 | 0,50 |
| Propylparaben | 0,20 | 0,20 | 0,20 |
| Dow Corning 9040 | 1,00 | 1,00 | 1,00 |
| Glycerin | 4,50 | 3,00 | 3,00 |
| 1,6-Hexandiol | 6,00 | 3,00 | - |
| Methylparaben | 0,20 | 0,20 | 0,20 |
| Tego Carbomer 140 | 0,30 | 0,30 | 0,30 |
| DSH-CN | 5,00 | 5,00 | 5,00 |
| Photosomes | - | 1,00 | - |
| Lipochroman-6 | 0,01 | 0,01 | 0,05 |
| Propylenglycol | 5,00 | 5,00 | 5,00 |
| Matrixyl | 3,00 | - | - |
| Matrixyl 3000 | - | 3,00 | - |
| Simulgel NS | 1,50 | 1,50 | 1,50 |
| Kronos 1171 (CI 77891) | 0,50 | 0,50 | - |
| Arlatone Dioic Acid | 1,00 | 0,50 | 0,20 |
| Sulforaphan | 0,1 | - | - |
| Allylisothiocyanat | - | 0,05 | 0,05 |
| Benzylisothiocyanat | - | 0,05 | 0,05 |
| Phenylethylisothiocyanat | - | 0,05 | 0,05 |
| Ethylisothiocyanat | - | 0,1 | 0,1 |
| Barbarin | - | 0,1 | 0,1 |
| Ascorbyltetraisopalmitat | - | - | 0,50 |
| Parfum | 0,35 | 0,35 | 0,35 |
| Aqua | ad 100 | ad 100 | ad 100 |

**4. Hautaufhellende Cremes in Form von Öl-in-Wasser-Emulsionen mit Lichtschutzfaktor**

| | 4.1 | 4.2 | 4.3 | 4.4 | 4.5 | 4.6 | 4.7 | 4.8 | 4.9 | 4.10 |
|---|---|---|---|---|---|---|---|---|---|---|
| Montanov 68 | 5,00 | 5,00 | 5,00 | 5,00 | 5,00 | 5,00 | 5,00 | 5,00 | 5,00 | 5,00 |
| Myritol 318 | 5,00 | 5,00 | 5,00 | 5,00 | 5,00 | 5,00 | 5,00 | 5,00 | 5,00 | 5,00 |
| Cetiol SB 45 | 0,50 | 0,50 | 0,50 | 0,50 | 0,50 | 0,50 | 0,50 | 0,50 | 0,50 | 0,50 |
| Novata AB | 2,00 | 2,00 | 2,00 | 2,00 | 2,00 | 2,00 | 2,00 | 2,00 | 2,00 | 2,00 |
| Stenol 1618 | 1,00 | 1,00 | 1,00 | 1,00 | 1,00 | 1,00 | 1,00 | 1,00 | 1,00 | 1,00 |
| Dimethicone 350 cSt | 0,50 | 0,50 | 0,50 | 0,50 | 0,50 | 0,50 | 0,50 | 0,50 | 0,50 | 0,50 |
| Controx KS | 0,25 | 0,25 | 0,25 | 0,25 | 0,25 | 0,25 | 0,25 | 0,25 | 0,25 | 0,25 |
| Parsol SLX | 4,00 | 3,00 | - | - | 3,00 | 4,00 | 3,00 | 3,00 | - | 2,00 |
| Parsol HS | 2,00 | 2,00 | 2,00 | - | - | 2,00 | - | - | - | 2,00 |
| Parsol 340 | - | - | - | 2,00 | 2,00 | - | 2,00 | - | - | - |
| Uvinul MBC 95 | - | - | - | - | - | - | - | 4,00 | 4,00 | - |
| Parsol1789 | 1,80 | 1,80 | 1,80 | - | - | 2,00 | 2,00 | 2,00 | 2,00 | 2,00 |
| Uvinul T 150 | - | 2,50 | - | 2,50 | 2,50 | - | 3,00 | 2,50 | - | 2,50 |
| Uvinul A Plus | - | - | - | - | 1,00 | - | - | - | - | - |
| Uvinul A plus B | - | - | - | 1,00 | - | - | - | - | - | - |
| Eusolex T-AVO | - | - | 2,00 | - | - | - | - | - | - | - |
| Tioveil TG | - | - | - | - | - | 2,00 | - | - | 2,00 | - |
| Propylparaben | 0,20 | 0,20 | 0,20 | 0,20 | 0,20 | 0,20 | 0,20 | 0,20 | 0,20 | 0,20 |
| Tego Carbomer 140 | 0,50 | 0,50 | 0,50 | 0,50 | 0,50 | 0,50 | 0,50 | 0,50 | 0,50 | 0,50 |
| 1,6-Hexandiol | 6,00 | 6,00 | 3,00 | 6,00 | 6,00 | 6,00 | 6,00 | 6,00 | 6,00 | 6,00 |
| Talkum | 0,50 | 0,50 | 0,50 | 0,50 | 0,50 | 0,50 | 0,50 | 0,50 | 0,50 | 0,50 |
| Methylparaben | 0,20 | 0,20 | 0,20 | 0,20 | 0,20 | 0,20 | 0,20 | 0,20 | 0,20 | 0,20 |
| Glycerin | 4,50 | 4,50 | 3,00 | 4,50 | 3,00 | 4,50 | 4,50 | 4,50 | 4,50 | 4,50 |
| Dry Flo Plus | 1,00 | 1,00 | 1,00 | 1,00 | 1,00 | 1,00 | 1,00 | 1,00 | 1,00 | 1,00 |
| Natipide 2 PG | 1,00 | 1,00 | 1,00 | 1,00 | 1,00 | 1,00 | 1,00 | 1,00 | 1,00 | 1,00 |
| Gatuline R/C | - | - | - | - | - | - | - | 2,00 | 2,00 | 2,00 |
| DSH-CN | 2,00 | 2,00 | 2,00 | 2,00 | 2,00 | 2,00 | 2,00 | 2,00 | 2,00 | 2,00 |
| Trilon A | 0,10 | 0,10 | 0,10 | 0,10 | 0,10 | 0,10 | 0,10 | 0,10 | 0,10 | 0,10 |
| Phenoxyethanol | 0,40 | 0,40 | 0,40 | 0,40 | 0,40 | 0,40 | 0,40 | 0,40 | 0,40 | 0,40 |
| Arlatone Dioic Acid | 1,00 | 0,50 | 0,10 | 0,20 | 1,00 | 1,00 | 1,00 | 1,00 | 1,00 | 1,00 |
| Sulforaphan/ Sulforaphen (2:1) | 0,50 | - | - | - | - | - | - | - | - | - |
| Goitrin | - | 0,01 | 0,01 | 0,01 | 0,01 | 0,01 | 0,01 | 0,01 | 0,01 | 0,01 |
| Iberin | - | 0,01 | 0,01 | 0,01 | 0,01 | 0,01 | 0,01 | 0,01 | 0,01 | 0,01 |
| Brassicin | - | 0,01 | 0,01 | 0,01 | 0,01 | 0,01 | 0,01 | 0,01 | 0,01 | 0,01 |
| Allylisothiocyanat | - | 0,01 | 0,01 | 0,01 | 0,01 | 0,01 | 0,01 | 0,01 | 0,01 | 0,01 |
| Nasturtiin | - | 0,01 | 0,01 | 0,01 | 0,01 | 0,01 | 0,01 | 0,01 | 0,01 | 0,01 |
| Sulforaphan | - | 0,08 | 0,08 | 0,08 | 0,08 | 0,08 | 0,08 | 0,08 | 0,08 | 0,08 |
| Parfum | 0,40 | 0,40 | 0,40 | 0,40 | 0,40 | 0,40 | 0,40 | 0,40 | 0,40 | 0,40 |
| Aqua | ad 100 | ad 100 | ad 100 | ad 100 | ad 100 | ad 100 | ad 100 | ad 100 | ad 100 | ad 100 |

**5. Hautaufhellende Öl-in-Wasser-Emulsionen**

| | 5.1 | 5.2 | 5.3 |
|---|---|---|---|
| Paraffinum Liquidum | 20,00 | 20,00 | 20,00 |
| Hostaphat KW 340 D | 2,50 | 2,50 | 2,50 |
| Cetearyl Alcohol | 1,40 | 1,40 | 1,40 |
| Eumulgin B 1 | 1,70 | 1,70 | 1,70 |
| Vitamin E Acetat | 0,25 | 0,25 | 0,25 |
| Propylparaben | 0,20 | 0,20 | 0,20 |
| Glycerin 86% pflanzlich | 2,00 | 2,00 | 2,00 |
| Hexandiol-1,6 | 5,00 | 5,00 | 5,00 |
| Sorbit 70% LS DAB | 1,00 | 1,00 | 1,00 |
| Methylparaben | 0,20 | 0,20 | 0,20 |
| Tego Carbomer 140 | 0,30 | 0,30 | 0,30 |
| Keltrol SF | 0,10 | 0,10 | 0,10 |
| D-Panthenol 75 % | 0,70 | 0,70 | 0,70 |
| Phenoxyethanol, rein | 0,40 | 0,40 | 0,40 |
| Calcium D-Pantothenat | 0,05 | 0,05 | 0,05 |
| Arlatone Dioic Acid | 1,00 | 0,20 | 0,50 |
| Sulforaphan | 0,2 | 0,08 | 0,08 |
| Goitrin | - | 0,01 | 0,01 |
| Iberin | - | 0,01 | 0,01 |
| Brassicin | - | 0,01 | 0,01 |
| Allylisothiocyanat | - | 0,01 | 0,01 |
| Nasturtiin | - | 0,01 | 0,01 |
| Parfum | 0,25 | 0,25 | 0,25 |
| Aqua | ad 100 | ad 100 | ad 100 |

**6. Hautaufhellende Wasser-in-Öl-Emulsion**

| | 6.1 | 6.2 | 6.3 |
|---|---|---|---|
| Lameform TGI | 3,00 | 3,00 | 3,00 |
| PEG-45/Dodecyl Glycol Copolymer | 0,50 | 0,50 | 0,50 |
| Microcrystalline Wax | 3,00 | 3,00 | 3,00 |
| Bis-Diglyceryl Polyacyladipate-2 | 1,00 | 1,00 | 1,00 |
| Paraffinöl | 8,00 | 8,00 | 8,00 |
| Vaseline | 2,00 | 2,00 | 2,00 |
| Vitamin E Acetat | 2,00 | 2,00 | 2,00 |
| Methylparaben | 0,30 | 0,30 | 0,30 |
| Propylparaben | 0,30 | 0,30 | 0,30 |
| Isopropylisostearat | 8,00 | 8,00 | 8,00 |
| Glycerin | 5,00 | 3,00 | 3,00 |
| Mg-Sulfat | 0,50 | 0,50 | 0,50 |
| Milchsäure 80%ig | 0,56 | 0,56 | 0,56 |
| Panthenol | 0,50 | 1,00 | 0,50 |
| Arlatone Dioic Acid | 1,00 | 0,20 | 0,50 |
| Natriumascorbylphosphat | - | 0,50 | - |
| Sulforaphan | 0,1 | 0,2 | 0,1 |
| Parfum | 0,20 | 0,20 | 0,20 |
| Aqua | ad 100 | ad 100 | ad 100 |

**7. Hautaufhellende Wasser-in-Silicon-Emulsionen**

| | 7.1 | 7.2 | 7.3 |
|---|---|---|---|
| Belsil DM 100 | 2,50 | 2,50 | 2,50 |
| Dow Corning 245 Fluid | 25,00 | 25,00 | 25,00 |
| Abil EM 90 | 2,00 | 2,00 | 2,00 |
| Natriumchlorid | 2,00 | 2,00 | 2,00 |
| Parfum | 0,30 | 0,30 | 0,30 |
| Symdiol 68 | 0,30 | 0,30 | 0,30 |
| Phenoxyethanol | 0,40 | 0,40 | 0,40 |
| Arlatone Dioic Acid | 1,00 | 0,20 | 0,10 |
| Sulforaphan | 0,1 | 0,2 | 0,1 |
| Aqua | ad 100 | ad 100 | ad 100 |

**8. Hautaufhellende Reinigungszusammensetzungen**

| | 8.1 | 8.2 | 8.3 |
|---|---|---|---|
| Dipropylenglycol | 10,00 | 10,00 | 10,00 |
| Chlorhexidindigluconat | 1,00 | 1,00 | 1,00 |
| Synperonic PE7L 64 | 3,00 | 3,00 | 3,00 |
| D-Panthenol | 0,50 | 0,50 | 0,50 |
| Hydagen CMF | 3,00 | 3,00 | 3,00 |
| PEG-40 Hydrogenated Castor Oil Trideceth 9 // Propylene Glycol | 0,50 | 0,50 | 0,50 |
| Chlorella Vulgaris Extract | 0,50 | 0,50 | 0,50 |
| Arlatone Dioic Acid | 1,00 | 0,50 | 0,20 |
| Sulforaphan | 0,1 | 0,2 | 0,1 |
| Parfume | 0,20 | 0,20 | 0,20 |
| Aqua | ad 100 | ad 100 | ad 100 |
| | | | |

| | 8.4 | 8.5 | 8.6 |
|---|---|---|---|
| Carbopol ETD 2001 | 1,40 | 1,40 | 1,40 |
| Sorbitol | 2,10 | 2,10 | 2,10 |
| Natriumbenzoat | 0,40 | 0,40 | 0,40 |
| Plantacare 2000UP | 7,50 | 7,50 | 7,50 |
| Cocoamidopropyl betaine | 3,40 | 3,40 | 3,40 |
| Texapon SB 3 | 5,00 | 5,00 | 5,00 |
| Cetiol HE | 0,50 | 0,50 | 0,50 |
| Lamesoft PO 65 | 5,00 | 5,00 | 5,00 |
| Controx KS | 0,05 | 0,05 | 0,05 |
| Ajidew NL 50 | 1,60 | 1,60 | - |
| Pantolacton | 1,00 | 1,00 | - |
| Trilon B | 0,25 | 0,25 | 0,25 |
| Natriumlactat | 1,80 | - | - |
| Arlatone Dioic Acid | 1,00 | 0,20 | 0,50 |
| Sulforaphan | 0,2 | 0,08 | 0,08 |
| Goitrin | - | 0,01 | 0,01 |
| Iberin | - | 0,01 | 0,01 |
| Brassicin | - | 0,01 | 0,01 |
| Allylisothiocyanat | - | 0,01 | 0,01 |
| Nasturtiin | - | 0,01 | 0,01 |
| Parfum | 0,40 | 0,40 | 0,40 |
| Aqua | ad 100 | ad 100 | ad 100 |
| | | | |

| | 8.7 | 8.8 | 8.9 |
|---|---|---|---|
| Hostapon SCI-65 | 22,50 | 22,50 | 22,50 |
| Talkum | 4,00 | 4,00 | 4,00 |
| Cutina FS 45 | 6,00 | 6,00 | 6,00 |
| Cocoamidopropyl betain 40% | 9,50 | 9,50 | 9,50 |
| Na-benzoat | 0,40 | 0,40 | 0,40 |
| Sorbitol 70% | 1,00 | 1,00 | 1,00 |
| Glycerin 86% | 1,00 | 1,00 | 1,00 |
| Arlatone Dioic Acid | 1,00 | 0,20 | 0,50 |
| Sulforaphan | 0,2 | 0,08 | 0,08 |
| Goitrin | - | 0,01 | 0,01 |
| Iberin | - | 0,01 | 0,01 |
| Brassicin | - | 0,01 | 0,01 |
| Allylisothiocyanat | - | 0,01 | 0,01 |
| Nasturtiin | - | 0,01 | 0,01 |
| Parfum | 0,30 | 0,30 | 0,30 |
| Aqua | ad 100 | ad 100 | ad 100 |

**Verwendete Rohstoffe**

| | | |
|---|---|---|
| Handelsname | INCI-Bezeichnung | Lieferant/ Hersteller |
| Abil EM 90 | Cetyl dimethicone copolyol | Goldschmidt |
| Ajidew NL 50 | AQUA, SODIUM PCA | Ajinomoto |
| Arlatone Dioic Acid | Octadecenedioic Acid | Uniquema |
| Baysilon M350 | Silikonöl | Bayer |
| Belsil DM 100 | Dimethicone | Wacker |
| Carbopol ETD 2001 | Carbomer | Noveon |
| Cetiol^{®} B | Dibutyl Adipate | Cognis |
| Cetiol^{®} HE | PEG-7 Glyceryl Cocoate | Cognis |
| Cetiol^{®} SB 45 | Butyrospermum Parkii (Shea butter) | Cognis |
| Cetiol^{®} SN | Cetearyl Isononanoate | Cognis |
| Controx KS | Tocopherol, Hydrogenated Palm Glycerides Citrate | Cognis |
| Cutina CBS | Glyceryl Stearate / Cetearyl Alcohol / Cetyl Palmitate / Cocoglycerides | Cognis |
| Cutina MD | Glyceryl Stearate | Cognis |
| Cutina FS 45 | Stearic Acid & Palmitic Acid | Cognis |
| Dow Corning 9040 | Cyclopentasiloxane (and) Dimethicone Crosspolymer (and) Cyclohexasiloxane | Dow Corning |
| Dow Corning 245 Fluid | Cyclomethicone | Dow Corning |
| Dow Corning 1501 Fluid | Cyclopentasiloxane (and) Dimethiconol | Dow Corning |
| Dry Flo Plus | Aluminium Starch Octenylsuccinate | National Starch |
| DSH-CN | Water, Dimethylsilanol Hyaluronate | Exsymol |
| Edenor L2 SM | Palmitin-/Stearinsäure | Cognis |
| Ederline L | Hexyldecanol, Pyrus Malus (Apple) Fruit Extract | Impag |
| Eumulgin B1 | Ceteareth-10 | Cognis |
| Eumulgin B3 | Ceteareth-30 | Cognis |
| Eusolex T-AVO | Titanium Dioxide (and) Silica | Merck KGaA |
| Fucogel 1000 | Biosaccharide Gum-1 | Solabia |
| Gatuline R/C | Fagus Silvatica (Beech Tree Buds) Extract | Gattefossé |
| Hostaphat KW 340 D | Mono, di and tri (alkyltetraglycolether) - o - Phosphoric acid esters | Clariant |
| Hostapon SCI-65 | Sodium Cocoyl Isethionate | Clariant |
| Hydagen CMF | Chitosan Glycolate | Cognis |
| Keltrol SF | Xanthan Gum | Kelco |
| Lameform TGI | Polyglyceryl-3 Diisostearate | Cognis |
| Lamesoft PO 65 | Coco-Glucoside & Glyceryl Oleate | Cognis |
| Lanette 22 | Behenyl Alcohol | Cognis |
| Lipochroman-6 | Dimethylmethoxy Chromanol | Centerchem |
| Lipoid S 75-3 | Aqua (Water), Hydrogenated Lecithin | Lipoid GmbH |
| Matrixyl | Aqua, Palmitoyl Pentapeptide-3 | Sederma |
| Matrixyl 3000 | Glycerin, Aqua, Butylene Glycol, Carbomer, Polysorbate 20, Palmitoyl Oligopeptide, Palmitoyl Tetrapeptide-1 | Sederma |
| Montanov 68 | Cetearyl Alcohol and Cetearyl Glucoside | Seppic |
| Myritol 318 | Caprylic/Capric Triglyceride | Cognis |
| Natipide 2 PG | Aqua (and) Lecithin (and) Propylene Glycol | Phospholipid GmbH |
| Neo Heliopan AP | Disodium Phenyl Dibenzimidazole Tetrasulfonate | Symrise |
| Novata AB | Cocoglycerides | Cognis |
| Parsol SLX | Polysilicone-15 | DSM |
| Parsol HS | Phenylbenzimidazole Sulfonic Acid | DSM |
| Parsol 340 | Octocrylene | DSM |
| Parsol 1789 | Butyl Methoxydibenzoylmethane | DSM |
| Pemulen TR 1 | Acrylates/C10-30 Alkyl Acrylate Crosspolymer | Noveon |
| Photosomes | Aqua (Water), Lecithin, Plankton Extract | Barnet |
| Plantacare 2000 UP | Decyl Glucoside, Aqua (Water), 51 - 55 Gew.-% Aktivsubstanz | Cognis |
| Seanergilium BG | Laminaria Digitata EXTRACT, Butylene glycol, Methylparaben | Coletica |
| Simulgel^{®} EPG | Aqua, Sodium Acrylate/Sodium Acryloyl-dimethyl Taurate Copolymer, Polyisobutene, Caprylyl/Capryl Glucoside | Seppic |
| Simulgel^{®} NS | Hydroxyethyl Acrylate / Sodium Acryloyl-dimethyl Taurate Copolymer / Squalane / Polysorbate 60 | Seppic |
| Stenol 1618 | Cetearyl Alcohol | Cognis |
| Symdiol 68 | 1,2-Octandiol, 1,2-Hexandiol | Symrise |
| Synperonic PE/L 64 | Poloxamer 184 | Uniqema |
| Tego Carbomer 140 | Carbomer | Goldschmidt |
| Texapon SB 3 | Disodium Laureth sulfosuccinate | Cognis |
| Tioveil TG | Caprylic/Capric Triglyceride (and) Titanium Dioxide (and) Alumina (and) Polyhydroxystearic Acid (and) Silica | Uniqema |
| Trilon A | Trisodium EDTA | BASF |
| Trilon B | Tetrasodium EDTA | BASF |
| Uvinul A Plus | Diethylamino Hydroxybenzoyl Hexyl Benzoate | BASF |
| Uvinul A Plus B | Ethylhexyl Methoxycinnamate (and) Diethylamino Hydroxybenzoyl Hexyl Benzoate | BASF |
| Uvinul MBC 95 | Methylbenzylidene Camphor | BASF |
| Uvinul T 150 | Ethylhexyl triazone | BASF |

## Patentansprüche

1. Kosmetische oder dermatologische Zusammensetzung zur Aufhellung der Haut, enthaltend eine Kombination aus mindestens einem der unter a) und mindestens einem der unter b) aufgeführten Wirkstoffe,
a) 8-Hexadecen-1,16-dicarbonsäure und/oder 7-Tetradecen-1,14-dicarbonsäure und/oder 9-Octadecen-1,18-dicarbonsäure und/oder 6-Dodecen-1,12-dicarbonsäure und/oder 5-Decen-1,10-dicarbonsäure und/oder Decandisäure (Sebacinsäure) und/oder Nonandisäure (Azelainsäure) in einer Gesamtmenge von 0,01 - 5 Gew.-%;
b) 0,0001 bis 5 Gew.-% Gesamtgehalt an mindestens einer Substanz, ausgewählt aus der Gruppe, bestehend aus Sulforaphan, Sulforaphen, Sulforaphan-Analoga und Sulforaphen-Analoga.

2. Kosmetische oder dermatologische Zusammensetzung nach Anspruch 1, **dadurch gekennzeichnet, dass** sie, bezogen auf ihr Gewicht, 8-Hexadecen-1,16-dicarbonsäure und/oder
7-Tetradecen-1,14-dicarbonsäure und/oder 9-Octadecen-1,18-dicarbonsäure und/oder 6-Dodecen-1,12-dicarbonsäure und/oder 5-Decen-1,10-dicarbonsäure und/oder Sebacinsäure und/oder Azelainsäure in einer Gesamtmenge von 0,05 - 3,0 Gew.-%, vorzugsweise 0,07 - 2,0 Gew.-% und insbesondere 0,1 - 1,0 Gew.-%, enthält.

3. Kosmetische oder dermatologische Zusammensetzung nach einem der Ansprüche 1 oder 2, **dadurch gekennzeichnet, dass** sie, bezogen auf ihr Gewicht, insgesamt 0,0005 bis 3,0 Gew.-%, vorzugsweise 0,001 bis 2,0 Gew.-% und insbesondere 0,01 bis 1,0 Gew.-% mindestens einer Substanz, ausgewählt aus der Gruppe, bestehend aus Sulforaphan und Sulforaphen, enthält.

4. Kosmetische oder dermatologische Zusammensetzung nach einem der Ansprüche 1 - 3, **dadurch gekennzeichnet, dass** das Gewichtsverhältnis der Gesamtmenge an 8-Hexa-decen-1,16-dicarbonsäure, 7-Tetradecen-1,14-dicarbonsäure, 9-Octadecen-1,18-dicarbonsäure, 6-Dodecen-1,12-dicarbonsäure, 5-Decen-1,10-dicarbonsäure, Sebacinsäure und/ oder Azelainsäure zum Gesamtgehalt an Sulforaphan, Sulforaphen, Sulforaphan-Analoga und Sulforaphen-Analoga 1000 : 1 bis 1: 1, vorzugsweise 500 : 1 bis 10 : 1 und insbesondere 300 : 1 bis 50 : 1, beträgt.

5. Kosmetische oder dermatologische Zusammensetzung nach einem der Ansprüche 1 - 4, **dadurch gekennzeichnet, dass** das mindestens eine Sulforaphan-Analogon ausgewählt ist aus folgenden Verbindungen: 6-Isothiocyanato-2-hexanon (GHP 1105), exo-2-Acetyl-6-iso-thiocyanatonorbornan (GHP 1066), exo-2-Isothiocyanato-6-methylsulfonylnorbornan (GHP 1068), 6-Isothiocyanato-2-hexanol (GHP 1106), 1-Isothiocyanato-4-dimethylphosphonyl-butan (GHP 1078), exo-2-(1'-Hydroxyethyl)-5-isothiocyanatonorbornan (GHP 1075), exo-2-Acetyl-5-isothiocyanatonorbornan (GHP 1067), 1-Isothiocyanato-8-(methylsulfinyl)-octan (Hirsutin), 1-Isothiocyanato-5-methylsulfonylpentan (GHP 1003), 1-Isothiocyanato-5-methylsulfinylpentan (Alyssin, GHP 1002), 1-Isothiocyanato-4-methylsulfonylbutan (Erysolin, GHP 1007), 1-Isothiocyanato-3-methylsulfinylpropan (Iberin, GHP 1009), 1-Isothiocyanato-3-methylsulfonylpropan (GHP 1010), cis-3-(Methylsulfonyl)cyclohexylisothiocyanat (GHP 1073), cis-3-(Methylsulfonyl)cyclohexylmethylisothiocyanat (GHP 1079), trans-3-(Methylsulfonyl)cyclohexylmethylisothiocyanat (GHP 1080), 2-Isothiocyanato-4-methylsulfonyl-ethylbutyrat (GHP 1074), 9-Isothiocyanato-5-nonanon (GHP 1081), 7-Isothiocyanato-3-heptanon (Norcappasalin), 8-Isothiocyanato-4-octanon (Cappasalin), 7-Isothiocyanato-4-heptanon (Capangulin), exo-2-Cyano-5-isothiocyanatonorbornan (GHP 1071), 1-Cyano-4-isothiocyanatobutan (GHP 1101), 5-Isothiocyanato-methylpentanoat (GHP 1102), 5-Isothiocyanatopentansäure (GHP 1103), 2-Isothiocyanato-ethylacetat (GHP 1061), 4-Isothiocyanato-methylbutyrat (Erypestrin), 4-Isothiocyanato-ethylbutyrat, 1-Isothiocyanato-2-methoxyphenol (GHP 1031), Erucin (4-Methylthiobutylisothiocyanat, GHP 1006), Iberverin (3-Methylthiopropylisothiocyanat, GHP 1008), Berteroin (5-Methylthiopentylisothiocyanat, GHP 1001), Lesquerellin (6-Methylthiohexylisothiocyanat), Jirsutin (8-Methylthiooctylisothiocyanat), Arabin (9-Methylthiononylisothiocyanat), Allylisothiocyanat, Brassicin (Indol-3-ylmethylisothiocyanat), Goitrin (2-Hydroxybut-3-enyl-isothiocyanat), Napin (But-3-enyl-isothiocyanat), Neobrassicin (N-Methoxyindol-3-ylmethyl-isothiocyanat), N-Acetylindol-3-ylmethyl-isothiocyanat, Nasturtiin ((2-Phenylethyl)-isothiocyanat), Barbarin (2-Hydroxy-2-phenylethyl-isothiocyanat), Tropaeolin (Benzylisothiocyanat), Sinalbin (4-Hydroxybenzylisothiocyanat), Capparin (Methylisothiocyanat), Lepidin (Ethylisothiocyanat), Putranjivin (Isopropylisothiocyanat), Jiaputin (2-Methylbutylisothiocyanat), Raphasatin (4-Methylthiobut-3-enyl-isothiocyanat), Cochlearin (1-Methylpropylisothiocyanat), Brassicanapin (4-Pentenylisothiocyanat), Napoleiferin (2-Hydroxypent-4-enylisothiocyanat),

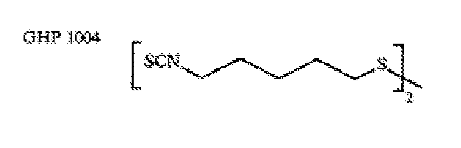

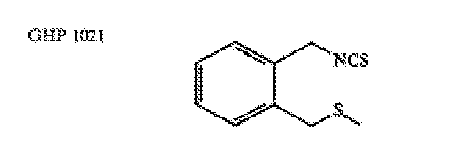

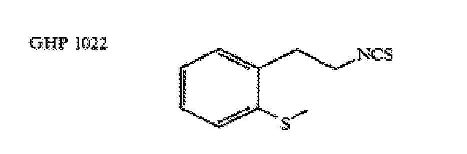

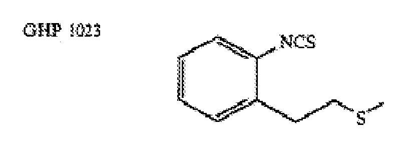

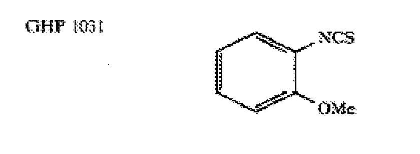

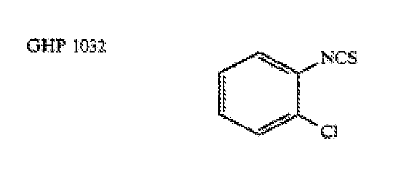

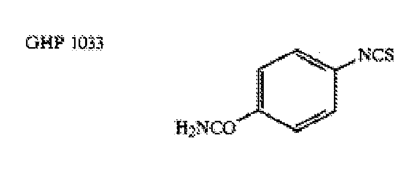

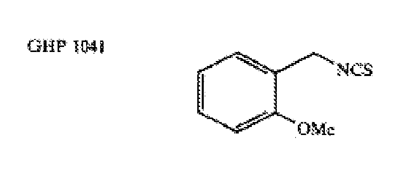

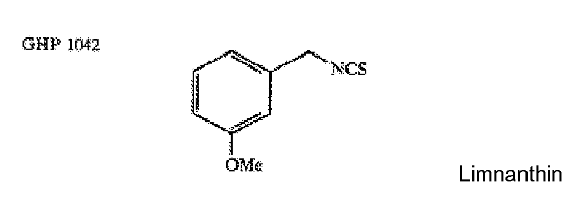

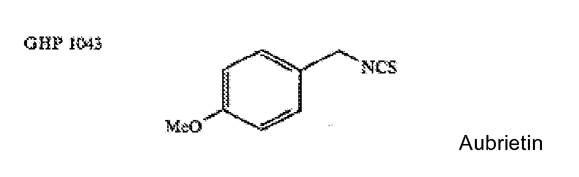

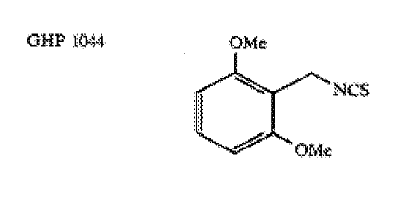

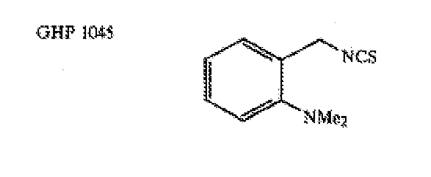

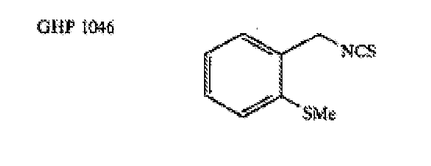

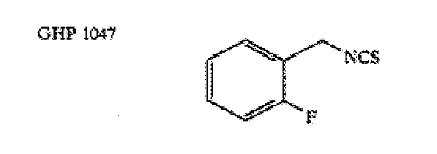

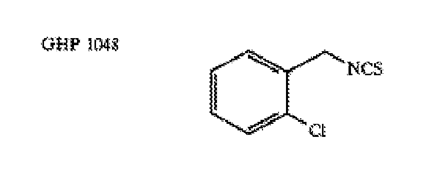

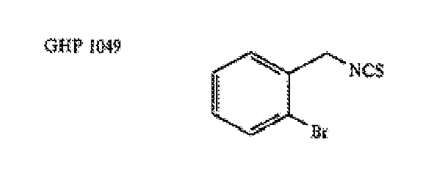

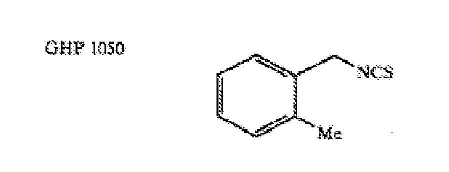

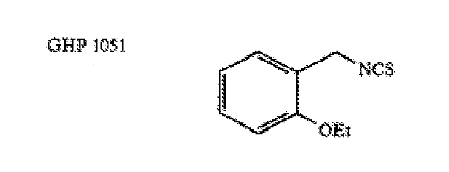

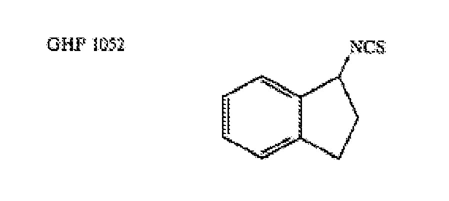

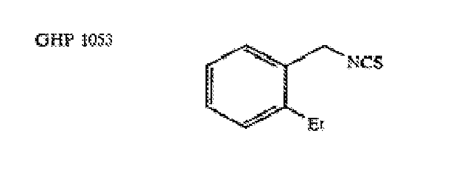

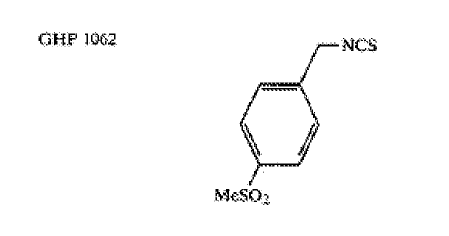

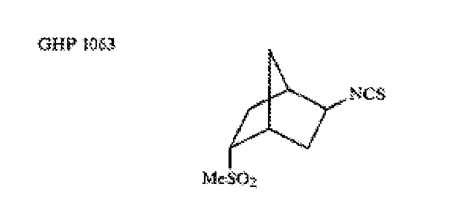

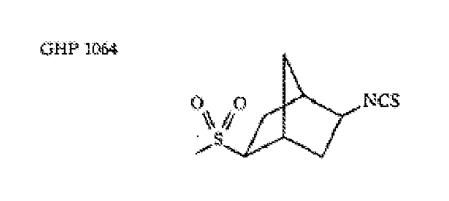

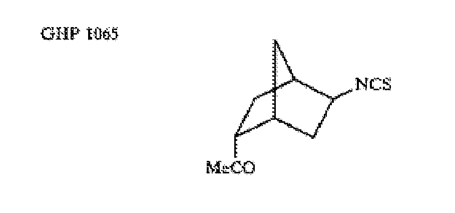

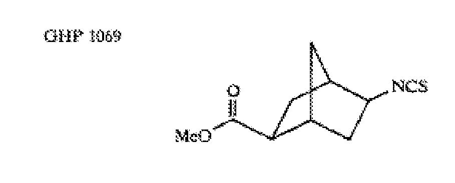

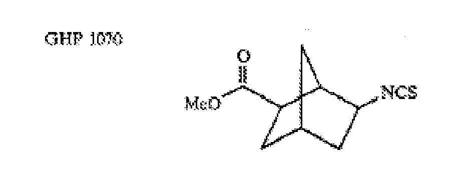

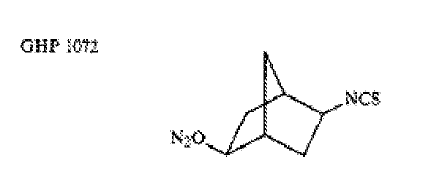

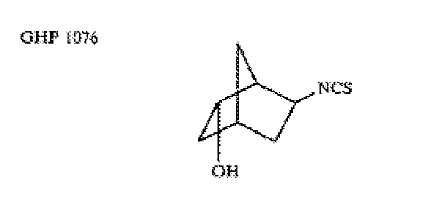

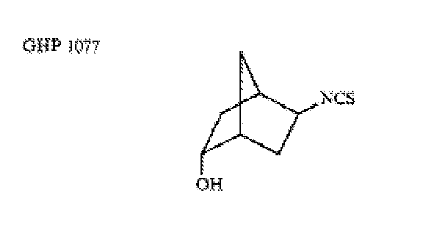

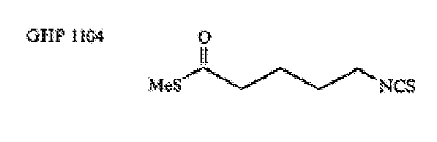

6. Kosmetische oder dermatologische Zusammensetzung nach einem der Ansprüche 1 - 5, **dadurch gekennzeichnet, dass** 0,01 - 5 Gew.-% 8-Hexadecen-1,16-dicarbonsäure und 0,0001 bis 5 Gew.-% Sulforaphan enthalten sind.

7. Kosmetische oder dermatologische Zusammensetzung nach einem der Ansprüche 1 - 6, **dadurch gekennzeichnet, dass** sie zusätzlich insgesamt 0,05 bis 10 Gew.-%, vorzugsweise 0,5 bis 5 Gew.-% und insbesondere 1 bis 3 Gew.-%, an mindestens einer weiteren hautaufhellenden Substanz enthält, die ausgewählt ist aus der Gruppe, bestehend aus Ascorbinsäure, Ascorbinsäuresalzen, Ascorbinsäureglycosiden, Ascorbinsäureglycosidsalzen, Ascorbinsäureestern von organischen Säuren, den Salzen der Ascorbinsäureester von organischen Säuren, Ascorbinsäureestern von anorganischen Säuren, den Salzen der Ascorbinsäureester von anorganischen Säuren, Extrakten aus der Wurzel von Glycyrrhiza glabra, Glycyrrhetin, Glycyrrhetinsäure und ihren Salzen, Extrakten aus verschiedenen Teilen des Maulbeerbaums (Morus spp., insbesondere aus Morus alba und hier insbesondere Extrakte aus der Baumrinde, dem Stamm, den Blättern und den Früchten), Extrakten aus der Schale von Citrus unshiu, Phytinsäure, Diacetylboldin (O,O-Diacetylboldin), Hexapeptide-2, Extrakten aus dem Pilz Nigrospora sphaerica, Hydrochinon, Kojisäure, Arbutin, Extrakten aus Scutellaria spp. (Helmkraut), insbesondere aus Scutellaria baicalensis (Baikal-Helmkraut) und hier insbesondere Extrakte aus der Wurzel, Extrakten aus *Waltheria indica* ("Sleepy Morning") und hier insbesondere Extrakte aus den Blättern, Extrakten aus Bärentraube (*Arctostaphylos uva-ursi* (L.), Ericaceae) und hier insbesondere Extrakte aus den Blättern, Extrakten aus Preiselbeere (Blätter und Blüten), Extrakten aus Heidelbeere (Früchte), Extrakten aus Blattknospen von Birnbäumen, Anissamenöl, Extrakten aus Brombeerblättern, weiterhin Extrakten aus *Pyrola rotundifolia* (Rundblättriges Wintergrün), Gurke und/oder Limone, weiterhin Cumarinsäure ((Z)-2-Hydroxyzimtsäure), Hydroxystilbenen, deren Estern, Salzen und Oligomeren, insbesondere Resveratrol, alpha-Viniferin und epsiion-Viniferin, Extrakten aus diversen Bestandteilen der Weinreben (Traubenfrüchte, Traubenkerne, Traubenschalen, Stiele), sowie Mischungen der vorgenannten Substanzen.

8. Kosmetische oder dermatologische Zusammensetzung nach einem der Ansprüche 1 - 7, **dadurch gekennzeichnet, dass** sie mindestens einen öllöslichen UV-Filter, ausgewählt aus der Gruppe, bestehend aus Alkyl- und/oder Alkoxy-substituierten Dibenzoylmethanderivaten und Polysilicone-15, enthält.

9. Kosmetische oder dermatologische Zusammensetzung nach einem der Ansprüche 1 - 8, **dadurch gekennzeichnet, dass** sie mindestens einen wasserlöslichen UV-Filter, umfassend ein Derivat der Benzimidazolsulfonsäure, enthält.

10. Kosmetische oder dermatologische Zusammensetzung nach einem der Ansprüche 1 - 9, **dadurch gekennzeichnet, dass** die UV-Filtersubstanz Ethylhexyl Triazone enthalten ist.

11. Kosmetische oder dermatologische Zusammensetzung nach einem der Ansprüche 1 - 10, **dadurch gekennzeichnet, dass** 0,01 - 5 Gew.-% 8-Hexadecen-1,16-dicarbonsäure und 0,0001 bis 5 Gew.-% Sulforaphan enthalten.

12. Kosmetische oder dermatologische Zusammensetzung nach einem der Ansprüche 1 - 11, **dadurch gekennzeichnet, dass** 0,01 - 5 Gew.-% 8-Hexadecen-1,16-dicarbonsäure und 0,0001 bis 5 Gew.-% Allylisothiocyanat enthalten.

13. Kosmetische oder dermatologische Zusammensetzung nach einem der Ansprüche 1 - 12, **dadurch gekennzeichnet, dass** 0,01 - 5 Gew.-% 8-Hexadecen-1,16-dicarbonsäure und 0,0001 bis 5 Gew.-% Benzylisothiocyanat enthalten.

14. Kosmetische oder dermatologische Zusammensetzung nach einem der Ansprüche 1 - 13, **dadurch gekennzeichnet, dass** sie mindestens eine Substanz, ausgewählt aus der Gruppe, bestehend aus Biotin, Biotinestern, Folsäure und Folsäureestern, in einer Gesamtmenge von 0,0001 bis 2 Gew.-%, vorzugsweise 0,01 bis 1 Gew.-%, jeweils bezogen auf das Gewicht der gesamten Zusammensetzung, enthält.

15. Nicht-therapeutische, kosmetische Verwendung einer kosmetischen oder dermatologischen Zusammensetzung nach mindestens einem der Ansprüche 1 bis 14 zur Aufhellung der Hautfarbe, zur Reduzierung der Pigmentierung der Haut, zum Ausgleich des Erscheinungsbildes einer ungleichmäßigen Hautpigmentierung und/oder zur Aufhellung von Altersflecken der Haut.
